# EUROPEAN PATENT SPECIFICATION

(11) **EP 3 016 969 B1**
(45) Date of publication and mention of the grant of the patent: **30.08.2017**
(21) Application number: 14749946.1
(22) Date of filing: 04.07.2014
(51) Int. Cl.: C07K 7/64, C07K 14/045, C07K 14/47, A61K 38/12, A61K 39/12

(54) **IMMUNOTHERAPY**
IMMUNTHERAPIE
IMMUNOTHÉRAPIE

(30) Priority: 05.07.2013 GB 201312133
(43) Date of publication of application: 11.05.2016
(60) Divisional application: 17173839.6
(73) Proprietor: The University of Birmingham, Edgbaston Birmingham West Midlands B15 2TT (GB)
(72) Inventor: COBBOLD, Mark, Boston, MA 02129 (US); MILLAR, David, University of Birmingham B15 2TT (GB)
(74) Representative: Potter Clarkson LLP
(86) International application number: PCT/GB2014/052052
(87) International publication number: WO 2015/001361

(56) References cited:
- WO-A1-2012/123755
- WO-A2-2011/053798
- US-A1- 2009 042 235
- A Childerstone ET AL: "The reactivity of naturally sensitized human CD4 cells and IgG antibodies to synthetic peptides derived from the amino terminal sequences of a 3800 MW Streptococcus mutans antigen", Immunology, 1 February 1990 (1990-02-01), pages 177-183, XP055147838, ENGLAND Retrieved from the Internet: URL:http://www.ncbi.nlm.nih.gov/pubmed/168 9692 [retrieved on 2014-10-21]

## Description

The present invention relates to immunotherapeutic agents. In particular, it relates to agents that can be used to prevent or treat a condition characterised by the presence of unwanted cells, such as tumours or other disease causing cells.

The listing or discussion of an apparently prior-published document in this specification should not necessarily be taken as an acknowledgement that the document is part of the state of the art or is common general knowledge.

Immunotherapeutic strategies for targeting malignant disease are an active area of translational clinical research, and have been for several decades. The current models dictate that cancer represents a functional mismatch of immune effector responses whereby the host generally has a robust and functional immune response against endogenous viral pathogens, but lacking such responses against the resident malignant tissue.

If a therapeutic strategy could be developed which can efficiently molecularly harness an endogenous cytotoxic anti-viral immune response to instead target malignant tissue, this may afford a new powerful and safe approach to treat malignant disease.

The majority of cytotoxic therapeutic antibodies rely on immunological effector mechanisms to deliver their anti-cancer effect such as complement dependent cytotoxicity (CDC) and Antibody Dependent Cellular Cytotoxicity (ADCC). Importantly, all cells (both healthy and malignant) have numerous mechanisms to limit attack by the immune response to avert autoimmunity. This is evident in the context of autoimmune disease where high levels of tissue-reactive antibodies, which although frequently evoke organ inflammation, rarely induce complete organ destruction. Indeed, autoimmune diseases where complete tissue destruction is observed, such as diabetes mellitus, are known to be dependent on CTL responses rather than antibody-directed mechanisms.

The agents of the invention are an example of re-directed immunotherapy. This refers to the concept of re-directing an existing immune response that normally target cells harbouring foreign antigens, to target unwanted cells in conditions such as cancer. The concept requires the presentation of marker antigens on unwanted cells such that they become a target for immune cells.

WO 95/17212 describes conjugates consisting of peptidic T cell antigens and cell binding partners and their use in re-directed immunotherapy. The conjugates comprise a binding partner with selectivity for target cells and a T cell antigen peptide, and are said to induce specific cytotoxicity of T cells in the treatment of cancer, autoimmune diseases, diabetes or allergic diseases. The conjugates are said to be internalised into target cells following binding of the binding partner to surface receptors, and the T cell antigen peptide is processed from the conjugate and expressed on the cell surface in the form of a complex with MHC molecules. Cytotoxicity of T cells for the target cells is thereby induced.

However, which binding partners enable internalisation and hence subsequent presentation of the T cell antigen peptide, and which do not, is difficult to predict from WO 95/17212. Further, the conjugates described in WO 95/17212 do not efficiently target the MHC Class-I antigen processing pathway. An advantage of the MHC Class-I pathway is that, unlike MHC Class-II molecules, MHC Class-I molecules are present on all cell types.

Smith et al (J Immunol 169: 99-107, 2002) describe the use of ricin to deliver cytotoxic T cell epitopes into the MHC Class I pathway of tumour cells, such that they are subsequently lysed. However, ricin is highly toxic and since it can bind to most cell types, it is not selective for tumour cells.

The agents of the present invention aim to circumvent all of the above problems whilst improving specificity, and exploit the fact that T cell antigen peptides can be presented without first being internalised into a cell and being engaged in the classical antigen processing pathways. In addition to the classical MHC Class-I processing pathway, which continuously feeds peptides from the intracellular compartment via targeted intracellular proteolysis by the proteosome, peptide transport through the TAP and MHC Class-I peptide loading within the ER, antigens can also be presented without internalisation. This less directed mechanism relies on a short half-life of some MHC Class-I associated peptides due to low affinity of some MHC bound peptides to the MHC molecule. Peptide dissociation provides empty MHC Class-I molecules which are able to bind T cell antigens (e.g. peptides) at the membrane. In the same way, T cell antigens can bind to MHC Class-II molecules and the present invention also circumvents the Class-II antigen processing pathway to directly load antigens at the cell membrane. Further, Group I CD1 molecules (CD1a, CD1b and CD1c) have been shown to present lipids to both cytotoxic alpha beta T cells as well as cytotoxic gamma delta T cells (Porcelli et al (1989) Nature, 341, 447-450).

The inventors have previously found that by introducing a cleavage site in close proximity to the T cell antigen, wherein the cleavage site is selectively cleaved in the vicinity of the unwanted cells, the T cell antigen can be liberated from a targeting moiety in the vicinity of the unwanted cells and can become bound by, for example, empty MHC molecules or Group I CD1 molecules, and elicit a T cell response (see WO 2012/123755). In this way, internalisation of the agent into the cell and direction of the antigen into the classical processing pathways is not necessary; the agent can target cells expressing MHC Class-I molecules compared to only MHC Class-II expressing cells as in WO 95/17212; and specificity is increased by virtue of the cleavage site only being cleaved in the vicinity of the unwanted cells. For example, tumour cells secrete proteases that are required by tumours for invasion of local tissues and metastasis, and so by including a tumour-specific protease cleavage site in the agent, the specificity of the agent for the tumour is increased. It follows that the use of a cleavage site to bypass the requirement for internalisation and classical processing in order for T cell antigens to be efficiently presented to T cells is a key advantage.

However, there still remains a need for more effective immunotherapeutic agents. For example, directing immunotherapeutic agents to the vicinity of cancer cells is not always easy, and ideally requires the use of a targeting moiety with high tumour penetration. The penetrance of large macromolecular entities, such as antibodies, into tumours is known to be particularly poor, and so refinements to improve upon this would be attractive. Also, it is highly desirable to simplify the manufacturing process of immunotherapeutic agents, so as to hasten drug development and reduce costs.

The inventors have now developed a new immunotherapeutic agent that seeks to address the above problems. Surprisingly and unexpectedly, the inventors have found that by presenting the T cell antigen peptide within a cyclic peptide there is no longer a need for a targeting moiety. This was especially unforeseen since a targeting moiety had previously been regarded as an element of the agent in order to elicit an effective T cell response at the desired location. Such an agent benefits from improved tumour penetration, a simplified manufacture process, lower production costs and development time, improved stability (eg to degradation by exopeptidases), reduced likelihood of immunogenicity, easier administration, easier formulation and a longer shelf life. Disclosed herein is an agent comprising:
a cyclic peptide comprising a T cell antigen peptide;
wherein in cyclised form the T cell antigen peptide is not capable of eliciting a T cell response; and
wherein the T cell antigen peptide is rendered capable of eliciting a T cell response by selective cleavage of one or more cleavage sites within the cyclic peptide.
As described above, the agent may be used for preventing or treating a condition characterised by the presence of unwanted cells, in which case the T cell antigen peptide is rendered capable of eliciting a T cell response by selective cleavage of one or more cleavage sites within the cyclic peptide in the vicinity of the unwanted cells.

Thus, the invention provides an agent for use in preventing or treating a condition characterised by the presence of unwanted cells, the agent comprising a cyclic peptide that comprises a T cell antigen peptide, wherein in cyclised form the T cell antigen peptide is not capable of eliciting a T cell response, and wherein the T cell antigen peptide is rendered capable of eliciting a T cell response by selective cleavage of one or more cleavage sites within the cyclic peptide in the vicinity of the unwanted cells.

By a 'cyclic peptide' we include the meaning of any peptide whose peptide chain forms a ring structure. It is preferred if the peptide chain forms a covalent ring structure, as described below. However, it will be appreciated that a ring structure may be formed by non-covalent linkage, for example in the case of a globular polypeptide where the ends of the peptide chain are buried within the centre of the structure and brought into the vicinity of one another by hydrophobic interactions.

There are various ways of forming a ring structure. For example, the amino and carboxy termini of the peptide may be joined together, or one terminus of the peptide may be attached to a side chain of an amino acid in the peptide chain, or the side chain of an amino acid in the peptide chain may be attached to the side chain of another amino acid in the peptide chain.

It will be appreciated that the termini of the peptide, or the terminus and side chain of the peptide, or the two side chains of amino acids in the peptide, may be linked together, directly or indirectly (eg by means of one or more linking moieties). Any homobifunctional or heterobifunctional linker capable of covalently linking the peptide chain to form a ring structure may be used.

Direct linkages may be via a standard peptide bond (*eg* a so-called 'head-to-tail' linker) or by any of the conventional ways of cross-linking molecules, such as those generally described in O'Sullivan et al Anal. Biochem. (1979) 100, 100-108. For example, one of the termini may be enriched with thiol groups and the other reacted with a bifunctional agent capable of reacting with those thiol groups, for example the N-hydroxysuccinimide ester of iodoacetic acid (NHIA) or N-succinimidyl-3-(2-pyridyldithio)propionate (SPDP), a heterobifunctional cross-linking agent which incorporates a disulphide bridge between the conjugated species. Amide and thioether bonds, for example achieved with m-maleimidobenzoyl-N-hydroxysuccinimide ester, are generally more stable *in vivo* than disulphide bonds.

It is known that bis-maleimide reagents allow the attachment of a thiol group (e.g. thiol group of a cysteine residue of an antibody) to another thiol-containing moiety (e.g. thiol group of a T cell antigen peptide or a linker intermediate), in a sequential or concurrent fashion. Other functional groups besides maleimide, which are reactive with a thiol group include iodoacetamide, bromoacetamide, vinyl pyridine, disulfide, pyridyl disulfide, isocyanate, and isothiocyanate.

Further useful cross-linking agents include S-acetylthioglycolic acid N-hydroxysuccinimide ester (SATA) which is a thiolating reagent for primary amines which allows deprotection of the sulphydryl group under mild conditions (Julian et al (1983) Anal. Biochem. 132, 68), dimethylsuberimidate dihydrochloride and N,N'-o-phenylenedimaleimide.

Particularly preferred crosslinking agents include sulfosuccinimidyl 4-[N-maleimidomethyl]cyclohexane-1-carboxylate (Sulfo-SMCC), sulfosuccinimidyl 6-(3'-[2-pyridyldithio]-propionamido) hexanoate (Sulfo-LC-SPDP) and *N*-[ß-Maleimidopropionic acid] hydrazide, trifluoroacetic acid salt (BMPH).

It will be understood that a large number of homobifunctional and heterobifunctional crosslinking chemistries would be appropriate to join termini and/or side chains, and any such chemistry may be used. For example, Click Chemistry using Staudinger Ligation Chemistry (phosphine-azido chemistry) may be used.

It is appreciated that the termini of the peptide, or the terminus and side chain of the peptide, or the two side chains of amino acids in the peptide do not need to be linked directly to each other, but may be attached via one or more linker moieties. For example, when the termini are linked together indirectly, the amino terminus of the peptide may be attached to a linking moiety which, in turn, is attached to the carboxy terminus of the peptide. Alternatively, the amino terminus of the peptide may be attached to a first linking moiety that is, in turn, attached to a second linking moiety which, is attached to the carboxy terminus of the peptide. Similar linking approaches may be used to link an amino acid side chain to a terminus of the peptide chain or to another amino acid side chain in the peptide chain. Thus, it is possible for the cyclic peptide to contain one or more linking moieties that are necessary to maintain the cyclic structure. In other words, the cyclic peptide may comprise a T cell antigen peptide and, optionally, one or more other moieties such as one or more linking moieties.

The one or more linking moieties may be any covalent entity, but are typically amino acids or peptides. Thus, the one or more linking moieties may correspond to a single amino acid, or they may correspond to a peptide having at least 2, 3, 4, 5, 6, 7, 8, 9 or 10 or more amino acids. Typically, linker peptides would have between 2 and 8 amino acids. Suitable linker peptides are those that adopt a random coil conformation, for example, the peptide may contain glycine or alanine or proline or a mixture of alanine, glycine and proline residues. Given that a purpose of a linker peptide is to provide a gap, however, it will be recognised that the actual peptide sequence is not critical. A particular example of a suitable linker sequence is GGGGS (SEQ ID No: 277).

In one embodiment, such linker moieties contain one or more selectively cleavable cleavage sites as described herein. Thus, the one or more linking moieties may be amino acids or peptides that contain one or more selectively cleavable cleavage sites (eg enzymatic cleavage sites such as protease cleavage sites). Further details of appropriate cleavage sites are given below.

Although the ring structure of cyclic peptides may be formed exclusively of normal peptide bonds, other bond types may also be used. Indeed, cyclic peptides can be classified according to the types of bonds that comprise the ring, and all categories are included in the invention. For example, homodetic cyclic peptides, such as cyclosporine A, are those in which the ring is composed exclusively of normal peptide bonds (i.e. between the alpha carboxyl of one residue to the alpha amine of another). Cyclic isopeptides contain at least one non-alpha amide linkage, such as a linkage between the side chain of one residue to the alpha carboxyl group of another residue, as in microcystin and bacitracin. Cyclic depsipeptides, such as aureobasidin A and HUN-7293, have at least one lactone (ester) linkage in place of one of the amides. Some cyclic depsipeptides are cyclised between the C-terminal carboxyl and the side chain of a Thr or Ser residue in the chain, such as kahalalide F, theonellapeptolide, and didemnin B. Bicyclic peptides such as the amatoxins amanitin and phalloidin contain a bridging group, generally between two of the side chains. In the amatoxins, this bridge is formed as a thioether between the Trp and Cys residues. Other bicyclic peptides include echinomycin, triostin A, and Celogentin C. There are also a number of cyclic peptide hormones which are cyclised through a disulfide bond between two cysteines, as in somatostatin and oxytocin. Tricyclic peptides are also included. Further examples are disclosed in EP 2257624 and WO 2009098450, including ones where tris-(bromomethyl) benzene (TBMB) is used to link 3 cysteine residues to form a bi-cycle structure.

In a preferred embodiment, the cyclic peptide is a so-called 'head to tail' cyclic peptide in which the termini of the peptide are linked together by a standard peptide bond.

The cyclic peptide of the agent of the invention can range anywhere from just a few amino acids in length to hundreds. Typically, the cyclic peptide has between 9 and 100 amino acids, such as between 10 and 90 amino acids, 20 and 80 amino acids, 30 and 70 amino acids and 40 and 60 amino acids. More typically, the cyclic peptide has between 9 and 30 amino acids. For example, the cyclic peptide may have at least 9, 10, 11, 12, 13, 14, 15, 16, 17, 18, 19, 20, 21, 22, 23, 24, 25, 26, 27, 28, 29 or 30 amino acids. It will be understood that the larger the peptide, the more likely it is to form secondary structures, become more immunogenic and/or become more susceptible to proteolytic degradation. Hence, in one embodiment, it is preferred if the cyclic peptide has a length of no more than 100 amino acids, such as no more than 90, 80, 70, 60, 50, 40 or 30 amino acids.

In one embodiment, the cyclic peptide comprising a T cell antigen peptide is a cyclic peptide that consists of the T cell antigen peptide. In other words, the cyclic peptide is a T cell antigen peptide in a cyclic form. In this case, it will be appreciated that the one or more cleavage sites (eg protease cleavage sites) may reside in the T cell antigen peptide itself. If the T cell antigen peptide were to terminate with an arginine residue, for example, this could be appropriately cleaved by trypsin. Further details of appropriate cleavage sites are given below.

In another embodiment, the cyclic peptide comprising a T cell antigen peptide is a cyclised peptide that contains a T cell antigen peptide and one or more other linking moieties to form a ring structure. The one or more linking moieties may be any suitable linking moieties to complete the ring structure and may optionally contain the one or more cleavage sites. Typically, the one or more other linking moieties are amino acids or peptides as described above.

Methods of designing and making cyclic peptides are well established in the art and are described, for example, in Demmer et al, 2009 (Design of Cyclic peptides, in Peptide and Protein Design for Biopharmaceutical Applications (Ed KJ Jensen), John Wiley & Sons, Ltd, Chicester, UK, doi: 10.1002/9780470749708.ch4), and in Lianshan & Tam, 2002 (Peptides Frontiers of Peptide Science, American Peptide Symposia Volume 5, 2002, pp19-22, Novel concepts for the synthesis of cyclic peptide libraries). Chemical synthesis of the cyclic peptides can be done by commercial biotech companies including JPT (Germany).

In an embodiment, the cyclic peptide of the invention does not comprise the sequence RIKPHQG (Arg-Ile-Lys-Pro-His-Gln-Gly) (SEQ ID No: 279) as described in US 2004/0092434.

Amino acid residues described herein are generally in the natural "L" isomeric form. However, residues in the "D" isomeric form can be substituted for L-amino acid residues in certain situations, provided that the agent of the invention still retains its function of preventing or treating a condition characterised by the presence of unwanted cells. The definition also includes, unless otherwise specifically indicated, chemically-modified amino acids, including amino acid analogues (such as penicillamine, 3-mercapto-D-valine), naturally-occurring non-proteogenic amino acids (such as norleucine), beta-amino acids, azapeptides, N-methylated amino acids and chemically-synthesised compounds that have properties known in the art to be characteristic of an amino acid. The term "proteogenic" indicates that the amino acid can be incorporated into a protein in a cell through well-known metabolic pathways. The definition also includes amino acids in which the functional side group has been chemically derivatised. Such derivatised molecules include, for example, those molecules in which free amino groups have been derivatised to form amine hydrochlorides, p-toluene sulfonyl groups, carbobenzoxy groups, t-butyloxycarbonyl groups, chloroacetyl groups or formyl groups. Free carboxyl groups may be derivatised to form salts, methyl and ethyl esters or other types of esters or hydrazides. Free hydroxyl groups may be derivatised to form O-acyl or O-alkyl derivatives. Also included as derivatives are those peptide portions that contain one or more naturally occurring amino acid derivatives of the twenty standard amino acids. The definition further includes peptoids, or poly-*N*-substituted glycines, whose side chains are appended to the nitrogen atom of the peptide backbone, rather than to the alpha-carbons (Renya et al, 1992 PNAS 89(20): 9367-9371). Accordingly, it is appreciated that the peptide making up the cyclic peptide can be a peptide "mimetic", i.e. a peptidomimetic which mimics the structural features of the peptide comprising or consisting of an amino acid sequence as described herein.

### T cell antigen peptide

By a 'T cell antigen peptide' we include the meaning of any peptide which can be presented to a T cell so as to elicit a T cell response. For example, the T cell antigen peptide may be presented to a T cell by an MHC molecule. Once the antigenic peptide is presented on the surface of the cell, the cell is recognised as foreign and becomes the target of T cells, some of which have the natural function of eliminating foreign cells either infected by foreign organisms such as viruses, fungi, bacteria, mycobacteria or protozoa, or which have become cancerous (eg malignant). Thus, it will be appreciated that the T cell antigen peptide may be one that is capable of being presented by a molecule on an unwanted cell. It is possible, however, that the T cell antigen peptide may be presented on a cell other than an unwanted cell but still in the vicinity of an unwanted cell, and by virtue of subsequent T cell activation, an unwanted cell is killed, for example by local production of cytokines by activated T cells. Such indirect killing may be desirable, for example to target tumour blood vessels and/or stromal cells which support tumour growth.

It will be appreciated that the T cell antigen peptide is one that can elicit an existing T cell response in the subject to which the agent of the invention is administered. Typically, the T cell antigen peptide is not one which generates a new primary T cell response for that antigen via cross-presentation in APCs. To put it another way, the T cell antigen peptide is one to which a number of T cells in the subject are already sensitised to. Determining whether a subject's cells are sensitised to a given peptide can be done by contacting isolated peripheral mononuclear blood cells from the subject with the peptide and using standard assays for cell proliferation, as described further below and in the Examples.

In an embodiment, the agent of the invention is not one which generates a new T cell response specific for the T cell antigen peptide contained in it. Accordingly, the invention includes an agent comprising a cyclic peptide that comprises a T cell antigen peptide; wherein in cyclised form the T cell antigen peptide is not capable of eliciting a T cell response; and wherein the T cell antigen peptide is rendered capable of eliciting a T cell response by selective cleavage of one or more cleavage sites in the cyclic peptide, and wherein the T cell antigen peptide is capable of eliciting an existing T cell response in a subject.

By 'T cell', we include all types of T cell including CD4+, CD8+, γδ T cells, and NK-T cells. Preferably, the T cell is a cytotoxic T cell such that a cytotoxic T cell response is elicited.

As is known in the art, the mechanism of antigen presentation will depend upon the type of T cell. Generally, CD4+ T cells recognise peptide antigens bound to MHC Class II molecules and CD8+ T cells recognise peptide antigens bound to MHC Class I molecules. It is understood that any presentation route may be used provided that the antigen elicits a T cell response. Typically, the T cell antigen peptide is one that is capable of binding to an MHC Class I or MHC Class II molecule.

Preferably, the T cell antigen peptide is an immunodominant antigenic peptide (eg a peptide that elicits an existing immunodominant response). By 'immunodominant' we include the meaning that the peptide antigen elicits a T cell response with high magnitude, sensitivity, tissue homing characteristics and efficiency in killing antigen bearing cells. Generally, an immunodominant response comprises more than 0.1% of a subject's CD8⁺ or CD4⁺ T cells. Determining the extent of a T cell response for a given peptide antigen can be done for example by contacting isolated peripheral mononuclear blood cells from the subject with the peptide antigen and using standard assays for cell proliferation known in the art. Suitable assays for determining the extent of an immune response include ELISpot, intracellular cytokine staining, HLA-peptide tetramer staining, proliferation assay, activation assays (eg CD69), CD107 mobilisation assays or metabolic assays (eg MTT).

The T cell antigen peptide may be an MHC Class I restricted antigen that binds only to MHC Class I molecules, or it may be an MHC Class II restricted antigen that binds only to MHC Class II molecules. It is appreciated that the peptide antigen may bind only to particular variant MHC Class I and/or MHC Class II molecules (e.g. natural variants found in particular subjects), or that the antigen may be capable of binding to any MHC Class I and/or MHC Class II molecule (i.e. the antigen is promiscuous).

In one embodiment, the T cell antigen peptide is capable of binding to a MHC Class I molecule such as any of HLA-A, HLA-B and HLA-C. Since MHC Class I molecules are expressed on all cell types, this allows to agent of the invention to redirect an immune response to any unwanted cell. Most preferably, the peptide is capable of binding to HLA types A1, A2.1, A3.2, A11, A24, B7, B8, B35, B44, Cw1, Cw2, Cw3, Cw4 and Cw6 or mixtures thereof, which are believed to cover more than 90% of the Caucasian and Asian populations.

In another embodiment, the T cell antigen peptide is capable of binding to a MHC Class II molecule such as any of HLA-DP, HLA-DQ or HLA-DR. Common MHC Class II types include DR1, DR3, DR4, DR7, DR52, DQ1, DQ2, DQ4, DQ8 and DP1. MHC Class II molecules are expressed on immune cells including antigen presenting cells such as dendritic cells, B cells and macrophages. Thus, when the T cell antigen peptide is MHC Class II restricted, the agent of the invention may be used to treat conditions such as lymphomas or autoimmune diseases.

An example of a promiscuous peptide that may be used is the PADRE MHC Class-II epitope defined in Alexander et al (2000) The Journal of Immunology 164: 1625-1633; aKXVAAWTLKAAaZC (a= d-Alanine, X = L-cyclohexylalanine, Z = aminocaproic acid)) (SEQ ID No: 1). Since this epitope is artificial, it would first need to be introduced to the patient in a vaccine to generate an immune response prior to administering the agent of the invention. Another promiscuous peptide that may be used is the tetanus fragment C peptide.

Conveniently, the T cell antigen peptide is an immunogenic peptide that is recognised by an MHC molecule. Such peptides usually have a length of 9 to 22 amino acids (for recognition in the MHC Class II complex) or of 8 to 13 amino acids (for recognition in the MHC Class I complex). Preferably, the peptide is an immunodominant peptide.

Examples of immunodominant peptides include viral derived peptides that elicit endogenous anti-viral responses. Thus, the peptide may be derived from an endogenous virus such as Varicella-Zoster virus, Herpes simplex virus, cytomegalovirus, Epstein Barr virus, or influenza. Particularly preferred examples include peptides derived from human cytomegalovirus (CMV or Human herpesvirus 5/HHV5) or Epstein-Barr Virus (EBV or HHV4); herpesviruses such as HHV1, HHV2 and HHV3; influenza virus A; influenza virus B; rhinovirus; adenovirus; and Hepadnaviridae.

For human cytomegalovirus (HHV5) the immunodominant antigens are well characterised (see Sylwester AW et al J Exp Med. 2005 Sep 5;202(5):673-85, incorporated herein by reference), and any such antigen described in Sylwester *et al* may be used in the present invention. In particular, Sylester *et al* synthesised consecutive 15mer peptides, overlapping by 10 amino acids, for 213 predicted human CMV proteins. This generated 13,687 peptides that were arranged in ORF or sub-ORF specific mixes. Peptides derived from ORFs UL55 (gB), UL 83 (pp65), UL 86, UL 99 (pp28), UL 122 (IE2), UL 36, UL 48, UL32 (pp150), UL 113, IRS-1, UL 123 (IE1), UL25, UL 141, UL 52 and UL 82 (pp71) were found to elicit the most CD 4+ T cell responses, and so it is particularly preferred if the peptide is derived from one of these ORFs. Alternatively, peptides derived from ORFs UL48, UL83 (pp66), UL 123 (IE1), UL 123 (IE2), US 32, UL 28, US 29, US3, UL 32 (pp150), UL 55 (gB), UL 94, UL 69, UL 105, UL 82 (pp71) and UL 99 (pp28) were found to elicit the most CD 8+ T cell responses, and so it is particularly preferred if the peptide is derived from one of these ORFs.

Particular cytomegalovirus T cell antigen peptides are listed below.

CD8+ T cell epitopes for cytomegalovirus antigens such as IE1 include YILEETSVM (SEQ ID No: 2), YVLEETSVM (SEQ ID No: 3), VLEETSVML (SEQ ID No: 4), VLAELVKQI (SEQ ID No: 5), ATTFLQTMLR (SEQ ID No: 6), EVISVMKRR (SEQ ID No: 7), CRVLCCYVL (SEQ ID No: 8), ELRRKMMYM (SEQ ID No: 9), ELKRKMIYM (SEQ ID No: 10), QIKVRVDMV (SEQ ID No: 11), CVETMCNEY (SEQ ID No: 12), RRKMMYMCY (SEQ ID No: 13), KRKMIYMCY (SEQ ID No: 14), RRIEEICMK (SEQ ID No: 15), DELRRKMMY (SEQ ID No: 16), KEVNSQLSL (SEQ ID No: 17), EEAIVAYTL (SEQ ID No: 18) and FPKTTNGCSQA (SEQ ID No: 19); for pp65 they include YSEHPTFTSQY (SEQ ID No: 20), NLVPMVATV (SEQ ID No: 21), MLNIPSINV (SEQ ID No: 22), RIFAELEGV (SEQ ID No: 23), QYDVPAALF (SEQ ID No: 24), FTSQYRIQGKL (SEQ ID No: 25), VYALPLKML (SEQ ID No: 26), QYVKVYLESF (SEQ ID No: 27), FVFPTKDVALR (SEQ ID No: 28), YTPDSTPCHR (SEQ ID No: 29), DTPVLPHETR (SEQ ID No: 30), TPRVTGGGAM (SEQ ID No: 31), RPHERNGFTVL (SEQ ID No: 32), IPSINVHHY (SEQ ID No: 33), FPTKDVAL (SEQ ID No: 34), CPSQEPMSIYVY (SEQ ID No: 35), QPSLILVSQY (SEQ ID No: 36), SEHPTFTSQY (SEQ ID No: 37), EFFDANDIY (SEQ ID No: 38); for pp150 they include TTVYPPSSTAK (SEQ ID No: 39), QTVTSTPVQGR (SEQ ID No: 40), NVRRSWEEL (SEQ ID No: 41), KARDHLAVL (SEQ ID No: 42); for gB they include RIWCLVVCV (SEQ ID No: 43); and for pp50 they include VTEHDTLLY (SEQ ID No: 44), RGDPFDKNY (SEQ ID No: 45), TVRSHCVSK (SEQ ID No: 46).

CD4+ T cell epitopes for cytomegalovirus antigens such as pp65 include PQYSEHPTFTSQYRIQ (SEQ ID No: 47), FTSQYRIQGKLEYRHT (SEQ ID No: 48), LLQTGIHVRVSQPSL (SEQ ID No: 49), NPQPFMRPHERNGFT (SEQ ID No: 50), EPDVYYTSAFVFPTK (SEQ ID No: 51), IIKPGKISHIMLDVA (SEQ ID No: 52), AGILARNLVPMVATV (SEQ ID No: 53), KYQEFFWDANDIYRI (SEQ ID No: 54); for gB they include DYSNTHSTRYV (SEQ ID No: 55), CMLTITTARSKYPYH (SEQ ID No: 56), and VFETSGGLVVFWQGI (SEQ ID No: 57); for IE1 they include VRVDMVRHRIKEHMLKKYTQ (SEQ ID No: 58) and NYIVPEDKREMWMACIKELH (SEQ ID No: 59); and for gH they include HELLVLVKKAQL (SEQ ID No: 60).

For Epstein Barr Virus (EBV or HHV4), immunodominant proteins are also well characterised and are provided in Hislop AD et al Annu Rev Immunol. 2007;25:587-617 (incorporated herein by reference). A list of T cell epitopes, adapted from Hislop *et al* is provided below.

**Table 3: CD8+ and CD4+ T cell epitopes identified in EBV lytic and latent cycle proteins (adapted from Hislop et al)**

| **CD8+ T cell epitopes** | | | | |
|---|---|---|---|---|
| EBV Antigen | Epitope coordinates | Epitope sequence | SEQ ID No | HLA restriction |
| *Latent cycle proteins* | | | | |
| EBNA1 | 72-80 | RPQKRPSCI | 61 | B7 |
| | 407-415 | HPVGEADYF | 62 | B53 |
| | 407-417 | HPVGEADYFEY | 63 | B35.01 |
| | 528-536 | IPQCRLTPL | 64 | B7 |
| | 574-582 | VLKDAIKDL | 65 | A2.03 |
| | | | | |
| EBNA2 | 14-23 | YHLIVDTDSL | 66 | B38 |
| | 42-51 | DTPLIPLTIF | 67 | A2/B51 |
| | 234-242 | RPTELQPTP | 68 | B55 |
| | | | | |
| EBNA3A | 158-166 | QAKWRLQTL | 69 | B8 |
| | 176-184 | AYSSWMYSY | 70 | A30.02 |
| | 246-253 | RYSIFFDY | 71 | A24 |
| | 325-333 | FLRGRAYGL | 72 | B8 |
| | 378-387 | KRPPIFIRRL | 73 | B27 |
| | 379-387 | RPPIFIRRL | 74 | B7 |
| | 406-414 | LEKARGSTY | 75 | B62 |
| | 450-458 | HLAAQGMAY | 76 | |
| | 458-466 | YPLHEQHGM | 77 | B35.01 |
| | 491-499 | VFSDGRVAC | 78 | A29 |
| | 502-510 | VPAPAGPIV | 79 | B7 |
| | 596-604 | SVRDRLARL | 80 | A2 |
| | 603-611 | RLRAEAQVK | 81 | A3 |
| | 617-625 | VQPPQLTLQV | 82 | B46 |
| | | | | |
| EBNA3B | 149-157 | HRCQAIRKK | 83 | B27.05 |
| | 217-225 | TYSAGIVQI | 84 | A24.02 |
| | 244-254 | RRARSLSAERY | 85 | B27.02 |
| | 279-287 | VSFIEFVGW | 86 | B58 |
| | 399-408 | AVFDRKSDAK | 87 | A11 |
| | 416-424 | IVTDFSVIK | 88 | A11 |
| | 488-496 | AVLLHEESM | 89 | B35.01 |
| | 657-666 | VEITPYKPTW | 90 | B44 |
| | | | | |
| EBNA3C | 163-171 | EGGVGWRHW | 91 | B44.03 |
| | 213-222 | QNGALAINTF | 92 | B62 |
| | 249-258 | LRGKWQRRYR | 93 | B27.05 |
| | 258-266 | RRIYDLIEL | 94 | B27.02/.04/.05 |
| | 271-278 | HHIWQNLL | 95 | B39 |
| | 281-290 | EENLLDFVRF | 96 | B44.02 |
| | 284-293 | LLDFVRFMGV | 97 | A2.01 |
| | 285-293 | LDFVRFMGV | 98 | B37 |
| | 335-343 | KEHVIQNAF | 99 | B44.02 |
| | 343-351 | FRKAQIQGL | 100 | B27.05 |
| | 881-889 | QPRAPIRPI | 101 | B7 |
| | | | | |
| EBNA-LP | 284-292 | SLREWLLRI | 102 | A2 |
| | | | | |
| LMP1 | 38-46 | FWLYIVMSD | 103 | |
| | 72-82 | FRRDLLCPLGA | 104 | B40 |
| | 125-133 | YLLEMLWRL | 105 | A2 |
| | 159-167 | YLQQNWWTL | 106 | A2 |
| | 166-174 | TLLVDLLWL | 107 | A2 |
| | 375-386 | DPHGPVQLSYYD | 108 | B51.1 |
| | | | | |
| LMP2 | 1-9 | MGSLEMVPM | 109 | B35.01 |
| | 61-75 | EDPYWGNGDRHSDYQ | 110 | |
| | 121-134 | NPVCLPVIVAPYLF | 111 | |
| | 125-133 | LPVIVAPYL | 112 | B53 |
| | 131-139 | PYLFWLAAI | 113 | A23 |
| | 141-154 | ASCFTASVSTVVTA | 114 | |
| | 144-152 | FTASVSTW | 115 | A68 |
| | 200-208 | IEDPPFNSL | 116 | B40.01 |
| | 236-244 | RRRWRRLTV | 117 | B27.04 |
| | 237-245 | RRWRRLTVC | 118 | B14.02 |
| | 240-250 | RRLTVCGGIMF | 119 | B27 |
| | 243-251 | TVCGGIMFL | 120 | A1 |
| | 249-262 | MFLACVLVLIVDAV | 121 | |
| | 257-265 | LIVDAVLQL | 122 | A2 |
| | 293-301 | GLGTLGAAI | 123 | A2 |
| | 329-337 | LLWTLWLL | 124 | A2.01 |
| | 340-350 | SSCSSCPLSKI | 125 | A11 |
| | 349-358 | ILLARLFLY | 126 | A29 |
| | 356-364 | FLYALALLL | 127 | A2 |
| | 419-427 | TYGPVFMCL | 128 | A24 |
| | 426-434 | CLGGLLTMV | 129 | A2.01 |
| | 442-451 | VMSNTLLSAW | 130 | A25 |
| | 453-461 | LTAGFLIFL | 131 | A2.06 |
| | 447-455 | LLSAWILTA | 132 | A2 |
| | | | | |

| *Lytic Cycle Proteins* | | | | |
|---|---|---|---|---|
| | | | | |
| BRLF1 | 25-39 | LVSDYCNVLNKEFT | 133 | B18 |
| | 25-33 | LVSDYCNVL | 134 | A2.05 |
| | 28-37 | DYCNVLNKEF | 135 | A24 |
| | 91-99 | AENAGNDAC | 136 | B45 |
| | 101-115 | IACPIVMRYYVLDHLI | 137 | A24/C2 |
| | 109-117 | YVLDHLIVV | 138 | A2.01 |
| | 121-135 | FFIQAPSNRVMIPAT | 139 | |
| | 134-142 | ATIGTAMYK | 140 | A11 |
| | 145-159 | KHSRVRAYTYSKVLG | 141 | A3 |
| | 225-239 | RALIKTLPRASYSSH | 142 | A2 |
| | 393-407 | ERPIFPHPSKPTFLP | 143 | Cw4 |
| | 529-543 | QKEEAAICGQMDLS | 144 | B61 |
| | 441-455 | EVCQPKRIRPFHPPG | 145 | |
| | | | | |
| BZLF1 | 52-64 | LPEPLPQGQLTAY | 146 | B35.08 |
| | 54-63 | EPLPQGQLTAY | 147 | B35.01 |
| | 81-89 | APENAYQAY | 148 | B35.01 |
| | 101-115 | LQHYREVAA | 149 | C8 |
| | 172-183 | DSELEIKRYKNR | 150 | B18 |
| | 186-201 | RKCCRAKFKQLLQHYR | 151 | C6 |
| | 190-197 | RAKFKQLL | 152 | B8 |
| | 209-217 | SENDRLRLL | 153 | B60 |
| | | | | |
| BMLF1 | 265-273 | KDTWLDARM | 154 | |
| | 280-288 | GLCTLVAML | 155 | A2.01 |
| | 397-405 | DEVEFLGHY | 156 | B18 |
| | 435-444 | SRLVRAILSP | 157 | B14 |
| | | | | |
| BMRF1 | 20-28 | CYDHAQTHL | 158 | A2 |
| | 86-100 | FRNLAYGRTCVLGKE | 159 | C3/C10 |
| | 116-128 | RPQGGSRPEFVKL | 160 | B7 |
| | 208-216 | TLDYKPLSV | 161 | A2.01 |
| | 268-276 | YRSGIIAVV | 162 | C6 |
| | 268-276 | YRSGIIAVV | 162 | B39 |
| | 286-295 | LPLDLSVILF | 163 | B53 |
| | | | | |
| BARF0 | | LLWAARPRL | 164 | A2 |
| | | | | |
| BCRF1 | 3-11 | RRLVVTLQC | 165 | B27 |
| | | | | |
| BALF2 | 418-426 | ARYAYYLQF | 166 | B27 |
| | | | | |
| BILF2 | 240-248 | RRRKGWIPL | 167 | B27 |
| | | | | |
| BLLF1 | | VLQWASLAV | 168 | A2 |
| (gp350) | | | | |
| | | | | |
| BALF4 | 276-284 | FLDKGTYTL | 169 | A2 |
| (gp110) | | ILIYNGWYA | 170 | A2 |
| | | VPGSETMCY | 171 | B35 |
| | | APGWLIWTY | 172 | B35 |
| | | | | |
| BXLF2 | | TLFIGSHVV | 173 | A2.01 |
| (gp85) | | SLVIVTTFV | 174 | A2.01 |
| | | LMIIPLINV | 175 | A2.01 |
| | | | | |

| **CD4+ T cell epitopes** | | | | |
|---|---|---|---|---|
| EBV Antigen | Epitope coordinates | Epitope sequence | (SEQ ID No) | HLA restriction |
| *Latent cycle proteins* | | | | |
| | | | | |
| EBNA1 | 71-85 | RRPQKRPSCIGCKGT | (176) | |
| | 403-417 | RPFFHPVGEADYFEY | (177) | |
| | 429-448 | VPPGAIEQGPADDPGEGPST | (178) | |
| | 434-458 | IEQGPTDDPGEGPSTGPRGQGDGGR | (179) | |
| | 455-469 | DGGRRKKGGWFGRHR | (180) | |
| | 474-493 | SNPKFENIAEGLRVLLARSH | (181) | |
| | 475-489 | NPKFENIAEGLRALL | (182) | |
| | 479-498 | ENIAEGLRVLLARSHVERTT | (183) | DQ7 |
| | 481-500 | IAEGLRALLARSHVERTTDE | (184) | DQ2/3 |
| | 485-499 | LRALLARSHVERTTD (185) | | |
| | 499-523 | EEGNWVAGVFVYGGSKTSLY NLRRG | (186) | |
| | 509-528 | VYGGSKTSLYNLRRGTALAI | (187) | DR11 |
| | 515-528 | TSLYNLRRGTALAI | (188) | DR1 |
| | 518-530 | YNLRRGTALAIPQ | (189) | DP3 |
| | 519-533 | NLRRGRTALAIPQCRL | (190) | |
| | 519-543 | EEGNWVAGVFVYGGSKTSLYN LRRG | (186) | |
| | 527-541 | AIPQCRLTPLSRLPF | (191) | DR13 |
| | 529-543 | PQCRLTPLSRLPFGM | (192) | DR14 |
| | 544-563 | APGPGPQPLRESIVCYFM (S43) | (193) | |
| | 549-568 | PQPGPLRESIVCYFMVFLQT(S44) | (194) | |
| | 551-570 | PGPLRESIVCYFMVFLQTHI | (195) | DR1 |
| | 554-573 | LRESIVCYFMVFLQTHIFAE | (196) | |
| | 554-578 | LRESIVCYFMVFLQTHIFAEVLKDA | (197) | |
| | 561-573 | YFMVFLQTHIFAE | (198) | DR11,12,13 |
| | 563-577 | MVFLQTHIFAEVLKD | (199) | DR15 |
| | 564-583 | VFLQTHIFAEVLKDAIKDL (200) | | DP5 |
| | 574-593 | VLKDAIKDLVMTKPAPTCNI (201) | | |
| | 589-613 | PTCNIKVTVCSFDDGVDLPPW FPPM (202) | | |
| | 594-613 | RVTVCSFDDGVDLPPWFPPM (203) | | |
| | 607-619 | PPWFPPMVEGAAA (204) | | DQ2 |
| | | | | |
| EBNA2 | 11-30 | GQTYHLIVDTLALHGGQTYH (205) | | DR4 |
| | 46-65 | IPLTIFVGENTGVPPPLPPP (206) | | |
| | 131-150 | MRMLWMANYIVRQSRGDRGL (207) | | |
| | 206-225 | LPPATLVPPRPTRPTTLPP (208) | | |
| | 276-295 | PRSTVFYNIPPMPLPPSQL (209) | | DR7,52a,52b,52c |
| | 280-290 | TVFYNIPPMPL (210) | | DQ2/DQ7 |
| | 301-320 | PAQPPPGVINDQQLHHLPSG (211) | | DR17 |
| | | | | |
| EBNA3A | 364-383 | EDLPCIVSRGGPKVKRPPIF (212) | | DR15 |
| | 780-799 | GPWVPEQWMFQGAPPSQGTP (213) | | DR1 |
| | 649-668 | QVADWRAPGVPAMQPQYF (214) | | |
| | | | | |
| EBNA3B | | | | |
| EBNA3C | 66-80 | NRGWMQRIRRRRRR (215) | | |
| | 100-119 | PHDITYPYTARNIRDAACRAV (216) | | DR16 |
| | 141-155 | ILCFVMAARQRLQDI (217) | | DR13 |
| | 386-400 | SDDELPYIDPNMEPV (218) | | DQ5 |
| | 401-415 | QQRPVMFVSRVPAKK (219) | | |
| | 546-560 | QKRAAPPTVSPSDTG (220) | | |
| | 586-600 | PPAAGPPAAGPRILA (221) | | |
| | 626-640 | PPWRMFMRERQLPQ (222) | | |
| | 649-660 | PQCFWEMRAGREITQ (223) | | |
| | 741-760 | PAPQAPYQGYQEPPAPQAPY (224) | | DR1/DR4 |
| | 916-930 | PSMPFASDYSQGAFT (225) | | |
| | 961-986 | AQEILSDNSEISVFPK (226) | | |
| | | | | |
| LMP1 | 11-30 | GPPRPPLGPPLSSSIGLALL (227) | | DR7 & DR9 |
| | 130-144 | LWRLGATIWQLLAFF (228) | | |
| | 181-206 | LIWMYYHGPRHTDEHHHDDS (229) | | DR16 |
| | 206-225 | QATDDSSHESDSNSNEGRHH (230) | | DQ2 |
| | 211-236 | SSHESDSNSNEGRHHLLVSG (231) | | DQB1*0601 |
| | 212-226 | SGHESDSNSNEGRHHH (232) | | |
| | 340-354 | TDGGGGHSHDSGHGG (233) | | |
| LMP2 | 73-87 | DYQPLGTQDQSLYLG (234) | | DR4 or DR16 |
| | 149-163 | STWTATGLALSLLL (235) | | |
| | 169-182 | SSYAAAQRKLLTPV (236) | | |
| | 189-208 | VTFFAICLTWRIEDPPFNSI | (237) | DRB1*0901 |
| | 194-213 | ICLTWRIEDPPFNSILFALL | (238) | DRB1*1001 |
| | 224-243 | VLVMLVLLILAYRRRWRRLT | (239) | |
| | 385-398 | STEFIPNLFCMLLL | (240) | |
| | 419-438 | TYGPVFMSLGGLLTMVAGAV | (241) | DQB1*0601 |
| | | | | |

| *Lytic Cycle Proteins* | | | | |
|---|---|---|---|---|
| BHRF1 | 171-189 | AGLTLSLLVICSYLFISRG | (242) | DR2 |
| | 122-133 | PYYWDLSVRGM | (243) | DR4 |
| | 45-57 | TWLRYHVLLEEI | (244) | DR4 |
| | | | | |
| BZLF1 | 174-188 | ELEIKRYKNRVASRK | (245) | DR13 |
| | 207-221 | KSSENDRLRLLLKQM | (246) | DQB1*0402 |
| | | | | |
| BLLF1 | 61-81 | LDLFGQLTPHTKAVYQPRGA | (247) | DRw15 |
| (gp350) | 65-79 | FGQLTPHTKAVYQPR | (248) | DRB1*1301 |
| | 130-144 | VYFQDVFGTMWCHHA | (249) | DQB1*0402 |
| | 163-183 | DNCNSTNI TAVVRAQGLDVTL | (250) | DRw11 |
| | | | | |
| BALF4 | 482-496 | AWCLEQKRQNMVLRE | (251) | DPB1*1301 |
| (gp110) | 575-589 | DNEIFLTKKMTEVCQ | (252) | DRB1*0801 |

Further examples of immunodominant peptides include HLA-A*0201-restricted epitopes (HCMV pp65 495-504 - NLVPMVATV (SEQ ID No: 21), HCMV IE1 VLEETSVML (SEQ ID No: 4), EBV LMP-2 356-364 FLYALALLL (SEQ ID No: 127), EBV BMLF-1 259-267 GLCTLVAML (SEQ ID No: 155)); HLA-A*0101-restricted epitopes (HCMV pp50 245-253 - VTEHDTLLY (SEQ ID No: 44) ; HCMV pp65 363-373 - YSEHPTFTSQY) (SEQ ID No: 20); HLA-A*0301-restricted epitopes (HCMV pp50 - TVRSHCVSK (SEQ ID No: 46)); HLA-B*0702-restricted epitopes (HCMV pp65 417-426 TPRVTGGGAM (SEQ ID No: 31), pp65 265-275 RPHERNGFTVL (SEQ ID No: 32)); and HLA-B*0801-restricted epitopes (HCMV IE1 88-96 - ELKRKMMYM (SEQ ID No: 253), IE1 88-96 QIKVRVDMV (SEQ ID No: 11), EBV BZLF-1 190-197 RAKFKQLL (SEQ ID No: 152), any of which may be used in the context of the invention.

Particularly preferred MHC Class I antigens included those derived from CMV such as CRVCCLYVL (SEQ ID No: 274; HLA-Cw07 binding peptide), NLVPMVATV (SEQ ID No: 21; HLA-A2 binding peptide), RPHERNGFTVL (SEQ ID No: 32; HLA-B7 binding peptide), TPRVTGGGAM (SEQ ID No: 31; HLA-B7 binding peptide), YSEHPTFTSQY (SEQ ID No; 20; HLA-A1 binding peptide) and VTEHDTLLY (SEQ ID No: 44; HLA-A1 binding peptide); and those derived from EBV including GLCTLVAML (SEQ ID No: 155) and CLGGLLTMV (SEQ ID No: 129; HLA-A2 binding peptides).

It is appreciated that the T cell antigen peptide may be one derived from a live vaccine such as Measles, Mumps, Rubella (MMR) or HHV3; or one derived from intracellular bacteria such as mycobacteria, particularly those evoked through immunization with BCG. Such peptides are well known in the art. Similarly, the T cell antigen (e.g. peptide) may be derived from the tetanus toxoid such as P2, P4 or P30. Thus, it will be understood that the T cell antigen peptide may be one that elicits an existing immune response in a subject that has been generated by prior vaccination against an infectious agent. It follows that in order to increase the number of T cells sensitised to a T cell antigen peptide, it may be desirable to vaccinate or boost a subject with a vaccine that comprises the T cell antigen peptide. For example, the subject may be vaccinated with a tetanus toxin, before being administered the agent of the invention comprising the relevant T cell antigen peptide.

It will be appreciated that because many people are vaccinated in childhood with these vaccines, they are likely to contain T cells which are sensitized to these T cell antigens. Thus, in one embodiment the T cell antigen peptide is one which is found in a childhood vaccine.

Although not preferred, the T cell antigen peptide may also be one that elicits an existing immune response in a subject that has been generated by exposing that subject's T cells to the antigen *in vitro.*

Peptides can be produced by well known chemical procedures, such as solution or solid-phase synthesis, or semi-synthesis in solution beginning with protein fragments coupled through conventional solution methods as is known in the art. Alternatively, the peptide can be synthesised by established methods including recombinant methods.

When the agent is for treating autoimmune or allergic diseases, it will be appreciated that the T cell antigen peptide may be an autoantigen or allergen respectively. In this way the immune response that is contributing to the disorder is redirected to unwanted cells so as to combat the disorder.

It is appreciated that the T cell antigen peptide may be chemically modified provided that it is still capable of eliciting a T cell response. Such chemical modification may include, for instance, the addition of a metal such as nickel, since it has been shown that in certain allergic patients there are T cells which recognise a peptide with a bound nickel atom (Romagnoli et al 1991, EMBO J 10: 1303-1306). The T cell antigen peptide can also be modified by an organic molecule which enhances the immunogenicity (Romero et al 1993, J Immunol 150: 3825-3831). Other modifications include phosphorylation, acetylation, alkylation, acylation, citrulination, nitration, sulphation and hydroxylation, forming salts with acids or bases, forming an ester or amide of a terminal carboxyl group, and attaching amino acid protecting groups such as N-t-butoxycarbonal.

It is appreciated that the T cell antigen peptide may comprise naturally occurring amino acids encoded by DNA, and/or one or more non-natural amino acids, including amino acids in the "D" isomeric form, provided that it is recognised by the corresponding T cell. Thus, the peptide may be a peptide 'mimetic' ie peptidomimetic which mimics the structural features of any of the peptides mentioned above. For example, the T cell antigen peptide may be a retro-inverso peptide.

Similarly, the T cell antigen peptide may be a mimotope, ie a peptide composed of natural or non-natural amino acids that mimics the structure of the natural epitope. Mimotopes often stimulate T cells more potently.

Preferably, the T cell antigen peptides are substantially non-toxic in the absence of T lymphocytes. By 'substantially non-toxic' we mean that the antigens have considerably lower or preferably no detectable toxicity, compared to toxins such as Pseudomonas exotoxin.

The skilled person will be able to identify further T cell antigen peptides that may be used in the invention using the database available at http://www.immuneepitope.org (Vita R, Zarebski L, Greenbaum JA, Emami H, Hoof I, Salimi N, Damle R, Sette A, Peters B. The immune epitope database 2.0. Nucleic Acids Res. 2010 Jan; 38(Database issue):D854-62. Epub 2009 Nov 11).

In one embodiment, the cyclic peptide comprises more than one T cell antigen peptide, such as 2, 3, 4 or 5 T cell antigens. Having more than one T cell antigen peptide may be desirable to make the agent of the invention more relevant to a higher proportion of the patient population. For instance, the agent may contain a range of T cell antigen peptides that bind to different types of MHC molecule. Having a combination of HLA-A*0101, HLA-A*0201, HLA-A*0301 and HLA-A*1101 restricted antigens within the agent, for example, would effectively cover 90% of the human population. The agent may contain multiple T cell antigen peptides, some of which bind to MHC Class I molecules and some of which bind to MHC Class II molecules. Having both Class I and Class II type T cell antigen peptides may be advantageous as Class II type T cell antigen peptides are known to act as helpers for Class I type T cell antigen peptides. In this embodiment, it may be convenient for the cyclic peptide to contain cleavage sites between each of the peptide antigens, as is described further below.

### Unwanted cell

The unwanted cell may be any cell whose presence in a host is undesired. Thus, the cell may be a tumour cell (benign or malignant), a cell from a tumour microenvironment such as tumour fibroblasts or tumour blood vessels, a virally infected cell, a cell introduced as part of gene therapy, or a normal cell which one wishes to destroy for a particular reason. For instance, it may be desirable to eliminate a subpopulation of immune cells such as T lymphocytes in autoimmune disease or such as B lymphocytes in allergic disease.

By a 'condition characterised by the presence of unwanted cells' we include any biological or medical condition or disorder in which at least part of the pathology is mediated by the presence of unwanted cells. The condition may be caused by the presence of the unwanted cells or else the presence of the unwanted cells may be an effect of the condition. Examples of particular conditions include tumours (benign or malignant), autoimmune conditions, cardiovascular diseases, degenerative diseases, diabetes, allergic disease (eg asthma), neurodegenerative diseases such as Alzheimer's, transplantation patients and infectious diseases. It will be appreciated that the agent also has utility in regenerative medicine (eg laboratory grown organs or tissues). It is particularly preferred if the condition is a tumour (eg a malignant disease) and the unwanted cells are tumour cells or tumour associated tissue.

For autoimmune disease, the unwanted cells may represent cells of the adaptive or innate immune response, preferably T cells, but more preferably B cells. For cardiovascular disease, the unwanted cells may represent cells within atheromatous lesions such as macrophages. For degenerative diseases, the unwanted cells may represent cells which induce the neurodegenerative changes, for instance in Alzheimer's disease they may be microglia or astrocytes. For other degenerative diseases any cell which facilitates the process of degeneration or apoptosis may be considered a target. For processes such as aging where unwanted tissue builds up, for example in benign prostatic hyperplasis, non-malignant prostatic tissue would be a preferred target. For allergic disease, cells which participate in the allergic reaction such as tissue mast cells may be considered an ideal target, but also IgE secreting cells such as plasma cells or B cells. In transplantation, alloreactive lymphocytes would represent a preferred target cell. In the context of infectious disease, any cell harbouring a virus, bacteria or fungal pathogen may be considered a preferred target cell for example an HIV infected cell.

In an embodiment the unwanted cell is not an antigen presenting cell such as a professional antigen presenting cell with cross-presentation capability.

In a further embodiment, the unwanted cell is not a cancer cell.

### Selective cleavage

By "in cyclised form the T cell antigen peptide is not capable of eliciting a T cell response", we include the meaning that when contained within the cyclic peptide, prior to selective cleavage of one or more cleavage sites within the cyclic peptide, the T cell antigen peptide is unable to elicit a T cell response. For example, the T cell antigen peptide may be unable to bind to an MHC molecule when contained within the cyclic peptide and/or the T cell antigen peptide may be unable to be recognised by a T cell receptor. Assessing whether the T cell antigen peptide is capable of eliciting a T cell response can be done by contacting isolated T cells (eg isolated peripheral mononuclear blood cells from a subject) known to be responsive to that T cell antigen peptide, with the cyclic peptide containing the T cell antigen peptide, and using standard assays for cell proliferation known in the art.

By "the T cell antigen peptide is rendered capable of eliciting a T cell response by selective cleavage of one or more cleavage sites in the cyclic peptide", we include the meaning that, following selective cleavage of one or more cleavage sites in the cyclic peptide, the T cell antigen peptide is now able to elicit a T cell response. For example, cleavage of the one or more cleavage sites may linearise the T cell antigen peptide such that it is now able to elicit a T cell response. The cleavage may allow the T cell antigen peptide to bind to a MHC molecule and/or be recognised by a T cell receptor, whereas previously it could not. Hence, whether cleavage renders a T cell antigen peptide capable of eliciting a T cell response can be determined by assessing the presence of a T cell response before and after cleavage, as described above.

It will be appreciated that cleavage of one or more cleavage sites in the cyclic peptide may simply linearise a previously cyclised T cell antigen peptide, or result in the detachment of the T cell antigen peptide from the cyclic peptide. For example, cleavage of the one or more cleavage sites may release the T cell antigen peptide from the cyclic structure so that it is free to elicit a T cell immune response.

In one embodiment, the cyclic peptide comprising a T cell antigen peptide may be a cyclic peptide that consists of a peptide T cell antigen peptide that is arranged in a cyclic configuration. By being arranged in a cyclic configuration, the T cell antigen peptide is prevented from being able to elicit a T cell response (eg it may be unable to bind to an MHC molecule and/or be unable to be recognised by a T cell receptor). However, upon selective cleavage of one or more cleavage sites in the cyclic peptide, the T cell antigen peptide is converted from a cyclic form to a linear form.

In another embodiment, the cyclic peptide comprising a T cell antigen peptide may be a cyclic peptide that contains a T cell antigen peptide and one or more other moieties (eg linker moieties) to form a ring structure. In this case, selective cleavage of one or more cleavage sites in the cyclic peptide may release the T cell antigen peptide from the one or more other moieties. For example, the T cell antigen peptide may be flanked by two cleavage sites and their cleavage results in the detachment of the T cell antigen peptide from the one or more other moieties. Alternatively, selective cleavage of one or more cleavage sites in the cyclic peptide may open the ring structure without detaching the T cell antigen peptide from the one or more other moieties. In each case, however, selective cleavage of the one or more cleavage sites must be able to render the T cell antigen peptide capable of eliciting a T cell response. For larger linking groups, it is preferred if the T cell antigen peptide is detached from them completely, following selective cleavage.

By "selective cleavage", we include the meaning of the cleavage site being cleavable only under selected conditions. In other words, the one or more cleavage sites may be cleavable in a directed manner under controlled conditions. For example, the one or more cleavage sites may be ones that are recognised by a particular enzyme (eg proteases recognising particular peptide sequences) and their cleavage is by selective enzymatic cleavage.

Preferably, the selective cleavage is such that the one or more cleavage sites are cleaved only in the vicinity of unwanted cells. In other words, the one or more cleavage sites are cleavable selectively in the vicinity of the unwanted cells.

By "cleavage site that is cleavable selectively in the vicinity of the unwanted cells" we include the meaning of a site that can only be cleaved by a molecule which resides selectively in the vicinity of the unwanted cells, so as to render the T cell antigen peptide capable of eliciting a T cell response. Preferably, the molecule that cleaves the cleavage site resides in the vicinity of the unwanted cells at a concentration at least five times or ten times higher than the concentration of the molecule outside the vicinity of the unwanted cells, and more preferably at a concentration at least 100 or 500 or 1000 times higher. Most preferably, the molecule that cleaves the cleavage site is found only in the vicinity of the unwanted cells. For example, when the unwanted cells are particular tumour cells (e.g. breast tumour cells), the cleavage site may be one that is cleaved by a molecule which resides selectively in the particular tumour (e.g. breast tumour) but which molecule does not reside outside the vicinity of the particular tumour (e.g. breast tumour).

It will be appreciated that selective cleavage of the one or more cleavage sites (and therefore rendering the T cell antigen peptide capable of eliciting a T cell response) in the vicinity of normal cells may be tolerated if those normal cells can be functionally replaced by other therapeutic means or if they are not essential to life. Thus, selective cleavage of one or more cleavage sites that occurs in the vicinity of cancer cells as well as, for example, in the vicinity of endocrine tissues or organs, is not precluded. In this case, the agent of the invention acts to redirect an immune response to both unwanted cells and to other cells that can be functionally replaced by therapeutic means. In a life-saving situation for example, the tissue or organ may be sacrificed provided its function was either not essential to life, for instance in the case of the testes, prostate or pancreas, or could be supplied by hormone replacement therapy. Such considerations would apply to the thyroid gland, parathyroids, adrenal cortex and ovaries, for example. It follows that the one or more cleavage sites may be selectively cleavable in the vicinity of unwanted cells as opposed to wanted cells, wherein the unwanted cells may include cells whose presence in a host is undesired and optionally cells whose presence in the host is desired but whose presence can be functionally replaced by therapeutic means.

By 'in the vicinity of cells', we include the meaning of either at or near to the surface of the cells, or both, or in the environment that immediately surrounds the cells e.g. blood, lymph, and other body fluids. In a particularly preferred embodiment, the one or more cleavage sites are selectively cleaved outside of the unwanted cell, at or near its surface, so that the T cell antigen peptide can be presented by the unwanted cell to T cells without needing to be internalised.

The one or more cleavage sites are selectively cleaved in the vicinity of the unwanted cells so that the T cell antigen peptide is preferentially presented by unwanted cells rather than by wanted cells. Thus, it is preferred that the one or more cleavage sites are ones that are selectively cleaved, such that the T cell antigen peptide is rendered capable of eliciting a T cell response, in the vicinity of (e.g. at or near to the cell surface) the unwanted cells at least five times or ten times more than the extent to which they are cleaved in the vicinity of wanted cells, and more preferably at least 100 or 500 or 1000 times more. Most preferably, the one or more cleavage sites are not cleaved in the vicinity of wanted cells, and therefore the T cell antigen peptide is not rendered capable of eliciting a T cell response in the vicinity of wanted cells.

For a given unwanted cell, the skilled person will be able to identify an appropriate cleavage site that is selectively cleavable in the vicinity of the unwanted cell, using established methods in the art. For example, which proteases cleave which peptides can be assessed by consulting peptide libraries and studying an MS analysis of the fragmentation profile following cleavage. Also, published literature of protease cleavage motifs and peptide cleavage data can be searched as described further below. Gene expression and proteomic data may also be analysed to identify which proteases are expressed by particular unwanted cells.

By virtue of the one or more cleavage sites being selectively cleavable in the vicinity of the unwanted cells, the T cell antigen peptide is selectively rendered capable of eliciting a T cell response in the vicinity of the unwanted cells. The inventors believe that this allows the unwanted cells to present the T cell antigen peptide to T cells, thereby redirecting an existing immune response to the unwanted cells.

In a preferred embodiment, the one or more cleavage sites are ones that are cleaved in the vicinity of the unwanted cells and outside of the unwanted cells, for example at the cell surface. In this way, the T cell antigen peptide is rendered capable of eliciting a T cell response (eg by being able to bind to an MHC molecule and/or being capable of being recognised by a T cell receptor) in the vicinity of the external surface of the cell and can be presented to a T cell directly, without the need to be internalised and engage the appropriate processing pathways. Accordingly, the invention includes an agent for preventing or treating a condition characterised by the presence of unwanted cells, the agent comprising a cyclic peptide comprising a T cell antigen peptide, wherein in cyclised form the T cell antigen peptide is not capable of eliciting a T cell response, and wherein the T cell is rendered capable of eliciting a T cell response by selective cleavage of one or more cleavage sites in the cyclic peptide in the vicinity of and outside of the unwanted cells. Preferably, the T cell antigen peptide is one which does not generate a new primary T cell response for that antigen, but rather elicits an existing T cell response in a subject.

The inventors also believe, however, that the T cell antigen peptide may be rendered capable of eliciting a T cell response, inside the cell, without the need to undergo classical antigen processing. Thus, it will be understood that the one or more cleavage sites need not be cleaved at the surface of the cell but may be cleaved within the cell, for example via a receptor cycling pathway or in near-membrane compartments such as vesicles (e.g. pinocytic vesicles).

The one or more cleavage sites may be ones that are selectively cleavable by an enzyme such as any of a protease, a nuclease, a lipase, a lyase, a phosphatase or a carbohydrase, which may or may not be membrane bound. Thus, the T cell may be rendered capable of eliciting a T cell response by selective enzymatic cleavage of one or more enzymatic cleavage sites.

Preferably, the cleavage site is a protease cleavage site (eg one that resides within the T cell antigen peptide or within a peptide linking moiety within the cyclic peptide). Hence, when the unwanted cells are tumour cells, the one or more cleavage sites may be cleavable selectively by proteases that reside in the vicinity of the tumour cells. In other words, one or more protease cleavage sites may be ones that are cleavable by a tumour associated protease. It is well known that during tumour development, tumours aberrantly express proteases which allow them to invade local tissues and eventually metastasise.

The protease may include any of a cysteine protease (including the Cathepsin family B, L, S etc), an aspartyl protease (including Cathepsin D and E) and a serine protease (including Cathepsin A and G, Thrombin, Plasmin, Urokinase, Tissue Plasminogen Activator). The protease may be a metalloproteinase (MMP1-28) including both membrane bound (MMP14-17 and MMP24-25) and secreted forms (MMP1-13 and MMP18-23 and MMP26-28). The protease may belong to the A Disintegrin and Metalloproteinase (ADAM) and A Disintegrin, or Metalloproteinase with Thrombospondin Motifs (ADAMTS) families of proteases. Other examples include CD10 (CALLA) and prostate specific antigen (PSA). It is appreciated that the proteases may or may not be membrane bound.

Protease cleavage sites are well known in the scientific literature, and linker sequences comprising such cleavage sites can be readily constructed using established genetic engineering techniques, or by synthetic synthesis techniques known in the art. Such linker sequences may be used to configure the T cell antigen peptide into a cyclic peptide as described further below.

The protease cleavage site may be one that is cleavable by any of the proteases listed in Table 4 below, which indicates the expression of selected proteases in various tumour types. Candidate substrates for the proteases are provided. Thus, in order to treat a particular tumour type, the skilled person will typically select one or more protease cleavage sites that are selectively cleaved by proteases known to be highly expressed in that tumour type, as seen from the table. For example, to treat breast cancer, it is preferred to use one or more protease cleavage sites cleavable by any of uPA, tPA, matriptase, matriptase 2, Cathepsin K, Cathepsin O, MMP1, MMP2, MMP3, MMP11, MMP12, MMP17, ADAM9, ADAM12, ADAM15, ADAM17, ADAM28 or ADAMTS15, and so on.

Similarly, Table 5 lists tumour sites in which ADAM protease overexpression has been reported, and so in an embodiment, the one or more cleavage sites are ones that are selectively cleavable by one of the ADAM proteases listed in Table 5. Accordingly, the agent may be used to prevent or treat the corresponding tumour type.

The cleavage site may be selectively cleavable by any of the following human proteases (MEROPS peptidase database number provided in parentheses; Rawlings N.D., Morton F.R., Kok, C.Y., Kong, J. & Barrett A.J. (2008) MEROPS: the peptidase database. Nucleic Acids Res. 36 Database issue, D320-325) : pepsin A (MER000885), gastricsin (MER000894), memapsin-2 (MER005870), renin (MER000917), cathepsin D (MER000911), cathepsin E (MER000944), memapsin-1 (MER005534), napsin A (MER004981), Mername-AA034 peptidase (MER014038), pepsin A4 (MER037290), pepsin A5 (*Homo sapiens*) (MER037291), hCG1733572 *(Homo sapiens*)-type putative peptidase (MER107386), napsin B pseudogene (MER004982), CYMP g.p. (*Homo sapiens*) (MER002929), subfamily A1A unassigned peptidases (MER181559), mouse mammary tumor virus retropepsin (MER048030), rabbit endogenous retrovirus endopeptidase (MER043650), S71-related human endogenous retropepsin (MER001812), RTVL-H-type putative peptidase (MER047117), RTVL-H-type putative peptidase (MER047133), RTVL-H-type putative peptidase (MER047160), RTVL-H-type putative peptidase (MER047206), RTVL-H-type putative peptidase (MER047253), RTVL-H-type putative peptidase (MER047260), RTVL-H-type putative peptidase (MER047291), RTVL-H-type putative peptidase (MER047418), RTVL-H-type putative peptidase (MER047440), RTVL-H-type putative peptidase (MER047479). RTVL-H-type putative peptidase (MER047559), RTVL-H-type putative peptidase (MER047583). RTVL-H-type putative peptidase (MER015446), human endogenous retrovirus retropepsin homologue 1 (MER015479), human endogenous retrovirus retropepsin homologue 2 (MER015481), endogenous retrovirus retropepsin pseudogene 1 (*Homo sapiens* chromosome 14) (MER029977), endogenous retrovirus retropepsin pseudogene 2 (*Homo sapiens* chromosome 8) (MER029665), endogenous retrovirus retropepsin pseudogene 3 (*Homo sapiens* chromosome 17) (MER002660), endogenous retrovirus retropepsin pseudogene 3 *(Homo sapiens* chromosome 17) (MER030286), endogenous retrovirus retropepsin pseudogene 3 *(Homo sapiens* chromosome 17) (MER047144). endogenous retrovirus retropepsin pseudogene 5 *(Homo sapiens* chromosome 12) (MER029664). endogenous retrovirus retropepsin pseudogene 6 *(Homo sapiens* chromosome 7) (MER002094), endogenous retrovirus retropepsin pseudogene 7 (*Homo sapiens* chromosome 6) (MER029776), endogenous retrovirus retropepsin pseudogene 8 *(Homo sapiens* chromosome Y) (MER030291), endogenous retrovirus retropepsin pseudogene 9 *(Homo sapiens* chromosome 19) (MER029680), endogenous retrovirus retropepsin pseudogene 10 *(Homo sapiens* chromosome 12) (MER002848), endogenous retrovirus retropepsin pseudogene 11 *(Homo sapiens* chromosome 17) (MER004378), endogenous retrovirus retropepsin pseudogene 12 *(Homo sapiens* chromosome 11) (MER003344), endogenous retrovirus retropepsin pseudogene 13 (*Homo sapiens* chromosome 2 and similar) (MER029779), endogenous retrovirus retropepsin pseudogene 14 (*Homo sapiens* chromosome 2) (MER029778), endogenous retrovirus retropepsin pseudogene 15 (*Homo sapiens* chromosome 4) (MER047158), endogenous retrovirus retropepsin pseudogene 15 (*Homo sapiens* chromosome 4) (MER047332), endogenous retrovirus retropepsin pseudogene 15 (*Homo sapiens* chromosome 4) (MER003182), endogenous retrovirus retropepsin pseudogene 16 (MER047165), endogenous retrovirus retropepsin pseudogene 16 (MER047178), endogenous retrovirus retropepsin pseudogene 16 (MER047200), endogenous retrovirus retropepsin pseudogene 16 (MER047315), endogenous retrovirus retropepsin pseudogene 16 (MER047405), endogenous retrovirus retropepsin pseudogene 16 (MER030292), endogenous retrovirus retropepsin pseudogene 17 (*Homo sapiens* chromosome 8) (MER005305), endogenous retrovirus retropepsin pseudogene 18 (*Homo sapiens* chromosome 4) (MER030288), endogenous retrovirus retropepsin pseudogene 19 (*Homo sapiens* chromosome 16) (MER001740), endogenous retrovirus retropepsin pseudogene 21 (*Homo sapiens*) (MER047222), endogenous retrovirus retropepsin pseudogene 21 (*Homo sapiens*) (MER047454), endogenous retrovirus retropepsin pseudogene 21 (*Homo sapiens*) (MER047477), endogenous retrovirus retropepsin pseudogene 21 (*Homo sapiens*) (MER004403), endogenous retrovirus retropepsin pseudogene 22 (*Homo sapiens* chromosome X) (MER030287), subfamily A2A non-peptidase homologues (MER047046), subfamily A2A non-peptidase homologues (MER047052), subfamily A2A non-peptidase homologues (MER047076), subfamily A2A non-peptidase homologues (MER047080), subfamily A2A non-peptidase homologues (MER047088), subfamily A2A non-peptidase homologues (MER047089), subfamily A2A non-peptidase homologues (MER047091), subfamily A2A non-peptidase homologues (MER047092), subfamily A2A non-peptidase homologues (MER047093), subfamily A2A non-peptidase homologues (MER047094), subfamily A2A non-peptidase homologues (MER047097), subfamily A2A non-peptidase homologues (MER047099), subfamily A2A non-peptidase homologues (MER047101), subfamily A2A non-peptidase homologues (MER047102), subfamily A2A non-peptidase homologues (MER047107), subfamily A2A non-peptidase homologues (MER047108), subfamily A2A non-peptidase homologues (MER047109), subfamily A2A non-peptidase homologues (MER047110), subfamily A2A non-peptidase homologues (MER047111), subfamily A2A non-peptidase homologues (MER047114), subfamily A2A non-peptidase homologues (MER047118), subfamily A2A non-peptidase homologues (MER047121), subfamily A2A non-peptidase homologues (MER047122), subfamily A2A non-peptidase homologues (MER047126), subfamily A2A non-peptidase homologues (MER047129), subfamily A2A non-peptidase homologues (MER047130), subfamily A2A non-peptidase homologues (MER047134), subfamily A2A non-peptidase homologues (MER047135), subfamily A2A non-peptidase homologues (MER047137), subfamily A2A non-peptidase homologues (MER047140), subfamily A2A non-peptidase homologues (MER047141), subfamily A2A non-peptidase homologues (MER047142), subfamily A2A non-peptidase homologues (MER047148), subfamily A2A non-peptidase homologues (MER047149), subfamily A2A non-peptidase homologues (MER047151), subfamily A2A non-peptidase homologues (MER047154), subfamily A2A non-peptidase homologues (MER047155), subfamily A2A non-peptidase homologues (MER047156), subfamily A2A non-peptidase homologues (MER047157), subfamily A2A non-peptidase homologues (MER047159), subfamily A2A non-peptidase homologues (MER047161), subfamily A2A non-peptidase homologues (MER047163), subfamily A2A non-peptidase homologues (MER047166), subfamily A2A non-peptidase homologues (MER047171), subfamily A2A non-peptidase homologues (MER047173), subfamily A2A non-peptidase homologues (MER047174), subfamily A2A non-peptidase homologues (MER047179), subfamily A2A non-peptidase homologues (MER047183), subfamily A2A non-peptidase homologues (MER047186), subfamily A2A non-peptidase homologues (MER047190), subfamily A2A non-peptidase homologues (MER047191), subfamily A2A non-peptidase homologues (MER047196), subfamily A2A non-peptidase homologues (MER047198), subfamily A2A non-peptidase homologues (MER047199), subfamily A2A non-peptidase homologues (MER047201), subfamily A2A non-peptidase homologues (MER047202), subfamily A2A non-peptidase homologues (MER047203), subfamily A2A non-peptidase homologues (MER047204), subfamily A2A non-peptidase homologues (MER047205), subfamily A2A non-peptidase homologues (MER047207), subfamily A2A non-peptidase homologues (MER047208), subfamily A2A non-peptidase homologues (MER047210), subfamily A2A non-peptidase homologues (MER047211), subfamily A2A non-peptidase homologues (MER047212), subfamily A2A non-peptidase homologues (MER047213), subfamily A2A non-peptidase homologues (MER047215), subfamily A2A non-peptidase homologues (MER047216), subfamily A2A non-peptidase homologues (MER047218), subfamily A2A non-peptidase homologues (MER047219), subfamily A2A non-peptidase homologues (MER047221), subfamily A2A non-peptidase homologues (MER047224), subfamily A2A non-peptidase homologues (MER047225), subfamily A2A non-peptidase homologues (MER047226), subfamily A2A non-peptidase homologues (MER047227), subfamily A2A non-peptidase homologues (MER047230), subfamily A2A non-peptidase homologues (MER047232), subfamily A2A non-peptidase homologues (MER047233), subfamily A2A non-peptidase homologues (MER047234), subfamily A2A non-peptidase homologues (MER047236), subfamily A2A non-peptidase homologues (MER047238), subfamily A2A non-peptidase homologues (MER047239), subfamily A2A non-peptidase homologues (MER047240), subfamily A2A non-peptidase homologues (MER047242), subfamily A2A non-peptidase homologues (MER047243), subfamily A2A non-peptidase homologues (MER047249), subfamily A2A non-peptidase homologues (MER047251), subfamily A2A non-peptidase homologues (MER047252), subfamily A2A non-peptidase homologues (MER047254), subfamily A2A non-peptidase homologues (MER047255), subfamily A2A non-peptidase homologues (MER047263), subfamily A2A non-peptidase homologues (MER047265), subfamily A2A non-peptidase homologues (MER047266), subfamily A2A non-peptidase homologues (MER047267), subfamily A2A non-peptidase homologues (MER047268), subfamily A2A non-peptidase homologues (MER047269), subfamily A2A non-peptidase homologues (MER047272), subfamily A2A non-peptidase homologues (MER047273), subfamily A2A non-peptidase homologues (MER047274), subfamily A2A non-peptidase homologues (MER047275), subfamily A2A non-peptidase homologues (MER047276), subfamily A2A non-peptidase homologues (MER047279), subfamily A2A non-peptidase homologues (MER047280), subfamily A2A non-peptidase homologues (MER047281), subfamily A2A non-peptidase homologues (MER047282), subfamily A2A non-peptidase homologues (MER047284), subfamily A2A non-peptidase homologues (MER047285), subfamily A2A non-peptidase homologues (MER047289), subfamily A2A non-peptidase homologues (MER047290), subfamily A2A non-peptidase homologues (MER047294), subfamily A2A non-peptidase homologues (MER047295), subfamily A2A non-peptidase homologues (MER047298), subfamily A2A non-peptidase homologues (MER047300), subfamily A2A non-peptidase homologues (MER047302), subfamily A2A non-peptidase homologues (MER047304), subfamily A2A non-peptidase homologues (MER047305), subfamily A2A non-peptidase homologues (MER047306), subfamily A2A non-peptidase homologues (MER047307), subfamily A2A non-peptidase homologues (MER047310), subfamily A2A non-peptidase homologues (MER047311), subfamily A2A non-peptidase homologues (MER047314), subfamily A2A non-peptidase homologues (MER047318), subfamily A2A non-peptidase homologues (MER047320), subfamily A2A non-peptidase homologues (MER047321), subfamily A2A non-peptidase homologues (MER047322), subfamily A2A non-peptidase homologues (MER047326), subfamily A2A non-peptidase homologues (MER047327), subfamily A2A non-peptidase homologues (MER047330), subfamily A2A non-peptidase homologues (MER047333), subfamily A2A non-peptidase homologues (MER047362), subfamily A2A non-peptidase homologues (MER047366), subfamily A2A non-peptidase homologues (MER047369), subfamily A2A non-peptidase homologues (MER047370), subfamily A2A non-peptidase homologues (MER047371), subfamily A2A non-peptidase homologues (MER047375), subfamily A2A non-peptidase homologues (MER047376), subfamily A2A non-peptidase homologues (MER047381), subfamily A2A non-peptidase homologues (MER047383), subfamily A2A non-peptidase homologues (MER047384), subfamily A2A non-peptidase homologues (MER047385), subfamily A2A non-peptidase homologues (MER047388), subfamily A2A non-peptidase homologues (MER047389), subfamily A2A non-peptidase homologues (MER047391), subfamily A2A non-peptidase homologues (MER047394), subfamily A2A non-peptidase homologues (MER047396), subfamily A2A non-peptidase homologues (MER047400), subfamily A2A non-peptidase homologues (MER047401), subfamily A2A non-peptidase homologues (MER047403), subfamily A2A non-peptidase homologues (MER047406), subfamily A2A non-peptidase homologues (MER047407), subfamily A2A non-peptidase homologues (MER047410), subfamily A2A non-peptidase homologues (MER047411), subfamily A2A non-peptidase homologues (MER047413), subfamily A2A non-peptidase homologues (MER047414), subfamily A2A non-peptidase homologues (MER047416), subfamily A2A non-peptidase homologues (MER047417), subfamily A2A non-peptidase homologues (MER047420), subfamily A2A non-peptidase homologues (MER047423), subfamily A2A non-peptidase homologues (MER047424), subfamily A2A non-peptidase homologues (MER047428), subfamily A2A non-peptidase homologues (MER047429), subfamily A2A non-peptidase homologues (MER047431), subfamily A2A non-peptidase homologues (MER047434), subfamily A2A non-peptidase homologues (MER047439), subfamily A2A non-peptidase homologues (MER047442), subfamily A2A non-peptidase homologues (MER047445), subfamily A2A non-peptidase homologues (MER047449), subfamily A2A non-peptidase homologues (MER047450), subfamily A2A non-peptidase homologues (MER047452), subfamily A2A non-peptidase homologues (MER047455), subfamily A2A non-peptidase homologues (MER047457), subfamily A2A non-peptidase homologues (MER047458), subfamily A2A non-peptidase homologues (MER047459), subfamily A2A non-peptidase homologues (MER047463), subfamily A2A non-peptidase homologues (MER047468), subfamily A2A non-peptidase homologues (MER047469), subfamily A2A non-peptidase homologues (MER047470), subfamily A2A non-peptidase homologues (MER047476), subfamily A2A non-peptidase homologues (MER047478), subfamily A2A non-peptidase homologues (MER047483), subfamily A2A non-peptidase homologues (MER047488), subfamily A2A non-peptidase homologues (MER047489), subfamily A2A non-peptidase homologues (MER047490), subfamily A2A non-peptidase homologues (MER047493), subfamily A2A non-peptidase homologues (MER047494), subfamily A2A non-peptidase homologues (MER047495), subfamily A2A non-peptidase homologues (MER047496), subfamily A2A non-peptidase homologues (MER047497), subfamily A2A non-peptidase homologues (MER047499), subfamily A2A non-peptidase homologues (MER047502), subfamily A2A non-peptidase homologues (MER047504), subfamily A2A non-peptidase homologues (MER047511), subfamily A2A non-peptidase homologues (MER047513), subfamily A2A non-peptidase homologues (MER047514), subfamily A2A non-peptidase homologues (MER047515), subfamily A2A non-peptidase homologues (MER047516), subfamily A2A non-peptidase homologues (MER047520), subfamily A2A non-peptidase homologues (MER047533), subfamily A2A non-peptidase homologues (MER047537), subfamily A2A non-peptidase homologues (MER047569), subfamily A2A non-peptidase homologues (MER047570), subfamily A2A non-peptidase homologues (MER047584), subfamily A2A non-peptidase homologues (MER047603), subfamily A2A non-peptidase homologues (MER047604), subfamily A2A non-peptidase homologues (MER047606), subfamily A2A non-peptidase homologues (MER047609), subfamily A2A non-peptidase homologues (MER047616), subfamily A2A non-peptidase homologues (MER047619), subfamily A2A non-peptidase homologues (MER047648), subfamily A2A non-peptidase homologues (MER047649), subfamily A2A non-peptidase homologues (MER047662), subfamily A2A non-peptidase homologues (MER048004), subfamily A2A non-peptidase homologues (MER048018), subfamily A2A non-peptidase homologues (MER048019), subfamily A2A non-peptidase homologues (MER048023), subfamily A2A non-peptidase homologues (MER048037), subfamily A2A unassigned peptidases (MER047164), subfamily A2A unassigned peptidases (MER047231), subfamily A2A unassigned peptidases (MER047386), skin aspartic protease (MER057097), presenilin 1 (MER005221), presenilin 2 (MER005223), impas 1 peptidase (MER019701), impas 1 peptidase (MER184722), impas 4 peptidase (MER019715), impas 2 peptidase (MER019708), impas 5 peptidase (MER019712), impas 3 peptidase (MER019711), possible family A22 pseudogene (*Homo sapiens* chromosome 18) (MER029974), possible family A22 pseudogene (*Homo sapiens* chromosome 11) (MER023159), cathepsin V (MER004437), cathepsin X (MER004508), cathepsin F (MER004980), cathepsin L (MER000622), cathepsin S (MER000633), cathepsin O (MER001690), cathepsin K (MER000644), cathepsin W (MER003756), cathepsin H (MER000629), cathepsin B (MER000686), ipeptidyl-peptidase I (MER001937), bleomycin hydrolase (animal) (MER002481), tubulointerstitial nephritis antigen (MER016137), tubulointerstitial nephritis antigen-related protein (MER021799), cathepsin L-like pseudogene 1 *(Homo sapiens*) (MER002789), cathepsin B-like pseudogene (chromosome 4, *Homo sapiens*) (MER029469), cathepsin B-like pseudogene (chromosome 1, *Homo sapiens*) (MER029457), CTSLL2 g.p. (*Homo sapiens*) (MER005210), CTSLL3 g.p. *(Homo sapiens*) (MER005209), calpain-1 (MER000770), calpain-2 (MER000964), calpain-3 (MER001446), calpain-9 (MER004042), calpain-8 (MER021474), calpain-15 (MER004745), calpain-5 (MER002939), calpain-11 (MER005844), calpain-12 (MER029889), calpain-10 (MER013510), calpain-13 (MER020139), calpain-14 (MER029744), Mername-AA253 peptidase (MER005537), calpamodulin (MER000718), hypothetical protein flj40251 (MER003201), ubiquitinyl hydrolase-L1 (MER000832), ubiquitinyl hydrolase-L3 (MER000836), ubiquitinyl hydrolase-BAP1 (MER003989), ubiquitinyl hydrolase-UCH37 (MER005539), ubiquitin-specific peptidase 5 (MER002066), ubiquitin-specific peptidase 6 (MER000863), ubiquitin-specific peptidase 4 (MER001795), ubiquitin-specific peptidase 8 (MER001884), ubiquitin-specific peptidase 13 (MER002627), ubiquitin-specific peptidase 2 (MER004834), ubiquitin-specific peptidase 11 (MER002693), ubiquitin-specific peptidase 14 (MER002667), ubiquitin-specific peptidase 7 (MER002896), ubiquitin-specific peptidase 9X (MER005877), ubiquitin-specific peptidase 10 (MER004439), ubiquitin-specific peptidase 1 (MER004978), ubiquitin-specific peptidase 12 (MER005454), ubiquitin-specific peptidase 16 (MER005493), ubiquitin-specific peptidase 15 (MER005427), ubiquitin-specific peptidase 17 (MER002900), ubiquitin-specific peptidase 19 (MER005428), ubiquitin-specific peptidase 20 (MER005494), ubiquitin-specific peptidase 3 (MER005513). ubiquitin-specific peptidase 9Y (MER004314), ubiquitin-specific peptidase 18 (MER005641), ubiquitin-specific peptidase 21 (MER006258), ubiquitin-specific peptidase 22 (MER012130), ubiquitin-specific peptidase 33 (MER014335), ubiquitin-specific peptidase 29 (MER012093), ubiquitin-specific peptidase 25 (MER011115), ubiquitin-specific peptidase 36 (MER014033), ubiquitin-specific peptidase 32 (MER014290), ubiquitin-specific peptidase 26 (*Homo sapiens-*type) (MER014292), ubiquitin-specific peptidase 24 (MER005706), ubiquitin-specific peptidase 42 (MER011852), ubiquitin-specific peptidase 46 (MER014629), ubiquitin-specific peptidase 37 (MER014633), ubiquitin-specific peptidase 28 (MER014634), ubiquitin-specific peptidase 47 (MER014636), ubiquitin-specific peptidase 38 (MER014637), ubiquitin-specific peptidase 44 (MER014638), ubiquitin-specific peptidase 50 (MER030315), ubiquitin-specific peptidase 35 (MER014646), ubiquitin-specific peptidase 30 (MER014649), Mername-AA091 peptidase (MER014743), ubiquitin-specific peptidase 45 (MER030314), ubiquitin-specific peptidase 51 (MER014769), ubiquitin-specific peptidase 34 (MER014780), ubiquitin-specific peptidase 48 (MER064620), ubiquitin-specific peptidase 40 (MER015483), ubiquitin-specific peptidase 41 (MER045268), ubiquitin-specific peptidase 31 (MER015493), Mername-AA129 peptidase (MER016485), ubiquitin-specific peptidase 49 (MER016486), Mername-AA187 peptidase (MER052579), USP17-like peptidase (MER030192), ubiquitin-specific peptidase 54 (MER028714), ubiquitin-specific peptidase 53 (MER027329), ubiquitin-specific endopeptidase 39 [misleading] (MER064621), Mername-AA090 non-peptidase homologue (MER014739), ubiquitin-specific peptidase 43 [misleading] (MER030140), ubiquitin-specific peptidase 52 [misleading] (MER030317), NEK2 pseudogene (MER014736), C19 pseudogene (*Homo sapiens*: chromosome 5) (MER029972), Mername-AA088 peptidase (MER014750), autophagin-2 (MER013564), autophagin-1 (MER013561), autophagin-3 (MER014316), autophagin-4 (MER064622), Cezanne deubiquitinylating peptidase (MER029042), Cezanne-2 peptidase (MER029044), tumor necrosis factor alpha-induced protein 3 (MER029050), trabid peptidase (MER029052), VCIP135 deubiquitinating peptidase (MER152304), otubain-1 (MER029056), otubain-2 (MER029061), CylD protein (MER030104), UfSP1 peptidase (MER042724), UfSP2 peptidase (MER060306), DUBA deubiquitinylating enzyme (MER086098), KIAA0459 (*Homo sapiens*)-like protein (MER122467), Otud1 protein (MER125457), glycosyltransferase 28 domain containing 1, isoform CRA_c (*Homo sapiens*)-like (MER123606), hin1L g.p. (*Homo sapiens*) (MER139816), ataxin-3 (MER099998), ATXN3L putative peptidase (MER115261), Josephin domain containing 1 (*Homo sapiens*) (MER125334), Josephin domain containing 2 (*Homo sapiens*) (MER124068), YOD1 peptidase (MER116559), legumain (plant alpha form) (MER044591), legumain (MER001800), glycosylphosphatidylinositol:protein transamidase (MER002479), legumain pseudogene (*Homo sapiens*) (MER029741), family C13 unassigned peptidases (MER175813), caspase-1 (MER000850), caspase-3 (MER000853), caspase-7 (MER002705), caspase-6 (MER002708), caspase-2 (MER001644), caspase-4 (MER001938), caspase-5 (MER002240), caspase-8 (MER002849), caspase-9 (MER002707), caspase-10 (MER002579), caspase-14 (MER012083), paracaspase (MER019325), Mername-AA143 peptidase (MER021304), Mername-AA186 peptidase (MER020516), putative caspase (*Homo sapiens*) (MER021463), FLIP protein (MER003026), Mername-AA142 protein (MER021316), caspase-12 pseudogene (*Homo sapiens*) (MER019698), Mername-AA093 caspase pseudogene (MER014766), subfamily C14A non-peptidase homologues (MER185329), subfamily C14A non-peptidase homologues (MER179956), separase (*Homo sapiens-*type) (MER011775), separase-like pseudogene (MER014797), SENP1 peptidase (MER011012), SENP3 peptidase (MER011019), SENP6 peptidase (MER011109), SENP2 peptidase (MER012183), SENP5 peptidase (MER014032), SENP7 peptidase (MER014095), SENP8 peptidase (MER016161), SENP4 peptidase (MER005557), pyroglutamyl-peptidase I (chordate) (MER011032), Mername-AA073 peptidase (MER029978), Sonic hedgehog protein (MER002539), Indian hedgehog protein (MER002538), Desert hedgehog protein (MER012170), dipeptidyl-peptidase III (MER004252), Mername-AA164 protein (MER020410), LOC138971 g.p. (*Homo sapiens*) (MER020074), Atp23 peptidase (MER060642), prenyl peptidase 1 (MER004246), aminopeptidase N (MER000997), aminopeptidase A (MER001012), leukotriene A4 hydrolase (MER001013), pyroglutamyl-peptidase II (MER012221), cytosol alanyl aminopeptidase (MER002746), cystinyl aminopeptidase (MER002060), aminopeptidase B (MER001494), aminopeptidase PILS (MER005331), arginyl aminopeptidase-like 1 (MER012271), leukocyte-derived arginine aminopeptidase (MER002968), aminopeptidase Q (MER052595), aminopeptidase O (MER019730), Tata binding protein associated factor (MER026493), angiotensin-converting enzyme peptidase unit 1 (MER004967), angiotensin-converting enzyme peptidase unit 2 (MER001019), angiotensin-converting enzyme-2 (MER011061), Mername-AA153 protein (MER020514), thimet oligopeptidase (MER001737), neurolysin (MER010991), mitochondrial intermediate peptidase (MER003665), Mername-AA154 protein (MER021317), leishmanolysin-2 (MER014492), leishmanolysin-3 (MER180031), matrix metallopeptidase-1 (MER001063), matrix metallopeptidase-8 (MER001084), matrix metallopeptidase-2 (MER001080), matrix metallopeptidase-9 (MER001085), matrix metallopeptidase-3 (MER001068), matrix metallopeptidase-10 (*Homo sapiens-type*) (MER001072), matrix metallopeptidase-11 (MER001075), matrix metallopeptidase-7 (MER001092), matrix metallopeptidase-12 (MER001089), matrix metallopeptidase-13 (MER001411), membrane-type matrix metallopeptidase-1 (MER001077), membrane-type matrix metallopeptidase-2 (MER002383), membrane-type matrix metallopeptidase-3 (MER002384), membrane-type matrix metallopeptidase-4 (MER002595), matrix metallopeptidase-20 (MER003021), matrix metallopeptidase-19 (MER002076), matrix metallopeptidase-23B (MER004766), membrane-type matrix metallopeptidase-5 (MER005638), membrane-type matrix metallopeptidase-6 (MER012071), matrix metallopeptidase-21 (MER006101), matrix metallopeptidase-22 (MER014098), matrix metallopeptidase-26 (MER012072), matrix metallopeptidase-28 (MER013587), matrix metallopeptidase-23A (MER037217), macrophage elastase homologue (chromosome 8, *Homo sapiens*) (MER030035), Mername-AA156 protein (MER021309), matrix metallopeptidase-like 1 (MER045280), subfamily M10A non-peptidase homologues (MER175912), subfamily M10A non-peptidase homologues (MER187997), subfamily M10A non-peptidase homologues (MER187998), subfamily M10A non-peptidase homologues (MER180000), meprin alpha subunit (MER001111), meprin beta subunit (MER005213), procollagen C-peptidase (MER001113), mammalian tolloid-like 1 protein (MER005124), mammalian-type tolloid-like 2 protein (MER005866), ADAMTS9 peptidase (MER012092), ADAMTS14 peptidase (MER016700), ADAMTS15 peptidase (MER017029), ADAMTS16 peptidase (MER015689), ADAMTS17 peptidase (MER016302), ADAMTS18 peptidase (MER016090), ADAMTS19 peptidase (MER015663), ADAM8 peptidase (MER003902), ADAM9 peptidase (MER001140), ADAM10 peptidase (MER002382), ADAM12 peptidase (MER005107), ADAM19 peptidase (MER012241), ADAM15 peptidase (MER002386), ADAM17 peptidase (MER003094), ADAM20 peptidase (MER004725), ADAMDEC1 peptidase (MER000743), ADAMTS3 peptidase (MER005100), ADAMTS4 peptidase (MER005101), ADAMTS1 peptidase (MER005546), ADAM28 peptidase (*Homo sapiens-type*) (MER005495), ADAMTS5 peptidase (MER005548), ADAMTS8 peptidase (MER005545), ADAMTS6 peptidase (MER005893).

ADAMTS7 peptidase (MER005894), ADAM30 peptidase (MER006268), ADAM21 peptidase (*Homo sapiens-type*) (MER004726), ADAMTS10 peptidase (MER014331), ADAMTS12 peptidase (MER014337), ADAPTS13 peptidase (MER015450), ADAM33 peptidase (MER015143), ovastacin (MER029996), ADAMTS20 peptidase (Homo *sapiens-*type) (MER026906), procollagen I N-peptidase (MER004985), ADAM2 protein (MER003090), ADAM6 protein (MER047044), ADAM7 protein (MER005109), ADAM18 protein (MER012230), ADAM32 protein (MER026938), non-peptidase homologue (Homo *sapiens* chromosome 4) (MER029973), family M12 non-peptidase homologue (Homo *sapiens* chromosome 16) (MER047654), family M12 non-peptidase homologue (Homo *sapiens* chromosome 15) (MER047250), ADAM3B protein *(Homo sapiens-*type) (MER005199), ADAM11 protein (MER001146), ADAM22 protein (MER005102), ADAM23 protein (MER005103), ADAM29 protein (MER006267), protein similar to ADAM21 peptidase preproprotein *(Homo sapiens*) (MER026944), Mername-AA225 peptidase homologue (*Homo sapiens*) (MER047474), putative ADAM pseudogene (chromosome 4, *Homo sapiens*) (MER029975), ADAM3A g.p. (*Homo sapiens*) (MER005200), ADAM1 g.p. (*Homo sapiens*) (MER003912), subfamily M12B non-peptidase homologues (MER188210), subfamily M12B non-peptidase homologues (MER188211), subfamily M12B non-peptidase homologues (MER188212), subfamily M12B non-peptidase homologues (MER188220), neprilysin (MER001050), endothelin-converting enzyme 1 (MER001057), endothelin-converting enzyme 2 (MER004776), DINE peptidase (MER005197), neprilysin-2 (MER013406), Kell blood-group protein (MER001054), PHEX peptidase (MER002062), i-AAA peptidase (MER001246), i-AAA peptidase (MER005755), paraplegin (MER004454), Afg3-like protein 2 (MER005496), Afg3-like protein 1A (MER014306), pappalysin-1 (MER002217), pappalysin-2 (MER014521), farnesylated-protein converting enzyme 1 (MER002646), metalloprotease-related protein-1 (MER030873), aminopeptidase AMZ2 (MER011907), aminopeptidase AMZ1 (MER058242), carboxypeptidase A1 (MER001190), carboxypeptidase A2 (MER001608), carboxypeptidase B (MER001194), carboxypeptidase N (MER001198), carboxypeptidase E (MER001199), carboxypeptidase M (MER001205), carboxypeptidase U (MER001193), carboxypeptidase A3 (MER001187), metallocarboxypeptidase D peptidase unit 1 (MER003781), metallocarboxypeptidase Z (MER003428), metallocarboxypeptidase D peptidase unit 2 (MER004963), carboxypeptidase A4 (MER013421), carboxypeptidase A6 (MER013456), carboxypeptidase A5 (MER017121), metallocarboxypeptidase O (MER016044), cytosolic carboxypeptidase-like protein 5 (MER033174), cytosolic carboxypeptidase 3 (MER033176), cytosolic carboxypeptidase 6 (MER033178), cytosolic carboxypeptidase 1 (MER033179), cytosolic carboxypeptidase 2 (MER037713), metallocarboxypeptidase D non-peptidase unit (MER004964), adipocyte-enhancer binding protein 1 (MER003889), carboxypeptidase-like protein X1 (MER013404), carboxypeptidase-like protein X2 (MER078764), cytosolic carboxypeptidase (MER026952), family M14 non-peptidase homologues (MER199530), insulysin (MER001214), mitochondrial processing peptidase beta-subunit (MER004497), nardilysin (MER003883), eupitrilysin (MER004877), mitochondrial processing peptidase non-peptidase alpha subunit (MER001413), ubiquinol-cytochrome c reductase core protein I (MER003543), ubiquinol-cytochrome c reductase core protein II (MER003544), ubiquinol-cytochrome c reductase core protein domain 2 (MER043998), insulysin unit 2 (MER046821), nardilysin unit 2 (MER046874), insulysin unit 3 (MER078753), mitochondrial processing peptidase subunit alpha unit 2 (MER124489), nardilysin unit 3 (MER142856), LOC133083 g.p. *(Homo sapiens*) (MER021876), subfamily M16B non-peptidase homologues (MER188757), leucyl aminopeptidase (animal) (MER003100.), Mername-AA040 peptidase (MER003919), leucyl aminopeptidase-1 (*Caenorhabditis-*type) (MER013416), methionyl aminopeptidase 1 (MER001342), methionyl aminopeptidase 2 (MER001728), aminopeptidase P2 (MER004498), Xaa-Pro dipeptidase (eukaryote) (MER001248), aminopeptidase P1 (MER004321), mitochondrial intermediate cleaving peptidase 55 kDa (MER013463), mitochondrial methionyl aminopeptidase (MER014055), Mername-AA020 peptidase homologue (MER010972), proliferation-association protein 1 (MER005497), chromatin-specific transcription elongation factor 140 kDa subunit (MER026495), proliferation-associated protein 1-like (*Homo sapiens* chromosome X) (MER029983), Mername-AA226 peptidase homologue (*Homo sapiens*) (MER056262), Mername-AA227 peptidase homologue (*Homo sapiens*) (MER047299), subfamily M24A non-peptidase homologues (MER179893), aspartyl aminopeptidase (MER003373), Gly-Xaa carboxypeptidase (MER033182), carnosine dipeptidase II (MER014551), carnosine dipeptidase I (MER015142), Mername-AA161 protein (MER021873), aminoacylase (MER001271), glutamate carboxypeptidase II (MER002104), NAALADASE L peptidase (MER005239), glutamate carboxypeptidase III (MER005238), plasma glutamate carboxypeptidase (MER005244), Mername-AA103 peptidase (MER015091), Fxna peptidase (MER029965), transferrin receptor protein (MER002105), transferrin receptor 2 protein (MER005152), glutaminyl cyclise (MER015095), glutamate carboxypeptidase II (*Homo sapiens*)*-type* non-peptidase homologue (MER026971), nicalin (MER044627), membrane dipeptidase (MER001260), membrane-bound dipeptidase-2 (MER013499), membrane-bound dipeptidase-3 (MER013496), dihydro-orotase (MER005767), dihydropyrimidinase (MER033266), dihydropyrimidinase related protein-1 (MER030143), dihydropyrimidinase related protein-2 (MER030155), dihydropyrimidinase related protein-3 (MER030151), dihydropyrimidinase related protein-4 (MER030149), dihydropyrimidinase related protein-5 (MER030136), hypothetical protein like 5730457F11RIK (MER033184), 1300019j08rik protein (MER033186)), guanine aminohydrolase (MER037714), Kae1 putative peptidase (MER001577), OSGEPL1-like protein (MER013498), S2P peptidase (MER004458), subfamily M23B non-peptidase homologues (MER199845), subfamily M23B non-peptidase homologues (MER199846), subfamily M23B non-peptidase homologues (MER199847), subfamily M23B non-peptidase homologues (MER137320), subfamily M23B non-peptidase homologues (MER201557), subfamily M23B non-peptidase homologues (MER199417), subfamily M23B non-peptidase homologues (MER199418), subfamily M23B non-peptidase homologues (MER199419), subfamily M23B non-peptidase homologues (MER199420), subfamily M23B non-peptidase homologues (MER175932), subfamily M23B non-peptidase homologues (MER199665), Poh1 peptidase (MER020382), Jab1/MPN domain metalloenzyme (MER022057), Mername-AA165 peptidase (MER021865), Brcc36 isopeptidase (MER021890), histone H2A deubiquitinase MYSM1 (MER021887), AMSH deubiquitinating peptidase (MER030146), putative peptidase (*Homo sapiens* chromosome 2) (MER029970), Mername-AA168 protein (MER021886), COP9 signalosome subunit 6 (MER030137), 26S proteasome non-ATPase regulatory subunit 7 (MER030134), eukaryotic translation initiation factor 3 subunit 5 (MER030133), IFP38 peptidase homologue (MER030132), subfamily M67A non-peptidase homologues (MER191181), subfamily M67A unassigned peptidases (MER191144), granzyme B (*Homo sapiens-*type) (MER000168), testisin (MER005212), tryptase beta (MER000136), kallikrein-related peptidase 5 (MER005544), corin (MER005881), kallikrein-related peptidase 12 (MER006038), DESC1 peptidase (MER006298), tryptase gamma 1 (MER011036), kallikrein-related peptidase 14 (MER011038), hyaluronan-binding peptidase (MER003612), transmembrane peptidase, serine 4 (MER011104), intestinal serine peptidase (rodent) (MER016130), adrenal secretory serine peptidase (MER003734), tryptase delta 1 (*Homo sapiens*) (MER005948), matriptase-3 (MER029902), marapsin (MER006119), tryptase-6 (MER006118), ovochymase-1 domain 1 (MER099182), transmembrane peptidase, serine 3 (MER005926), kallikrein-related peptidase 15 (MER000064), Mername-AA031 peptidase (MER014054), TMPRSS13 peptidase (MER014226), Mername-AA038 peptidase (MER062848), Mername-AA204 peptidase (MER029980), cationic trypsin (*Homo sapiens-type*) (MER000020), elastase-2 (MER000118), mannan-binding lectin-associated serine peptidase-3 (MER031968), cathepsin G (MER000082), myeloblastin (MER000170), granzyme A (MER001379), granzyme M (MER001541), chymase *(Homo sapiens*-type) (MER000123), tryptase alpha (MER000135), granzyme K (MER001936), granzyme H (MER000166), chymotrypsin B (MER000001), elastase-1 (MER003733), pancreatic endopeptidase E (MER000149), pancreatic elastase II (MER000146), enteropeptidase (MER002068), chymotrypsin C (MER000761), prostasin (MER002460), kallikrein 1 (MER000093), kallikrein-related peptidase 2 (MER000094), kallikrein-related peptidase 3 (MER000115), mesotrypsin (MER000022), complement component C1 r-like peptidase (MER016352), complement factor D (MER000130), complement component activated C1r (MER000238), complement component activated C1s (MER000239), complement component C2a (MER000231), complement factor B (MER000229), mannan-binding lectin-associated serine peptidase 1 (MER000244), complement factor I (MER000228), pancreatic endopeptidase E form B (MER000150), pancreatic elastase IIB (MER000147), coagulation factor Xlla (MER000187), plasma kallikrein (MER000203) coagulation factor Xia (MER000210), coagulation factor IXa (MER000216), coagulation factor VIIa (MER000215), coagulation factor Xa (MER000212), thrombin (MER000188), protein C (activated) (MER000222), acrosin (MER000078), hepsin (MER000156), hepatocyte growth factor activator (MER000186), mannan-binding lectin-associated serine peptidase 2 (MER002758), u-plasminogen activator (MER000195), t-plasminogen activator (MER000192), plasmin (MER000175), kallikrein-related peptidase 6 (MER002580), neurotrypsin (MER004171), kallikrein-related peptidase 8 (MER005400), kallikrein-related peptidase 10 (MER003645), epitheliasin (MER003736), kallikrein-related peptidase 4 (MER005266), prosemin (MER004214),
chymopasin (MER001503), kallikrein-related peptidase 11 (MER004861), kallikrein-related peptidase 11 (MER216142), trypsin-2 type A (MER000021), HtrA1 peptidase *(Homo sapiens-*type) (MER002577), HtrA2 peptidase (MER208413), HtrA2 peptidase (MER004093), HtrA3 peptidase (MER014795), HtrA4 peptidase (MER016351), Tysnd1 peptidase (MER050461), TMPRSS12 peptidase (MER017085), HAT-like putative peptidase 2 (MER021884), trypsin C (MER021898), kallikrein-related peptidase 7 (MER002001), matriptase (MER003735), kallikrein-related peptidase 13 (MER005269), kallikrein-related peptidase 9 (MER005270), matriptase-2 (MER005278), umbelical vein peptidase (MER005421), LCLP peptidase (MER001900), spinesin (MER014385), marapsin-2 (MER021929), complement factor D-like putative peptidase (MER056164), ovochymase-2 (MER022410), HAT-like 4 peptidase (MER044589), ovochymase 1 domain 1 (MER022412), epidermis-specific SP-like putative peptidase (MER029900), testis serine peptidase 5 (MER029901), Mername-AA258 peptidase (MER000285), polyserase-IA unit 1 (MER030879), polyserase-IA unit 2 (MER030880), testis serine peptidase 2 (human-type) (MER033187), hypothetical acrosin-like peptidase (*Homo sapiens)* (MER033253), HAT-like 5 peptidase (MER028215), polyserase-3 unit 1 (MER061763), polyserase-3 unit 2 (MER061748), peptidase similar to tryptophan/serine protease (MER056263), polyserase-2 unit 1 (MER061777), Mername-AA123 peptidase (MER021930). HAT-like 2 peptidase (MER099184), hCG2041452-like protein (MER099172), hCG22067 *(Homo sapiens)* (MER099169), brain-rescue-factor-1 *(Homo sapiens)* (MER098873), hCG2041108 *(Homo sapiens)* (MER099173), polyserase-2 unit 2 (MER061760), polyserase-2 unit 3 (MER065694), Mername-AA201 (peptidase homologue) MER099175, secreted trypsin-like serine peptidase homologue (MER030000 ), polyserase-1A unit 3 (MER029880), azurocidin (MER000119), haptoglobin-1 (MER000233), haptoglobin-related protein (MER000235), macrophage-stimulating protein (MER001546), hepatocyte growth factor (MER000185), protein Z (MER000227), TESP1 protein (MER047214), LOC136242 protein (MER016132), plasma kallikrein-like protein 4 (MER016346), PRSS35 protein (MER016350), DKFZp586H2123-like protein (MER066474), apolipoprotein (MER000183), psi-KLK1 pseudogene (*Homo sapiens*) (MER033287), tryptase pseudogene I (MER015077), tryptase pseudogene II (MER015078), tryptase pseudogene III (MER015079), subfamily S1A unassigned peptidases (MER216982), subfamily S1A unassigned peptidases (MER216148), amidophosphoribosyltransferase precursor (MER003314), glutamine-fructose-6-phosphate transaminase 1 (MER003322), glutamine:fructose-6-phosphate amidotransferase (MER012158), Mername-AA144 protein (MER021319), asparagine synthetase (MER033254), family C44 non-peptidase homologues (MER159286), family C44 unassigned peptidases (MER185625) family C44 unassigned peptidases (MER185626), secernin 1 (MER045376), secernin 2 (MER064573), secernin 3 (MER064582), acid ceramidase precursor (MER100794), N-acylethanolamine acid amidase precursor (MER141667), proteasome catalytic subunit 1 (MER000556), proteasome catalytic subunit 2 (MER002625), proteasome catalytic subunit 3 (MER002149), proteasome catalytic subunit 1i (MER000552), proteasome catalytic subunit 2i (MER001515), proteasome catalytic subunit 3i (MER000555), proteasome catalytic subunit 5t (MER026203), protein serine kinase c17 (MER026497), proteasome subunit alpha 6 (MER000557), proteasome subunit alpha 2 (MER000550), proteasome subunit alpha 4 (MER000554), proteasome subunit alpha 7 (MER033250), proteasome subunit alpha 5 (MER000558), proteasome subunit alpha 1 (MER000549), proteasome subunit alpha 3 (MER000553), proteasome subunit XAPC7 (MER004372), proteasome subunit beta 3 (MER001710), proteasome subunit beta 2 (MER002676), proteasome subunit beta 1 (MER000551), proteasome subunit beta 4 (MER001711), Mername-AA230 peptidase homologue *(Homo sapiens)* (MER047329), Mername-AA231 pseudogene *(Homo sapiens)* (MER047172), Mername-AA232 pseudogene (*Homo sapiens*) (MER047316), glycosylasparaginase precursor (MER003299), isoaspartyl dipeptidase (threonine type) (MER031622), taspase-1 (MER016969), gamma-glutamyltransferase 5 (mammalian-type) (MER001977), gamma-glutamyltransferase 1 (mammalian-type) (MER001629), gamma-glutamyltransferase 2 *(Homo sapiens*) (MER001976), gamma-glutamyltransferase-like protein 4 (MER002721), gamma-glutamyltransferase-like protein 3 (MER016970), similar to gamma-glutamyltransferase 1 precursor *(Homo sapiens)* (MER026204), similar to gamma-glutamyltransferase 1 precursor *(Homo sapiens)* (MER026205), Mername-AA211 putative peptidase (MER026207), gamma-glutamyltransferase 6 (MER159283), gamma-glutamyl transpeptidase homologue (chromosome 2, *Homo sapiens)* (MER037241), polycystin-1 (MER126824), KIAA1879 protein (MER159329), polycystic kidney disease 1-like 3 (MER172554), gamma-glutamyl hydrolase (MER002963), guanine 5"-monophosphate synthetase (MER043387), carbamoyl-phosphate synthase *(Homo sapiens*-type) (MER078640), dihydro-orotase (N-terminal unit) *(Homo sapiens-type)* (MER060647), DJ-1 putative peptidase (MER003390), Mername-AA100 putative peptidase (MER014802), Mername-AA101 non-peptidase homologue (MER014803), KIAA0361 protein (*Homo sapiens*-type) (MER042827), FLJ34283 protein *(Homo sapiens)* (MER044553), non-peptidase homologue chromosome 21 open reading frame 33 *(Homo sapiens*) (MER160094), family C56 non-peptidase homologues (MER177016), family C56 non-peptidase homologues (MER176613), family C56 non-peptidase homologues (MER176918). EGF-like module containing mucin-like hormone receptor-like 2 (MER037230), CD97 antigen (human type) (MER037286), EGF-like module containing mucin-like hormone receptor-like 3 (MER037288), EGF-like module containing mucin-like hormone receptor-like 1 (MER037278), EGF-like module containing mucin-like hormone receptor-like 4 (MER037294), cadherin EGF LAG seven-pass G-type receptor 2 precursor *(Homo sapiens)* (MER045397), Gpr64 (*Mus musculus*)-type protein (MER123205), GPR56 *(Homo sapiens)-type* protein (MER122057), latrophilin 2 (MER122199), latrophilin-1 (MER126380), latrophilin 3 (MER124612), protocadherin Flamingo 2 (MER124239), ETL protein (MER126267), G protein-coupled receptor 112 (MER126114), seven transmembrane helix receptor (MER125448), Gpr114 protein (MER159320), GPR126 vascular inducible G protein-coupled receptor (MER140015), GPR125 *(Homo sapiens)-type* protein (MER159279), GPR116 *(Homo sapiens)-type* G-protein coupled receptor (MER159280), GPR128 *(Homo sapiens)-type* G-protein coupled receptor (MER162015), GPR133 *(Homo sapiens)-type* protein (MER159334), GPR110 G-protein coupled receptor (MER159277), GPR97 protein (MER159322), KPG_006 protein (MER161773), KPG_008 protein (MER161835), KPG_009 protein (MER159335), unassigned homologue (MER166269), GPR113 protein (MER159352), brain-specific angiogenesis inhibitor 2 (MER159746), PIDD auto-processing protein unit 1 (MER020001), PIDD auto-processing protein unit 2 (MER063690), MUC1 self-cleaving mucin (MER074260), dystroglycan (MER054741), proprotein convertase 9 (MER022416), site-1 peptidase (MER001948), furin (MER000375), proprotein convertase 1 (MER000376), proprotein convertase 2 (MER000377), proprotein convertase 4 (MER028255), PACE4 proprotein convertase (MER000383), proprotein convertase 5 (MER002578), proprotein convertase 7 (MER002984), tripeptidyl-peptidase II (MER000355), subfamily S8A non-peptidase homologues (MER201339), subfamily S8A non-peptidase homologues (MER191613), subfamily S8A unassigned peptidases (MER191611), subfamily S8A unassigned peptidases (MER191612), subfamily S8A unassigned peptidases (MER191614), tripeptidyl-peptidase I (MER003575), prolyl oligopeptidase (MER000393), dipeptidyl-peptidase IV (eukaryote) (MER000401), acylaminoacyl-peptidase (MER000408), fibroblast activation protein alpha subunit (MER000399), PREPL A protein (MER004227), dipeptidyl-peptidase 8 (MER013484), dipeptidyl-peptidase 9 (MER004923), FLJ1 putative peptidase (MER017240), Mername-AA194 putative peptidase (MER017353), Mername-AA195 putative peptidase (MER017367), Mername-AA196 putative peptidase (MER017368), Mername-AA197 putative peptidase (MER017371), C14orf29 protein (MER033244), hypothetical protein (MER033245), hypothetical esterase/lipase/thioesterase (MER047309), protein bat5 (MER037840), hypothetical protein flj40219 (MER033212), hypothetical protein flj37464 (MER033240), hypothetical protein flj33678 (MER033241), dipeptidylpeptidase homologue DPP6 (MER000403), dipeptidylpeptidase homologue DPP10 (MER005988), protein similar to *Mus musculus* chromosome 20 open reading frame 135 (MER037845), kynurenine formamidase (MER046020), thyroglobulin precursor (MER011604), acetylcholinesterase (MER033188), cholinesterase (MER033198), carboxylesterase D1 (MER033213), liver carboxylesterase (MER033220), carboxylesterase 3 (MER033224), carboxylesterase 2 (MER033226), bile salt-dependent lipase (MER033227), carboxylesterase-related protein (MER033231), neuroligin 3 (MER033232), neuroligin 4, X-linked (MER033235), neuroligin 4, Y-linked (MER033236), esterase D (MER043126), arylacetamide deacetylase (MER033237), KIAA1363-like protein (MER033242), hormone-sensitive lipase (MER033274), neuroligin 1 (MER033280), neuroligin 2 (MER033283), family S9 non-peptidase homologues (MER212939), family S9 non-peptidase homologues (MER211490), subfamily S9C unassigned peptidases (MER192341), family S9 unassigned peptidases (MER209181), family S9 unassigned peptidases (MER200434), family S9 unassigned peptidases (MER209507), family S9 unassigned peptidases (MER209142), serine carboxypeptidase A (MER000430), vitellogenic carboxypeptidase-like protein (MER005492), RISC peptidase (MER010960), family S15 unassigned peptidases (MER199442), family S15 unassigned peptidases (MER200437), family S15 unassigned peptidases (MER212825), lysosomal Pro-Xaa carboxypeptidase (MER000446), dipeptidyl-peptidase II (MER004952), thymus-specific serine peptidase (MER005538), epoxide hydrolase-like putative peptidase (MER031614), Loc328574-like protein (MER033246), abhydrolase domain-containing protein 4 (MER031616), epoxide hydrolase (MER000432), mesoderm specific transcript protein (MER199890), mesoderm specific transcript protein (MER017123), cytosolic epoxide hydrolase (MER029997), cytosolic epoxide hydrolase (MER213866), similar to hypothetical protein FLJ22408 (MER031608), CGI-58 putative peptidase (MER030163), Williams-Beuren syndrome critical region protein 21 epoxide hydrolase (MER031610), epoxide hydrolase (MER031612), hypothetical protein flj22408 (epoxide hydrolase) (MER031617), monoglyceride lipase (MER033247), hypothetical protein (MER033249), valacyclovir hydrolase (MER033259), Ccg1-interacting factor b (MER210738),glycosylasparaginase precursor (MER003299), isoaspartyl dipeptidase (threonine type) (MER031622). taspase-1 (MER016969), gamma-glutamyltransferase 5 (mammalian-type) (MER001977), gamma-glutamyltransferase 1 (mammalian-type) (MER001629), gamma-glutamyltransferase 2 *(Homo sapiens)* (MER001976), gamma-glutamyltransferase-like protein 4 (MER002721). gamma-glutamyltransferase-like protein 3 (MER016970). similar to gamma-glutamyltransferase 1 precursor *(Homo sapiens*) (MER026204). similar to gamma-glutamyltransferase 1 precursor *(Homo sapiens*) (MER026205). Mername-AA211 putative peptidase (MER026207). gamma-glutamyltransferase 6 (MER159283). gamma-glutamyl transpeptidase homologue (chromosome 2, *Homo sapiens*) (MER037241). polycystin-1 (MER126824), KIAA1879 protein (MER159329). polycystic kidney disease 1-like 3 (MER172554). gamma-glutamyl hydrolase (MER002963). guanine 5"-monophosphate synthetase (MER043387). carbamoyl-phosphate synthase *(Homo sapiens*-type) (MER078640). dihydro-orotase (N-terminal unit) *(Homo sapiens*-type) (MER060647). DJ-1 putative peptidase (MER003390). Mername-AA100 putative peptidase (MER014802). Mername-AA101 non-peptidase homologue (MER014803). KIAA0361 protein *(Homo sapiens-*type) (MER042827). FLJ34283 protein *(Homo sapiens)* (MER044553). non-peptidase homologue chromosome 21 open reading frame 33 *(Homo sapiens)* (MER160094). family C56 non-peptidase homologues (MER177016), family C56 non-peptidase homologues (MER176613). family C56 non-peptidase homologues (MER176918). EGF-like module containing mucin-like hormone receptor-like 2 (MER037230). CD97 antigen (human type) (MER037286). EGF-like module containing mucin-like hormone receptor-like 3 (MER037288). EGF-like module containing mucin-like hormone receptor-like 1 (MER037278). EGF-like module containing mucin-like hormone receptor-like 4 (MER037294). cadherin EGF LAG seven-pass G-type receptor 2 precursor (*Homo sapiens*) (MER045397), Gpr64 (*Mus musculus*)-type protein (MER123205). GPR56 *(Homo sapiens)-type* protein (MER122057). latrophilin 2 (MER122199). latrophilin-1 (MER126380). latrophilin 3 (MER124612). protocadherin Flamingo 2 (MER124239). ETL protein (MER126267). G protein-coupled receptor 112 (MER126114). seven transmembrane helix receptor (MER125448). Gpr114 protein (MER159320). GPR126 vascular inducible G protein-coupled receptor (MER140015). GPR125 *(Homo sapiens*)-type protein (MER159279). GPR116 *(Homo sapiens)-type* G-protein coupled receptor (MER159280). GPR128 *(Homo sapiens*)-type G-protein coupled receptor (MER162015). GPR133 (*Homo sapiens*)-type protein (MER159334) GPR110 G-protein coupled receptor (MER159277), GPR97 protein (MER159322),KPG_006 protein (MER161773) KPG_008 protein (MER161835), KPG_009 protein (MER159335), unassigned homologue (MER166269), GPR113 protein (MER159352), brain-specific angiogenesis inhibitor 2 (MER159746), PIDD auto-processing protein unit 1 (MER020001), PIDD auto-processing protein unit 2 (MER063690), MUC1 self-cleaving mucin (MER074260), dystroglycan (MER054741), proprotein convertase 9 (MER022416), site-1 peptidase (MER001948), furin (MER000375), proprotein convertase 1 (MER000376), proprotein convertase 2 (MER000377), proprotein convertase 4 (MER028255), PACE4 proprotein convertase (MER000383), proprotein convertase 5 (MER002578), proprotein convertase 7 (MER002984), tripeptidyl-peptidase II (MER000355), subfamily S8A non-peptidase homologues (MER201339), subfamily S8A non-peptidase homologues (MER191613), subfamily S8A unassigned peptidases (MER191611), subfamily S8A unassigned peptidases (MER191612), subfamily S8A unassigned peptidases (MER191614), tripeptidyl-peptidase I (MER003575), prolyl oligopeptidase (MER000393), dipeptidyl-peptidase IV (eukaryote) (MER000401), acylaminoacyl-peptidase (MER000408), fibroblast activation protein alpha subunit (MER000399), PREPL A protein (MER004227), dipeptidyl-peptidase 8 (MER013484), dipeptidyl-peptidase 9 (MER004923), FLJ1 putative peptidase (MER017240), Mername-AA194 putative peptidase (MER017353), Mername-AA195 putative peptidase (MER017367), Mername-AA196 putative peptidase (MER017368), Mername-AA197 putative peptidase (MER017371), C14orf29 protein (MER033244), hypothetical protein (MER033245), hypothetical esterase/lipase/thioesterase (MER047309), protein bat5 (MER037840), hypothetical protein flj40219 (MER033212), hypothetical protein flj37464 (MER033240), hypothetical protein flj33678 (MER033241), dipeptidylpeptidase homologue DPP6 (MER000403), dipeptidylpeptidase homologue DPP10 (MER005988), protein similar to *Mus musculus* chromosome 20 open reading frame 135 (MER037845), kynurenine formamidase (MER046020), thyroglobulin precursor (MER011604), acetylcholinesterase (MER033188), cholinesterase (MER033198), carboxylesterase D1 (MER033213), liver carboxylesterase (MER033220), carboxylesterase 3 (MER033224), carboxylesterase 2 (MER033226), bile salt-dependent lipase (MER033227), carboxylesterase-related protein (MER033231), neuroligin 3 (MER033232), neuroligin 4, X-linked (MER033235), neuroligin 4, Y-linked (MER033236), esterase D (MER043126), arylacetamide deacetylase (MER033237), KIAA1363-like protein (MER033242), hormone-sensitive lipase (MER033274), neuroligin 1 (MER033280), neuroligin 2 (MER033283), family S9 non-peptidase homologues (MER212939), family S9 non-peptidase homologues (MER211490), subfamily S9C unassigned peptidases (MER192341), family S9 unassigned peptidases (MER209181), family S9 unassigned peptidases (MER200434), family S9 unassigned peptidases (MER209507), family S9 unassigned peptidases (MER209142), serine carboxypeptidase A (MER000430), vitellogenic carboxypeptidase-like protein (MER005492), RISC peptidase (MER010960), family S15 unassigned peptidases (MER199442), family S15 unassigned peptidases (MER200437), family S15 unassigned peptidases (MER212825), ,lysosomal Pro-Xaa carboxypeptidase (MER000446), dipeptidyl-peptidase II (MER004952), thymus-specific serine peptidase (MER005538), epoxide hydrolase-like putative peptidase (MER031614), Loc328574-like protein (MER033246), abhydrolase domain-containing protein 4 (MER031616), epoxide hydrolase (MER000432), mesoderm specific transcript protein (MER199890), mesoderm specific transcript protein (MER017123), cytosolic epoxide hydrolase (MER029997), cytosolic epoxide hydrolase (MER213866), similar to hypothetical protein FLJ22408 (MER031608),_CGI-58 putative peptidase (MER030163), Williams-Beuren syndrome critical region protein 21 epoxide hydrolase (MER031610),_epoxide hydrolase (MER031612), hypothetical protein flj22408 (epoxide hydrolase) (MER031617), monoglyceride lipase (MER033247),_hypothetical protein (MER033249),_valacyclovir hydrolase (MER033259), Ccg1-interacting factor b (MER210738).

Particular preferred protease cleavage sites include Cathepsin B cleavage sites (eg those containing the valine-citrulline bond or other similar iterations of the sequence such as V-R, F-K and V-A, for example, as described further in Choe et al (J Biol Chem 5(281): 12824-32) which is incorporated herein by reference); matrix metalloproteinase 2 cleavage sites (eg AIPVSLR; SEQ ID No: 275) and matrix metalloproteinase 14 (membrane type 1) cleavage sites (eg PRSAKELR; SEQ ID No: 276).

It will be appreciated that for a given unwanted cell type, the skilled person can readily determine an appropriate protease cleavage site to use, for example by consulting scientific literature to determine which proteases are overexpressed by that cell type. Oncomine (https://www.oncomine.org) is an online cancer gene expression database, and so when the agent of the invention is for treating cancer, the skilled person may search the Oncomine database to identify a particular protease cleavage site that will be appropriate for treating a given cancer type. Alternative databases include European Bioinformatic Institute (http://www.ebi.ac.uk) in particular (http://www.ebi.ac.uk/gxa). Protease databases include PMAP (http://www.proteolysis.org), ExPASy Peptide Cutter (http://ca.expasy.org/tools/peptide cutter) and PMAP.Cut DB (http://cutdb.burnham.org).

It is noted that it may be desirable to screen a library of peptides incorporating multiple potential cleavage sites and evaluating the optimal cleavage site for a given unwanted cell (eg tumour). Such peptides may be useful as linkers to join the T cell antigen peptide to the targeting moiety as discussed below.

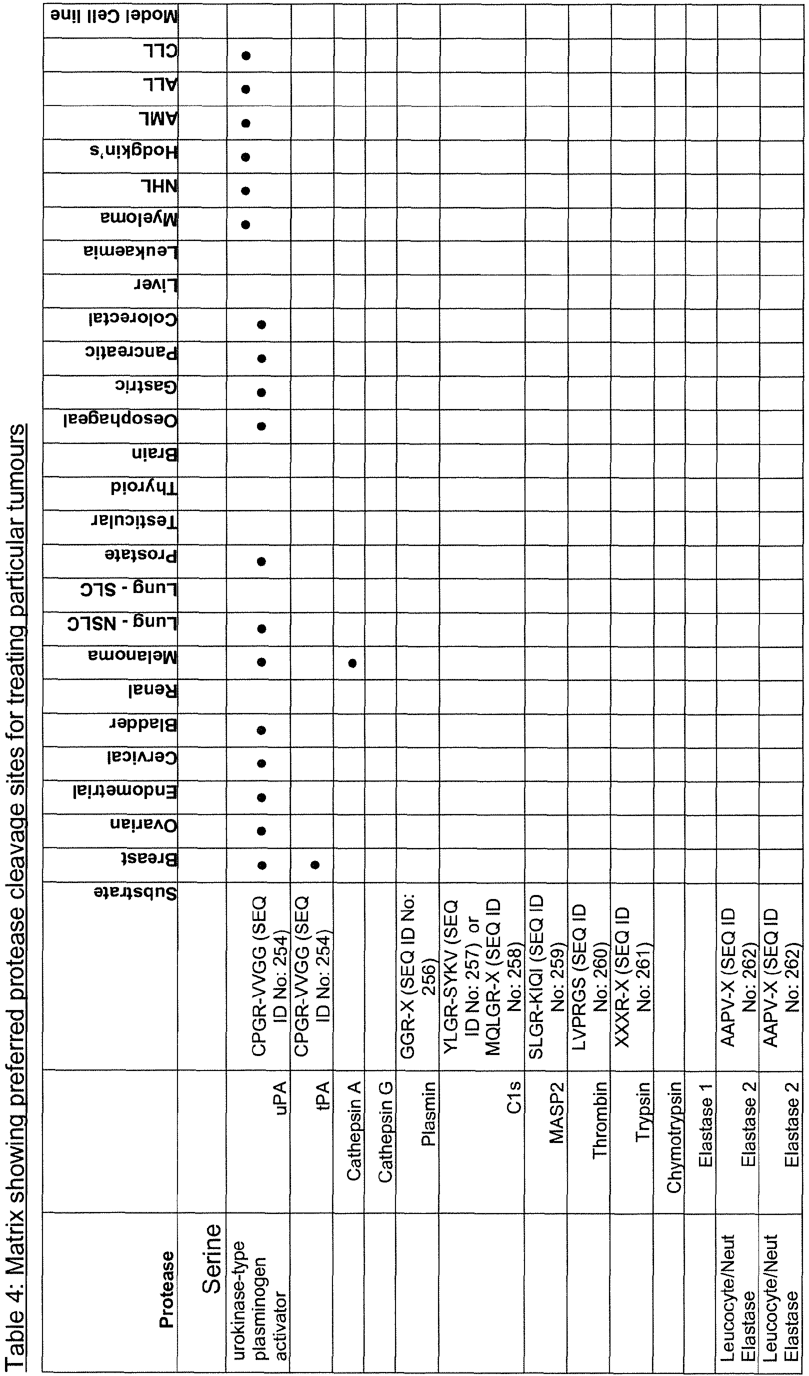

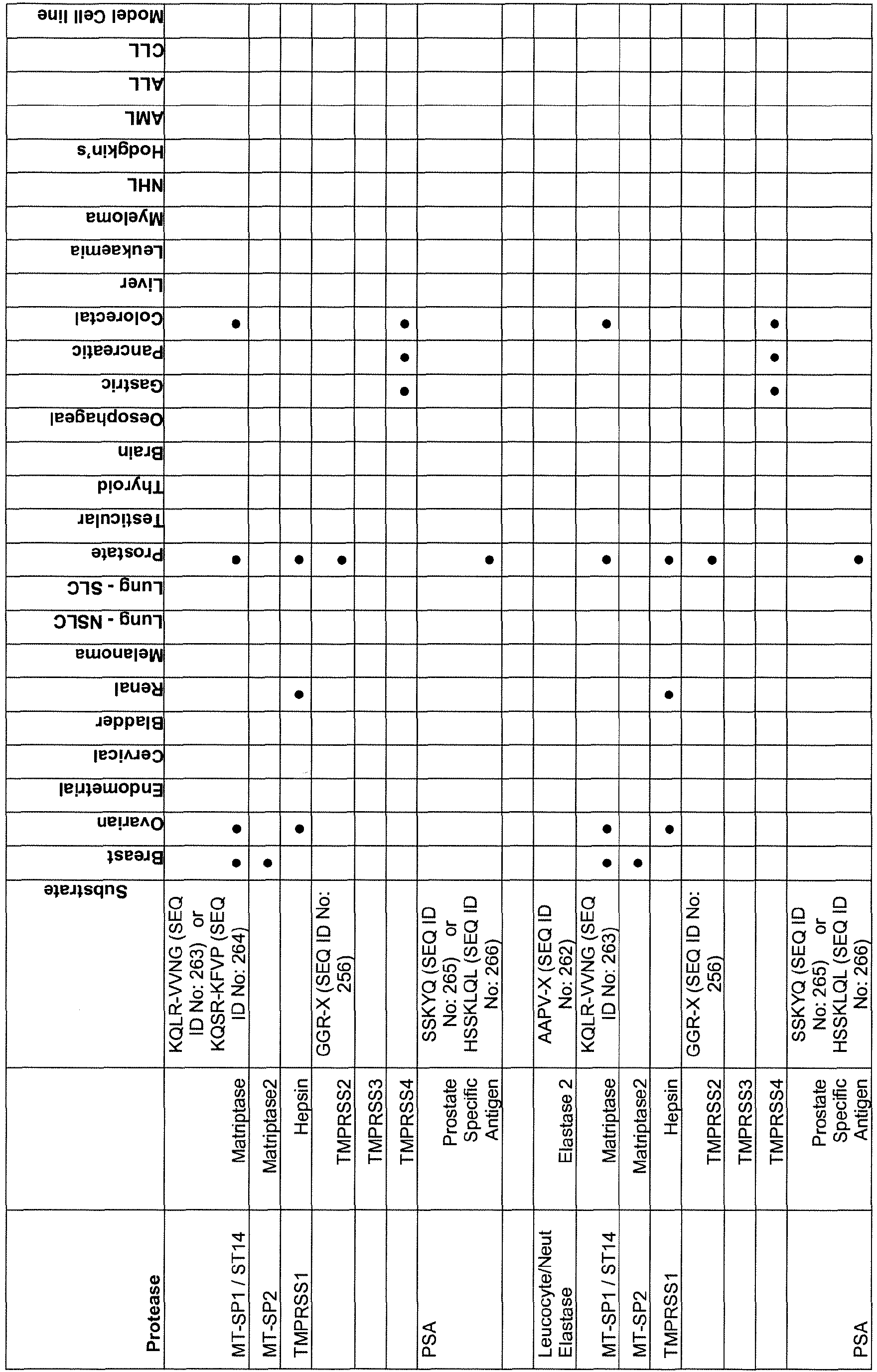

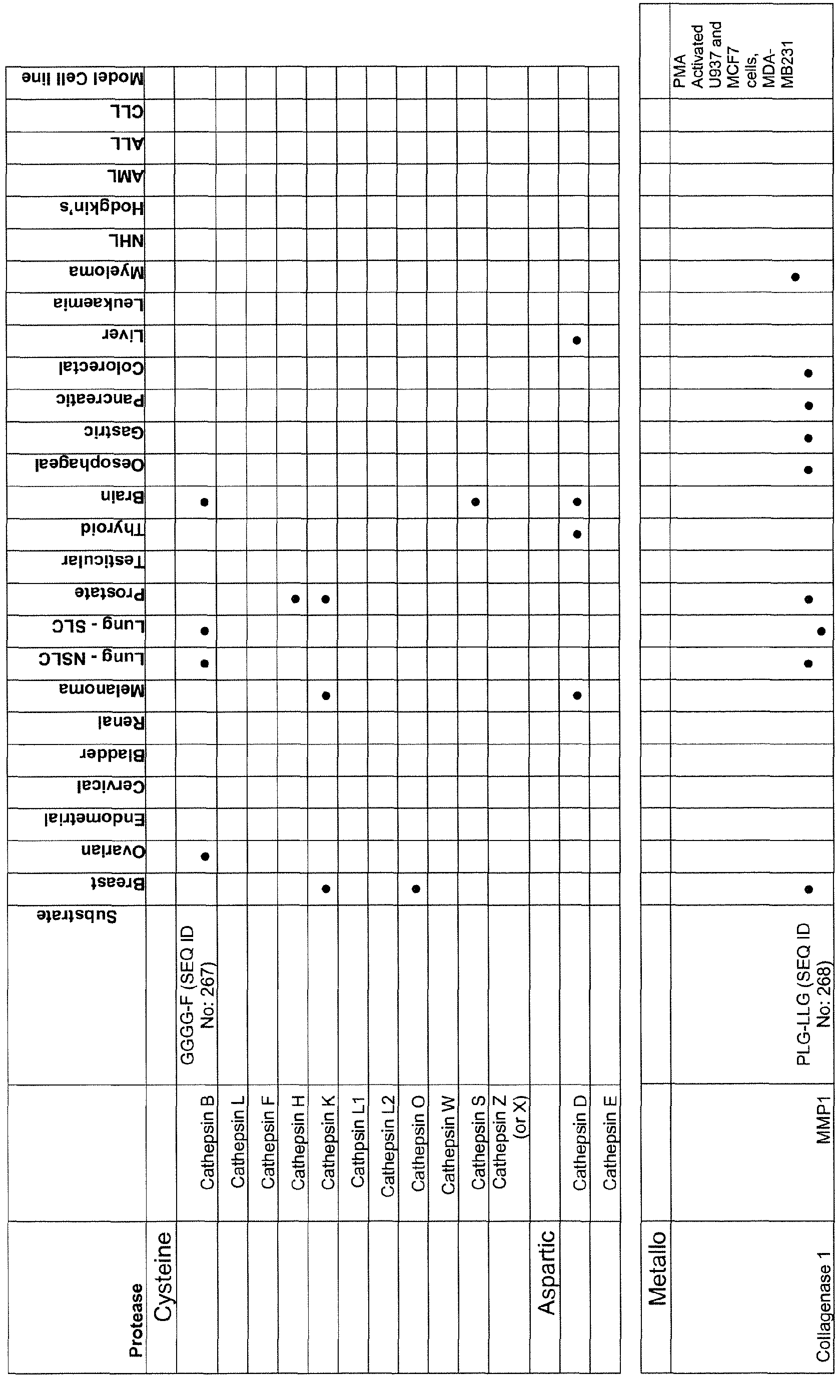

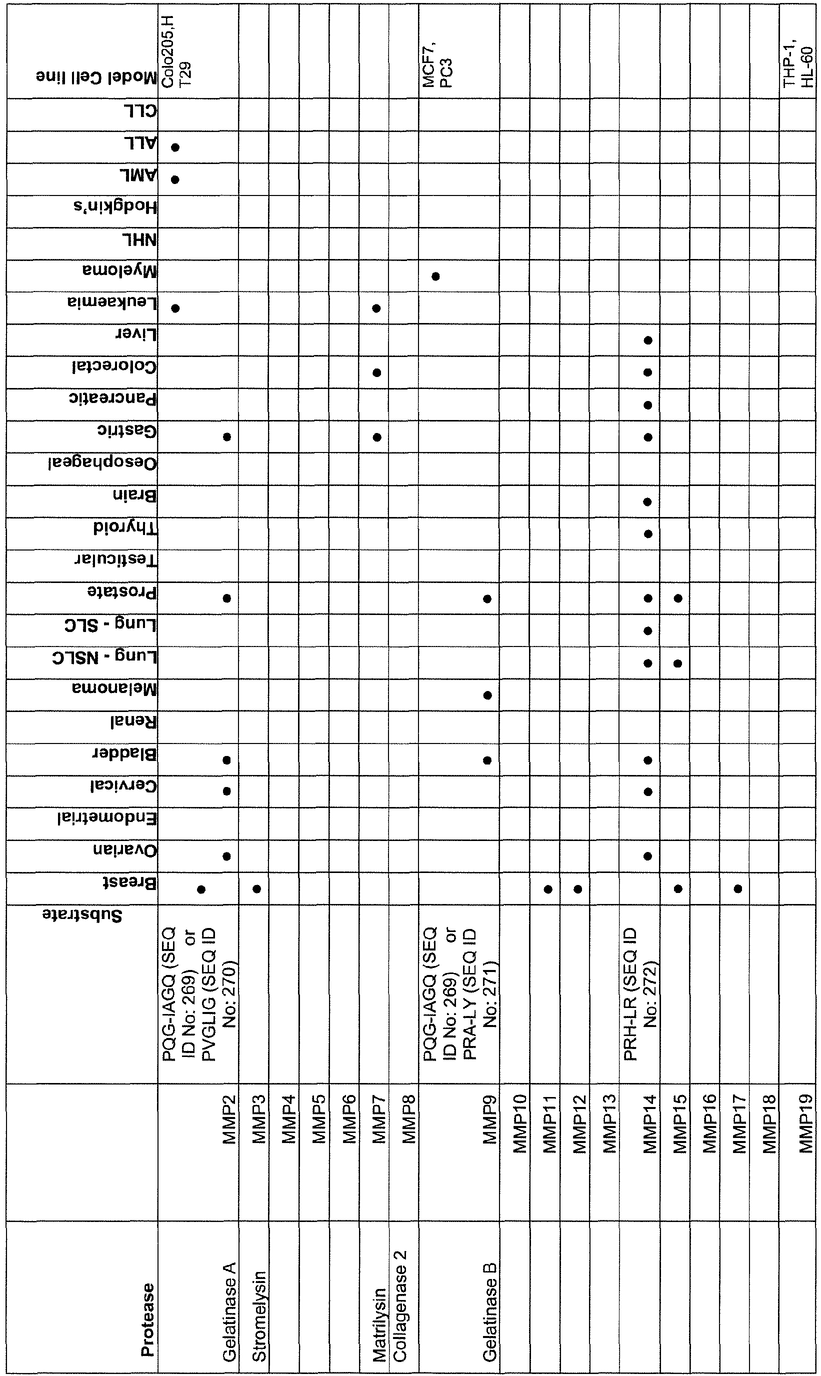

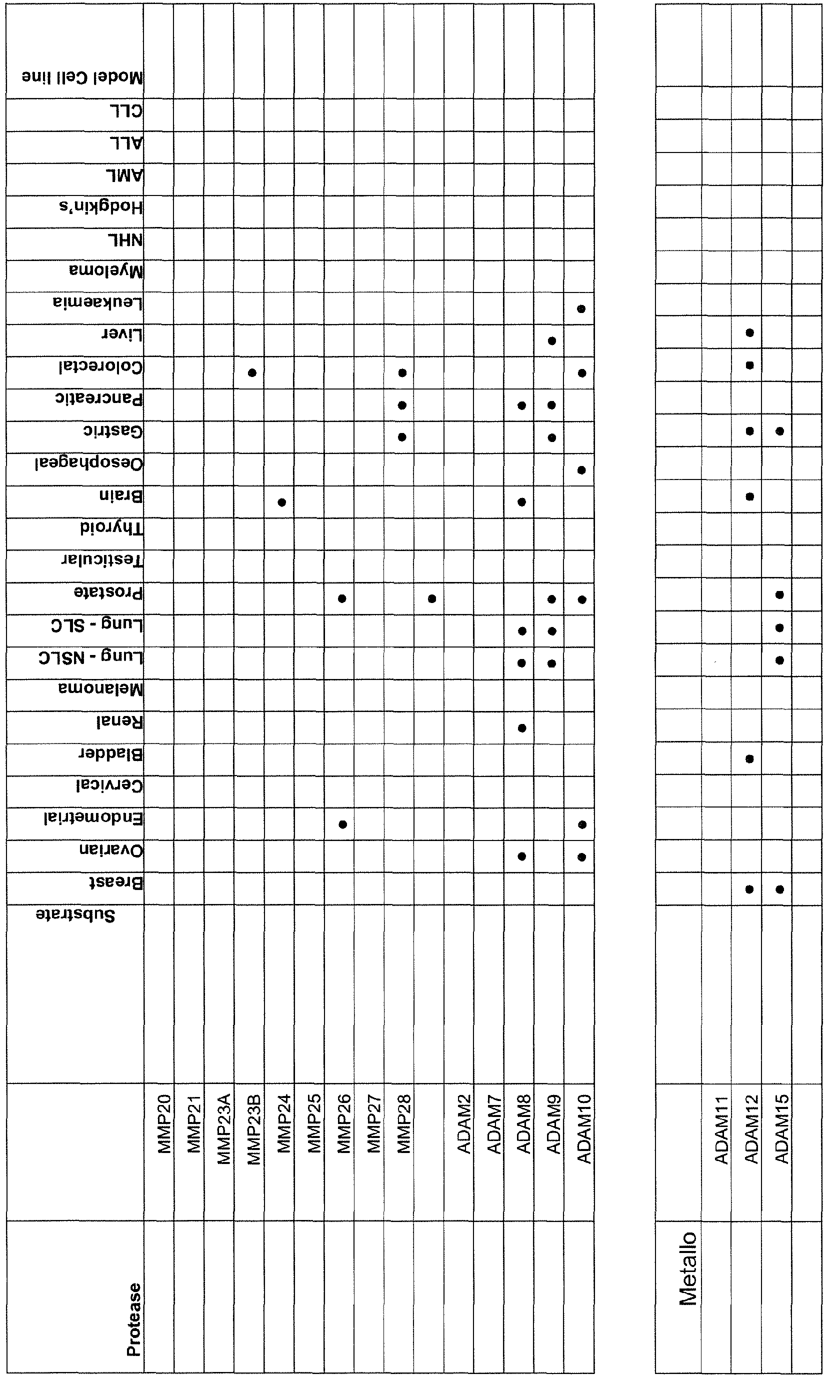

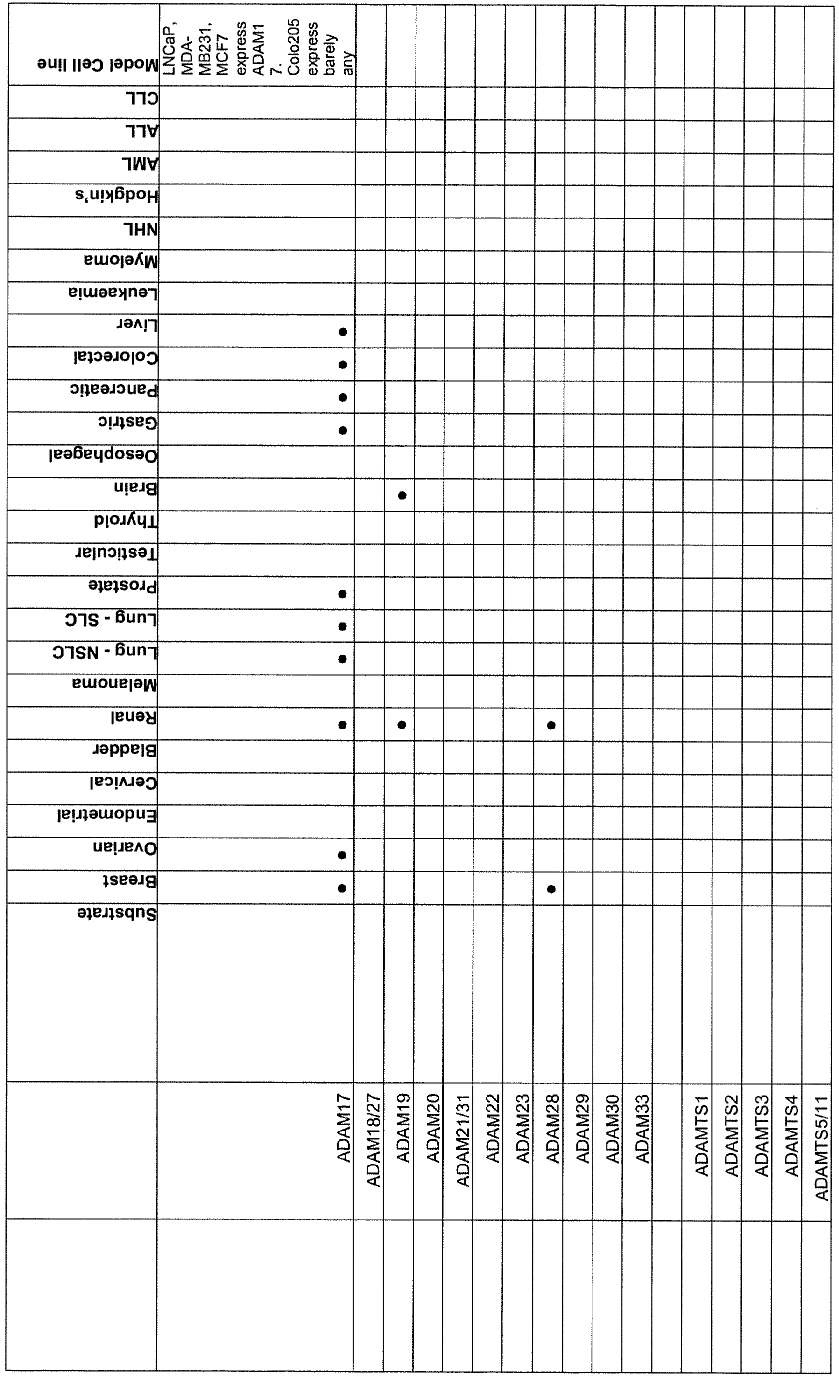

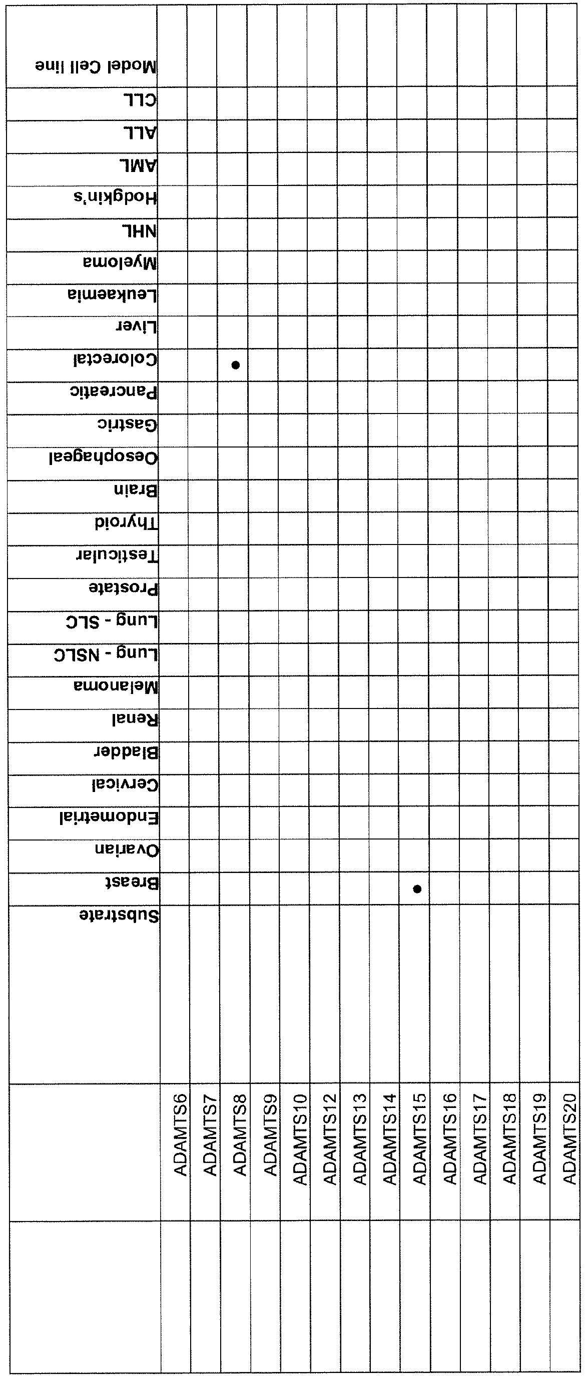

**Table 5: Tumour sites in which ADAM overexpression has been reported**

| **Protein** | **Tumour expression** |
|---|---|
| ADAM8 | Brain, kidney, lung, pancreas |
| ADAM9 | Breast, gastric, liver, lung, pancreas, prostate |
| ADAM10 | Colon, gastric, leukaemia, prostate, uterus, ovary |
| ADAM12 | Bladder, brain, breast, colon, gastric, liver |
| ADAM15 | Breast, gastric, lung, prostate |
| ADAM17 | Brain, breast, colon, gastric, kidney, liver, lung, ovary, pancreas, prostate |
| ADAM19 | Brain, kidney |
| ADAM28 | Breast, kidney, lung |

A number of the proteolytic ADAMs (a disintegrin and metalloproteinase) have been detected in cancers and mRNA or protein levels have been found to be upregulated relative to normal tissue (adapted from Nature Reviews Cancer 8, 932-941 (December 2008) | doi:10.1038/nrc2459).

In one embodiment, the cleavage site is one that is cleavable by an esterase.

Other cleavage sites include linkages which are labile under certain conditions in the vicinity of unwanted cells (eg tumour microenvironment). For example, the cleavage site may comprise disulphide bonds, which can be reduced in the hypoxic tumour microenvironment, or may comprise pH sensitive moieties that break in acidic conditions. It will be understood, however, that the cleavage site must be selectively cleavable in the vicinity of the unwanted cells and so such linkages must be more labile and preferably only labile in the vicinity of unwanted cells compared to in the vicinity of wanted cells.

Alternatively, but less preferred, the cleavage site may comprise nucleic acid (eg DNA or RNA) that is selectively cleavable in the vicinity of unwanted cells (eg by nucleases). Other less preferred cleavage sites include phosphate, lipid or disulphide containing moieties that may be cleavable by appropriate enzymes.

In an embodiment, the cyclic peptide comprises more than one selectively cleavable cleavage site (*eg* more than one enzymatic cleavage site such as a protease cleavage site). Thus, the cyclic peptide may comprise 2, 3, 4, 5, 6, 7, 8, 9 or 10 selectively cleavable cleavage sites (eg protease cleavage sites).

For example, a cyclic peptide comprising a T cell antigen peptide and one or more linking moieties may contain two cleavage sites, located within the one or more linking moieties, which cleavage sites flank the T cell antigen peptide on each side. Cleavage of the two cleavage sites will result in the detachment of the T cell antigen peptide from the one or more linking moieties, thereby rendering the T cell antigen peptide capable of eliciting a T cell response.

In another example, the cyclic peptide may comprise multiple T cell antigen peptides and one or more linking moieties, wherein multiple cleavage sites flank each side of the multiple T cell antigen peptides. Cleavage of the multiple cleavage sites will result in the detachment of each T cell antigen peptide from the other T cell antigen peptides and/or the one or more linking moieties, thereby rendering each T cell antigen peptide capable of eliciting a T cell response.

Where the cyclic peptide contains multiple cleavage sites, it will be appreciated that the multiple cleavage sites (eg enzymatic cleavage sites such as protease cleavage sites) may be the same or different. The cyclic peptide may contain two or more different cleavage sites such as two or more different protease cleavage sites, or it may contain two or more of the same cleavage site such as two or more of the same protease cleavage site. Having different selectively cleavable cleavage sites within the cyclic peptide may be useful to impart additionally specificity into the agent of the invention. For example, if the agent was to be used for treating a particular tumour, the agent may be designed to contain two different cleavage sites that are known to be selectively cleavable in the vicinity of that particular tumour (*eg* two different protease cleavage sites that are selectively cleaved by proteases known to be highly expressed in that tumour type).

In an embodiment, the cyclic peptide comprises the amino acid sequence FR∼GGANLVPMVATVAA (SEQ ID No: 278), where the underlined residues represent the T cell antigen peptide, the remaining residues correspond to peptide linkers comprising a cleavage site, and '∼' represents the scissile bond.

Although, the inventors have found that, by presenting the T cell antigen peptide within a cyclic peptide, a targeting moiety is not necessary to direct the agent of the invention to unwanted cells, it may still nevertheless be desirable to incorporate such a moiety, for example to further improve specificity of action. Hence, in an embodiment, the agent of the invention further comprises a targeting moiety. It will be appreciated that this may be within the cyclic structure of the cyclic peptide itself (*eg* a targeting peptide within the cyclised peptide chain), or otherwise attached to the cyclic peptide.

### Targeting moiety

By 'targeting moiety', we include the meaning of any moiety that is capable of targeting to the unwanted cells. Preferably, the targeting moiety is capable of targeting selectively to the unwanted cells. For example, it is preferred if the targeting moiety targets unwanted cells to a greater extent than it does normal cells, and most preferably targets only unwanted cells.

It will be appreciated that binding of the targeting moiety to normal cells may be tolerated if they can be functionally replaced by other therapeutic means or if they are not essential to life. Thus, a targeting moiety that targets to a cancer cell as well as, for example, an endocrine tissue or organ is not precluded. In this case, the targeting moiety acts to redirect an immune response to both unwanted cells and to other cells that can be functionally replaced by therapeutic means, as previously mentioned above.

It is also appreciated that since the one or more cleavage sites in the agent confers specificity on where the T cell antigen peptide is rendered capable of eliciting a T cell response, binding of the targeting moiety to normal cells, in the vicinity of which the one or more cleavage sites are not cleaved, may also be tolerated.

Most preferably, however, the targeting moiety targets selectively to unwanted cells as opposed to any other cells.

In one embodiment, the targeting moiety is a specific binding partner of an entity expressed by or associated with the unwanted cell. Typically, the expressed entity is expressed selectively on the unwanted cell. For example, the abundance of the expressed entity is typically 10 or 100 or 500 or 1000 or 5000 or 10000 higher on the unwanted cell than on other cells within the body to be treated. However, as mentioned above, the cleavage site provides additional specificity on where the T cell antigen peptide is rendered capable of eliciting a T cell response and so the binding partner may bind an entity that is similarly or even underexpressed on unwanted cells relative to other cells within the body.

By "binding partner" we include the meaning of a molecule that binds to an entity expressed by a particular cell. Preferably, the binding partner binds selectively to that entity. For example, it is preferred if the binding partner has a K_{d} value (dissociation constant) which is at least five or ten times lower (i.e. higher affinity) than for at least one other entity expressed by another cell (e.g. a normal cell type), and preferably more than 100 or 500 times lower. More preferably, the binding partner of that entity has a K_{d} value more than 1000 or 5000 times lower than for at least one other entity expressed by another cell (e.g. normal cell type). K_{d} values can be determined readily using methods well known in the art. However, as discussed above, it is appreciated that the binding partner may bind selectively to an entity expressed by an unwanted cell and by a normal cell provided that the normal cell may be functionally replaced or else is not essential to life. For example, in lymphoma, anti-CD20 (which targets all B cells) is very effective and kills all B cells, healthy and malignant. However, this can be tolerated as B cells are not critical for health. Further, in the case of melanoma, lymphoma, prostate cancer, thyroid, testicular or ovarian cancer, targeting healthy counterpart tissue would also be tolerated.

Typically, the binding partner is one that binds to an entity that is present or accessible to the binding partner in significantly greater concentrations in or on unwanted cells than in any normal cells of the host. Thus, the binding partner may bind to a surface molecule or antigen on the unwanted cell that is expressed in considerably higher amounts than on normal cells. Similarly, the binding partner may bind to an entity that has been secreted into the extracellular fluid by the unwanted cells to a greater extent than by normal cells. For example, the binding partner may bind to a tumour associated antigen which is expressed on the cell membrane or which has been secreted into tumour extracellular fluid.

The targeting moiety may be any of a polypeptide, a peptide, a small molecule or a peptidomimetic.

In a preferred embodiment, the targeting moiety is a peptide that can be incorporated into the peptide chain making up the cyclic peptide of the invention. The peptide may be one that binds to an entity that is present or accessible to the peptide in significantly greater concentrations in or on unwanted cells than in any normal cells of the host. For example, the peptide may be a receptor binding peptide (eg a EGFR binding peptide or a Her2 binding peptide), or a peptide with folate containing residues.

In a less preferred embodiment, the targeting moiety is an antibody that binds to an antigen expressed by the unwanted cell. Suitable antibody targets (with examples of unwanted cell types in parentheses) include: Her2/Neu (Epithelial malignancies); CD22 (B cells, autoimmune or malignant); EpCAM (CD326) (Epithelial malignancies); EGFR (epithelial malignancies); PMSA (Prostate Carcinoma); CD30 (B cell malignancies); CD20 (B cells, autoimmune, allergic or malignant); CD33 (Myeloid malignancies); membrane IgE (Allergic B cells); IgE Receptor (CD23) (Mast cells or B cells in allergic disease), CD80 (B cells, autoimmune, allergic or malignant); CD86 (B cells, autoimmune, allergic or malignant); CD2 (T cell or NK cell lymphomas); CA125 (multiple cancers including Ovarian carcinoma); Carbonic Anhydrase IX (multiple cancers including Renal Cell Carcinoma); CD70 (B cells, autoimmune, allergic or malignant); CD74 (B cells, autoimmune, allergic or malignant); CD56 (T cell or NK cell lymphomas); CD40 (B cells, autoimmune, allergic or malignant); CD19 (B cells, autoimmune, allergic or malignant); c-met/HGFR (Gastrointestinal tract and hepatic malignancies; TRAIL-R1 (multiple malignancies including ovarian and colorectal carcinoma); DR5 (multiple malignancies including ovarian and colorectal carcinoma); PD-1 (B cells, autoimmune, allergic or malignant); PD1 L (Multiple malignancies including epithelial adenocarcinoma); IGF-1R (Most malignancies including epithelial adenocarcinoma); VEGF-R2 (The vasculature associated with the majority of malignancies including epithelial adenocarcinomas; Prostate stem cell antigen (PSCA) (Prostate Adenocarcinoma); MUC1 (Epithelial malignancies); CanAg (tumors such as carcinomas of the colon and pancreas); Mesothelin (many tumours including mesothelioma and ovarian and pancreatic adenocarcinoma); P-cadherin (Epithelial malignancies, including breast adenocarcinoma); Myostatin (GDF8) (many tumours including sarcoma and ovarian and pancreatic adenocarcinoma); Cripto (TDGF1) (Epithelial malignancies including colon, breast, lung, ovarian, and pancreatic cancers); ACVRL1/ALK1 (multiple malignancies including leukaemias and lymphomas); MUC5AC (Epithelial malignancies, including breast adenocarcinoma); CEACAM (Epithelial malignancies, including breast adenocarcinoma); CD137 (B cells or T cells, autoimmune, allergic or malignant); CXCR4 (B cells or T cells, autoimmune, allergic or malignant); Neuropilin 1 (Epithelial malignancies, including lung cancer); Glypicans (multiple cancers including liver, brain and breast cancers); HER3/EGFR (Epithelial malignancies); PDGFRa (Epithelial malignancies); EphA2 (multiple cancers including neuroblastoma, melanoma, breast cancer, and small cell lung carcinoma); and CD138 (Myeloma).

Examples of antibodies include an anti-epidermal growth factor receptor antibody such as Cetuximab, an anti-Her2 antibody, an anti-CD20 antibody such as Rituximab, an anti-CD22 antibody such as Inotuzumab, an anti-CD70 antibody, an anti-CD33 antibody such as hp67.6 or Gemtuzumab, an anti-MUC1 antibody such as GP1.4 and SM3, an anti-CD40 antibody, an anti-CD74 antibody, an anti-P-cadherin antibody, an anti-EpCAM antibody, an anti-CD138 antibody, an anti-E-cadherin antibody, an anti-CEA antibody, and an anti-FGFR3 antibody.

Antibodies may be attached to the cyclic peptide by standard cross-linking procedures known in the art and described herein.

Examples of tumour-associated, immune cell-associated and infection reagent-related antigens which may be targeted by the targeting moiety are given in Table 1.

### Table 1: Cell surface antigens for targeting

**a) Tumour Associated Antigens**

| **Antigen** | **Antibody** | **Existing uses** |
|---|---|---|
| Carcino-embryonic Antigen | C46 (Amersham) | Imaging and therapy of colon/rectum tumours. |
| | 85A12 (Unipath) | |
| Placental Alkaline Phosphatase | H17E2 (ICRF, Travers & Bodmer) | Imaging and therapy of testicular and ovarian cancers. |
| Pan Carcinoma | NR-LU-10 (NeoRx Corporation) | Imaging and therapy of various carcinomas including small cell lung cancer. |
| Polymorphic Epithelial Mucin (Human milk fat globule) | HMFG1 (Taylor-Papadimitriou, ICRF) | Imaging and therapy of ovarian cancer and pleural effusions. |
| β-human Chorionic Gonadotropin | W14 | Targeting of carboxypeptidase to human xenograft choriocarcinoma in nude mice (Searle et al (1981) Br. J. Cancer 44, 137-144). |
| A carbohydrate on Human Carcinomas | L6 (IgG2a)¹ | Targeting of alkaline phosphatase (Senter et al (1988) PNAS USA 85, 4842-4846. |
| CD20 Antigen on B Lymphoma (normal and neoplastic) | 1F5 (IgG2a)² | Targeting of alkaline phosphatase (Senter et al (1988) PNAS USA 85, 4842-4846. |

| | | |
|---|---|---|
| ¹Hellström et al (1986) Cancer Res. 46, 3917-3923 ²Clarke et al (1985) Proc. Natl. Acad. Sci. USA 82, 1766-1770 | | |

Other antigens include alphafoetoprotein, Ca-125 and prostate specific antigen.

**b) Immune Cell Antigens**

| **Antigen** | **Antibody** | **Existing uses** |
|---|---|---|
| Pan T Lymphocyte Surface Antigen (CD3) | OKT-3 (Ortho) | As anti-rejection therapy for kidney transplants. |
| B-lymphocyte Surface Antigen (CD22) | RFB4 (Janossy, Royal Free Hospital) | Immunotoxin therapy of B cell lymphoma. |
| Pan T lymphocyte Surface Antigen (CD5) | H65 (Bodmer and Knowles, ICRF; licensed to Xoma Corp., USA) | Immunotoxin treatment of acute graft versus host disease, rheumatoid arthritis. |

**c) Infectious Agent-Related Antigens**

| **Antigen** | **Antibody** | **Existing uses** |
|---|---|---|
| Mumps virus-related | Anti-mumps polyclonal antibody | Antibody conjugated to diphtheria toxin for treatment of mumps. |
| Hepatitis B Surface Antigen | Anti HBs Ag | Immunotoxin against hepatoma. |

Alternatively, the targeting moiety may be any compound or part thereof that specifically binds, in a non-immune sense, to an entity expressed by unwanted cells or otherwise becomes associated with the unwanted cells. Thus, the specific binding partner may be any of a hormone, a growth factor, a cytokine, or a receptor ligand (e.g. agonist or antagonist).

For example, cytokines have previously been used to target toxins to invading bacterial. Using genetic engineering, recombinant proteins have been produced which contain for example IL-2 and a binding domain-deleted Pseudomonas exotoxin protein (Lorderboum-Galski *et al,* 1988 (62)). This immunotoxin was effective in experimental animal models (Kozak *et al,* 1990 (63)). Fusion proteins have also been produced with IL-4, IL-6, alpha -MSH, EGF and TNF- alpha (reviewed in Waldmann 1992 (35)), all of which are appropriate for use as targeting moieties in the present invention.

Particularly useful targeting moieties include cytokines such as IL-2, EGF, VEGF, Flt3L, HGF, IGF, IL-6, or IL-4. IL-2 and IL-4 can target to adult T cell leukaemia/lymphoma cells which express the high affinity IL-2 receptor whereas normal resting T-cells do not, or to T-cells expressing the IL-4 receptor. It has previously been shown that the monoclonal antibody MR6, which binds to the human IL-4 receptor, can inhibit the IL-4 induced proliferation of cloned helper T cells and the production of IgE by polyclonal B cells (Larche *et al,* 1988 (36)). Such targeting moieties may be used to eliminate a lymphoid cell subpopulation in autoimmune disease or allergy.

Insulin like growth factors (IGF-1 and IGF-11) are preferentially taken up by malignant cells and so may be used to target tumour cells. Similarly EGF can be used to target malignant cells which upregulate the EGF receptor. Also, tumour associated blood vessels overexpress VEGF receptor and so can be targeted by the family of VEGF growth factors.

Flt3 receptor is overexpressed in leukaemias and may be a therapeutic target for acute and chronic leukaemias and myeloproliferative disorders.

Myeloma cells express IL-6 receptor and also secrete IL-6 which acts in an autocrine fashion to stimulate cell proliferation. Thus IL-6 may be used as a targeting moiety for myeloma.

In another example, the targeting moiety is melanoma stimulating hormone (MSH) which binds to the MSH receptor which is expressed in high numbers in melanoma cells.

It is appreciated that a person skilled in the art can readily select suitable binding partners for any given unwanted cell, for example by identifying surface antigens or molecules specific for that unwanted cell and finding a binding partner for that antigen or molecule. Considerable research has already been carried out on antibodies and fragments thereof to tumour-associated antigens, immune cell antigens and infectious agents, as described above. Thus, conveniently, selecting an appropriate targeting moiety for a given cell type typically involves searching the literature. Alternatively, an unwanted cell is taken from a patient (e.g. by biopsy), and antibodies directed against the cell prepared. Such 'tailor-made' antibodies are already known. It has been demonstrated that antibodies confer binding to tumour cells not only from the patient they have been obtained from but also for a large number of other patients. Thus, a plurality of such antibodies has become commercially available. Other methods of identifying suitable binding partners for a given unwanted cell include genetic approaches (eg microarray), proteomic approaches (eg differential Mass spectrometry), immunological approaches (eg immunising animals with tumour cells and identifying antibody-secreting clones which specifically target malignant cells) and *in silico* approaches wherein targets are identified using a systems biology approach.

Further selective targets and suitable binding partners are shown in Table 2.

**Table 2: Binding partners for tumour-selective targets and tumour-associated antigens**

| **Target** | **Binding Partner** | **Disease** |
|---|---|---|
| Truncated EGFR | anti-EGFR mAb | Gliomas |
| Idiotypes | anti-id mAbs | B-cell lymphomas |
| EGFR (*c-erb*B1) | EGF, TGFα anti-EGFR mAb | Breast cancer |
| *c-erbB2* | mAbs | Breast cancer |
| IL-2 receptor | IL-2 anti-Tac mAb | Lymphomas and leukaemias |
| IL-4 receptor | IL-4 | Lymphomas and leukaemias |
| IL-6 receptor | IL-6 | Lymphomas and leukaemias |
| MSH (melanocyte-stimulating hormone) receptor | α-MSH | Melanomas |
| Transferrin receptor (TR) | Transferrin anti-TR mAb | Gliomas |
| gp95/gp97 | mAbs | Melanomas drug-resistant |
| p-glycoprotein cells | mAbs | |
| cluster-1 antigen (N-CAM) | mAbs | Small cell lung carcinomas |
| cluster-w4 | mAbs | Small cell lung carcinomas |
| cluster-5A | mAbs | Small cell lung carcinomas |
| cluster-6 (LeY) | mAbs | Small cell lung carcinomas |
| PLAP (placental alkaline phosphatase) | mAbs | Some seminomas Some ovarian; some non small cell lung cancer |
| CA-125 | mAbs | Lung, ovarian carcinoma |
| ESA (epithelial specific antigen) | mAbs | |
| CD 19, 22, 37 | mAbs | B-cell lymphomas |
| 250 kDa | mAbs | Melanoma |
| proteoglycan p55 | mAbs | Breast cancer |
| TCR-IgH fusion | mAbs | Childhood T-cell leukaemia |
| Blood gp A antigen (in B or O individuals) | mAbs | Gastric and colon tumours |
| Mucin protein core | mAbs | Breast cancer |

Further targets useful in preventing or treating various cancers are provided below.

| **Target** | **Cancer** |
|---|---|
| EpCam | Bladder |
| PMSA | Prostate |
| EGFR | Breast |
| | Lung |
| | Glioblastoma |
| | Colon |
| CD20 | Lymphoma |
| CD22 | Lymphoma |
| CD52 | Lymphoma |
| | Leukaemia |

Yet further selective targets useful for preventing or treating various conditions characterised by the presence of unwanted cells are provided below. For all of the examples below, therapeutic antibodies are already available or can be readily prepared by the skilled person.

| **Target** | **Unwanted cell** |
|---|---|
| Activin A | Many types of carcinoma, lymphoma, sarcoma and leukaemia |
| activin A, activin B and inhibin B | Many types of carcinoma, lymphoma, sarcoma and leukaemia |
| Adenocarcinoma antigen | Many types of carcinoma, |
| AFP (alpha-fetoprotein) | Many types of carcinoma, |
| amyloid beta (Abeta) | Alzheimer's Disease |
| amyloid beta (Abeta) peptide Aβ40 | Alzheimer's Disease |
| amyloid beta (Abeta) peptide soluble monomer | Alzheimer's Disease |
| amyloid beta (Abeta) peptides Aβ42 and Aβ40 | Alzheimer's Disease |
| ANGPT2 (Ang2, angiopoietin 2) | Multiple carcinomas |
| N-glycolyl GM3 ganglioside (N-glycolylneuraminic acid (NeuGc, NGNA) GM3 gangliosides, NeuGcGM3) Mus musculus IgM-kappa P3 | Brain tumours |
| BSG (basigin, Ok blood group, CD147) | Many types of carcinoma, lymphoma, sarcoma and leukaemia |
| CA 72-4 (tumour associated glycoprotein 72, TAG-72, TAG, HMW mucin-like glycoprotein) | Many types of carcinoma, |
| CA9 (carbonic anhydrase IX, CAIX, MN, G250) | Many types of carcinoma, |
| carcinoma associated antigen CTAA16.88 (complex of cytokeratin polypeptides (35-40kDa)) | Many types of carcinoma, |
| CCL11 (chemokine (C-C motif) ligand 11, chemokine CC 11, eotaxin1) | Many types of carcinoma and lymphoma/leukaemia |
| CCL2 (chemokine (C-C motif) 2, chemokine CC 2, monocyte chemoattractant protein-1, MCP-1, monocyte chemotactic and activating factor, MCAF, small inducible cytokine A2, SCYA2, HC11) | Many types of carcinoma and lymphoma/leukaemia |
| CCR4 (chemokine (C-C motif) receptor 4, chemokine CC receptor 4, CCR-4, CKR4, k5-5, CD194) | Many types of carcinoma and lymphoma/leukaemia |
| CD14 | Many types of carcinoma and lymphoma/leukaemia |
| CD15 (3-fucosyl-N-acetyl-lactosamine, Lewis x, stage-specific embryonic antigen 1, SSEA-1) | Many types of carcinoma and lymphoma/leukaemia |
| CD19 (B lymphocyte surface antigen B4, Leu-12) | Lymphoma and Acute lymphoblastic leukaemia |
| CD2 (lymphocyte function-antigen 2, LFA-2) | T-cell and NK-cell lymphoma |
| CD200 (OX-2) | T-cell and NK-cell lymphoma |
| CD22 (sialic acid binding Ig-like lectin 2, SIGLEC2, SIGLEC-2, B-lymphocyte cell adhesion molecule, BL-CAM, Leu-14 | Lymphoma and Acute lymphoblastic leukaemia |
| CD33 (sialic acid binding Ig-like lectin 3, SIGLEC3, SIGLEC-3, gpG7, p67) | Myeloid leukaemia and Stem cells |
| CD38 (ADP-ribosyl cyclase 1, cyclic ADP-ribose hydrolase 1, cADPr hydrolase 1, T10) | Myeloid leukaemia and many types of carcinoma |
| CD40 (tumor necrosis factor receptor superfamily member 5, TNFRSF5, p50) | Lymphoma and many types of carcinoma |
| CD40LG (CD40 ligand, CD40L, tumor necrosis factor ligand superfamily member 5, TNFSF5, tumor necrosis factor related activation protein, TRAP, CD154) | Lymphoma and many types of carcinoma |
| CD44 (homing function and Indian blood group system, chondroitin sulfate proteoglycan 8, CSPG8) | Myeloid leukaemia and many types of carcinoma including cancer stem cells |
| CD5 (T1, LEU-1) | T-cell lymphoma, T-cells and B-cell lymphomas such as chronic lymphocytic leukaemia. |
| CD52 | T-cell lymphoma, T-cells and B-cell lymphomas. Autoimmune induced immune cells may also be targeted. |
| CD6 (Tp120) | T-cell lymphoma, T-cells and B-cell lymphomas such as chronic lymphocytic leukaemia. |
| CD70 (tumor necrosis factor superfamily member 7, TNFSF7, CD27LG, CD27L) | Lymphoma and many types of carcinoma |
| CD74 (major histocompatibility class II invariant chain, MH2) | Lymphoma and many types of carcinoma |
| CD80 (B7-1, CD28LG1) | Lymphoma and many types of carcinoma |
| CD86 (B7-2, CD28LG2) | Lymphoma and many types of carcinoma |
| CEA (anticarcinoembryonic antigen) | Many types of carcinoma, |
| CEACAM3 (carcinoembryonic antigen-related cell adhesion molecule 3, CGM1, CD66d) | Many types of carcinoma, |
| CEACAM5 (carcinoembryonic antigen-related cell adhesion molecule 5, CEA, CD66e) | Many types of carcinoma, |
| CEACAM8 (carcinoembryonic antigen-related cell adhesion molecule 8, NCA-95, nonspecific cross- reacting antigen 95 kDa, granulocyte cell antigen, CGM6, CD66b) | Many types of carcinoma, |
| CIfA (Clumping factor A) | Many types of carcinoma, |
| complement C3b, C4b | Many types of unwanted cells. |
| CSF2 (colony stimulating factor 2 (granulocyte-macrophage), granulocyte-macrophage colony stimulating factor, GM-CSF) | Myeloid diseases |
| CSF2RA (colony-stimulating factor 2(granulocyte-macrophage) receptor alpha subunit, GM-CSF-R-alpha, CD116) | Myeloid diseases |
| CSPG4 (chondroitin sulfate proteoglycan 4, high molecular weight-melanoma-associated antigen, HMW-MAA) | Many types of carcinoma, lymphoma, sarcoma and leukaemia |
| CTLA4 (cytotoxic T lymphocyte-associated antigen 4, CD152) | Regulatory T-cells and unwanted immune cells. |
| ED-B (fibronectin extra domain B) | Many types of carcinoma, |
| EGFR (epidermal growth factor receptor, | Many types of carcinoma, lymphoma, |
| receptor tyrosine-protein kinase erbB-1, ERBB1, HER1, HER-1, ERBB) | sarcoma and leukaemia |
| EPCAM (epithelial cell adhesion molecule, tumor-associated calcium signal transducer 1, TACSTD1, gastrointestinal tumor-associated protein 2, GA733-2, epithelial glycoprotein 2, EGP-2, epithelial cell adhesion molecule, Ep-CAM, KSA, KS1/4 antigen, M4S, tumor antigen 17-1A, EpCAM, CD326) | Many types of carcinoma, lymphoma, sarcoma and leukaemia |
| ERBB2 (epidermal growth factor receptor 2, receptor tyrosine-protein kinase erbB-2, EGFR2, HER2, HER-2, p185c-erbB2, NEU, CD340 | Many types of carcinoma, lymphoma, sarcoma and leukaemia |
| ERBB3 (receptor tyrosine-protein kinase erbB-3, HER3) | Many types of carcinoma, lymphoma, sarcoma and leukaemia |
| FAP (fibroblast activation protein, alpha) | Many types of carcinoma, lymphoma, sarcoma and leukaemia. |
| FCER2 (immunoglobulin E Fc receptor low affinity II, Fc epsilon RII, CD23) | Many types of carcinoma, lymphoma, sarcoma and leukaemia in B-cells. |
| FCGR1 (immunoglobulin G Fc receptor high affinity I, Fc gamma RI, CD64, encoded by human FCGR1A, FCGR1B, FCGR1C) | Many types of carcinoma, lymphoma, sarcoma and leukaemia. |
| fibrin II beta chain (NH2 terminus) | Many types of carcinoma, lymphoma, sarcoma and leukaemia. |
| FLT1 (fms-related tyrosine kinase 1, vascular endothelial growth factor receptor 1, VEGFR-1, VEGFR, FLT, FRT, vascular permeability factor receptor) | Many types of carcinoma, sarcoma, lymphoma, sarcoma and leukaemia. In particular tumour blood vessels. |
| FOLH1 (folate hydrolase, prostate specific membrane antigen, PSMA) | Many types of carcinoma, lymphoma, sarcoma and leukaemia in particular prostate carcinoma and unwanted prostate tissue. |
| FOLR1 (folate receptor 1, folate receptor FR alpha, FR-alpha, adult folate-binding | Many types of carcinoma, lymphoma, sarcoma and leukaemia |
| protein, FBP, ovarian tumor-associated antigen MOv18) | |
| GD2 ganglioside | Brain tumours and unwanted neuronal tissue. |
| GD3 ganglioside | Brain tumours and unwanted neuronal tissue. |
| GLP1R (glucagon-like peptide 1 receptor) | Many types of carcinoma, lymphoma, sarcoma and leukaemia |
| GPNMB (glycoprotein transmembrane NMB, hematopoeitic growth factor inducible neurokinin-1 type, HGFIN) | Many types of carcinoma, lymphoma, sarcoma and leukaemia |
| hapten NP-cap (4-hydroxy-3-nitrophenacetyl caproic acid) | Many types of carcinoma, lymphoma, sarcoma and leukaemia |
| HAVCR1 (hepatitis A virus cellular receptor 1, T-cell immunoglobulin and mucin domain-containing protein 1, TIM1, KIM-1) | Hepatitis A infected cells. |
| HBV (hepatitis B virus) | HBV infected cells |
| HCMV (human cytomegalovirus) gB glycoprotein | CMV infected cells. |
| HCV (hepatitis C virus) | HCV infected cells |
| heat shock protein 90 homolog | Many types of carcinoma, lymphoma, sarcoma and leukaemia |
| HGF (hepatocyte growth factor, scatter factor, SF, hepatopoeitin A) | Hepatoma and hepatocellular carcinoma. Also unwanted hepatic tissue. |
| HIV-1 (human immunodeficiency virus) | HIV infected cells |
| HLA-DR10 (DRB1*1001) | Autologous or Allogeneic MHC Class-II expressing cells including tumour cells |
| HLA-DRB (HLA-DR beta) | Autologous or Allogeneic MHC Class-II expressing cells including tumour cells |
| HSV (herpes simplex virus) | HSV infected cells |
| ICAM1 (intercellular adhesion molecule 1, ICAM-1, CD54) | Many types of carcinoma, lymphoma, sarcoma and leukaemia |
| ICAM3 (intercellular adhesion molecule 3, ICAM-3, CD50) | Many types of carcinoma, lymphoma, sarcoma and leukaemia |
| Membrane Immunoglobulin IgE | IgE secreting B-cells and Plasma cells (cuasing allergic disease). |
| IgE Fc | IgE secreting B-cells and Plasma cells (cuasing allergic disease). |
| IGF1R (insulin-like growth factor 1 receptor, IGF1-R, IGF-1R, CD221) | Most types of carcinoma, lymphoma, sarcoma and leukaemia |
| IGHE connecting region (CO) M1 prime (in alternatively spliced heavy chain of membrane IgE on B cells | IgE secreting cells such as B-cells and plasma cells. Particularly unwanted in allergic disease. |
| IL2RA (interleukin-2 receptor, alpha subunit, IL-2RA, TAC, CD25) | B-cells and T-cells in either malignant or autoimmune disease. |
| IL2RB (interleukin-2 receptor beta subunit, IL-2RB, p70, CD122) | B-cells and T-cells in either malignant or autoimmune disease. |
| IL5RA (interleukin 5 receptor alpha subunit, CD125) | B-cells and T-cells in either malignant or autoimmune disease. |
| IL6R (interleukin 6 receptor, IL-6R, CD126) | B-cells and T-cells in either malignant or autoimmune disease. |
| ITGA2 {integrin alpha 2, GPIa, subunit of the alpha2beta1 integrin (VLA-2, collagen receptor), CD49b) | Many types of carcinoma, lymphoma, leukaemias. |
| ITGA2B_ITGB3 (integrin alpha2b_beta3, integrin αIIbβ3, GPIIbIIIa, fibrinogen receptor, CD41_CD61) | Many types of carcinoma, lymphoma, leukaemias. |
| ITGA4 (integrin alpha 4 subunit, CD49d) | Many types of carcinoma, lymphoma, leukaemias. |
| ITGA4_ITGB7 (integrin alpha4_beta7, integrin α4β7, lymphocyte Peyer's patch adhesion molecule 1, LPAM-1) | Many types of carcinoma, lymphoma, leukaemias. |
| ITGA5 (integrin alpha 5 subunit, CD49e) | Many types of carcinoma, lymphoma, leukaemias. |
| ITGAE_ITGB7 (integrin alphaE_beta7, integrin αEβ7, human mucosal lymphocyte antigen 1, HML-1) | Many types of carcinoma, lymphoma, leukaemias. |
| ITGAL (integrin alpha L subunit, lymphocyte function associated antigen 1, CD11 a) | Many types of carcinoma, lymphoma, leukaemias. |
| ITGAV_ITGB3 (integrin alphaV_beta3, integrin aVβ3, CD51_GPIIIa, vitronectin | Many types of carcinoma, lymphoma, leukaemias. |
| receptor, VNR, CD51_CD61 | |
| ITGB1 (integrin beta1 subunit, GPIIa, CD29) | Many types of carcinoma, lymphoma, leukaemias. |
| ITGB2 (integrin beta2 subunit, LFA-1, MAC-1, CD18) | Many types of carcinoma, lymphoma, leukaemias. |
| KDR (kinase insert domain receptor, vascular endothelial growth factor receptor 2, VEGFR2, VEGF-R2, FLK1, CD309) | Many types of carcinoma, lymphoma, leukaemias. |
| LTA (lymphotoxin alpha, TNF superfamily member 1, TNFSF1, LT) | Many types of carcinoma, lymphoma, leukaemias. |
| LTB (lymphotoxin beta, TNF superfamily member 3, TNFSF3, p33) | Many types of carcinoma, lymphoma, leukaemias. |
| MET (met proto-oncogene, hepatocyte growth factor HGF receptor, HGFR, scatter factor SF receptor, HGF/SF receptor, tyrosine protein kinase c-met, papillary renal cell carcinoma 2, RCCP2) | Many types of carcinoma, lymphoma, leukaemias. |
| MS4A1 (membrane-spanning 4-domains subfamily A member 1, CD20) | Many types of carcinoma, lymphoma, leukaemias. |
| MSLN (mesothelin, pre-pro-megakaryocyte-potentiating factor, megakaryocyte potentiating factor, MPF, CAK1) | Many types of carcinoma, lymphoma, leukaemias. |
| MST1 R (macrophage stimulating 1 receptor, macrophage stimulating protein receptor, MSP receptor, c-met-related tyrosine kinase, protein-tyrosine kinase 8, PTK8, RON, p185-Ron, CD136) | Many types of carcinoma, lymphoma, leukaemias. |
| MSTN (myostatin, growth differentiation factor 8, GDF8) | Many types of carcinoma, lymphoma, leukaemias. |
| MUC1 (mucin 1, polymorphic epithelial mucin, PEM, episialin, CD227) | Many types of carcinoma, lymphoma, leukaemias. |
| MUC1 sialylated carbohydrate, tumour-associated (CA242, cancer antigen 242) | Many types of carcinoma, lymphoma, leukaemias. |
| MUC16 (mucin 16, MUC-16, cancer antigen 125, CA125) | Many types of carcinoma, lymphoma, leukaemias. |
| MUC5AC (mucin 5AC, mucin 5 subtypes A and C tracheobronchial/gastric) | Many types of carcinoma, lymphoma, leukaemias. |
| N-glycolyl GM3 ganglioside (N-glycolylneuraminic acid (NeuGc, NGNA) GM3 ganglioside, NeuGcGM3) | Brain tumours and unwanted neural tissue. |
| NCA-90 (nonspecific cross-reacting antigens 90kDa glycoproteins, granulocyte cell antigen) | Many types of carcinoma, lymphoma, sarcoma and leukaemia |
| NCAM1 (neural cell adhesion molecule 1, NCAM-1, NCAM, CD56) | Brain tumours and unwanted neural tissue also many types of carcinoma and lymphoma. |
| Nectin-4 | Many types of carcinoma, lymphoma, sarcoma and leukaemia |
| NGF (nerve growth factor, nerve growth factor beta polypeptide, NGFB, beta-NGF) | Many types of carcinoma, lymphoma, sarcoma and leukaemia |
| NIP-cap (3-iodo-4-hydroxy-5-nitrophenylacetyl caproic acid) | Many types of carcinoma, lymphoma, sarcoma and leukaemia |
| NRP1 (neuropilin 1, NRP, vascular endothelial cell growth factor 165 receptor, VEGF165 receptor, VEGF165R, CD304) | Many types of carcinoma, lymphoma, sarcoma and leukaemia |
| PDGFRA (platelet-derived growth factor receptor alpha subunit, PDGFR2, CD140a) | Many types of carcinoma, lymphoma, sarcoma and leukaemia |
| phosphatidylserine | Many types of carcinoma, lymphoma, sarcoma and leukaemia particularly apoptotic cells. |
| PSCA (prostate stem cell antigen) | Many types of carcinoma and leukaemia. |
| RSV (human respiratory syncytial virus, glycoprotein F) | RSV infected cells |
| RTN4 (reticulon 4, neurite outgrowth inhibitor, NOGO) | Many types of carcinoma, lymphoma, sarcoma and leukaemia |
| SDC1 (syndecan-1, CD138) | Unwanted plasma cells found in plasma cell dyscrasias, particularly Myeloma, Amyloidosis and MGUS. |
| SELE (E-selectin, CD62E) | Many types of carcinoma and lymphoma. |
| SELL (L-selectin, CD62) | Many types of carcinoma and lymphoma. |
| SELP (P-selectin, CD62) | Many types of carcinoma and lymphoma. |
| SFRP1 (selected frizzled-related protein 1, fusion regulatory protein 1, FRP-1) | Many types of carcinoma and lymphoma. |
| SLAMF7 (SLAM family member 7, CD2 subset 1, CS1, CD2-like receptor-activating cytotoxic cells, CRACC,19A24, CD319) | Many types of unwanted cells including tumour cells and those involved in autoimmune disease. |
| SLC3A2 (solute carrier family 3 (activators of dibasic and neutral amino acid transport) member 2, 4F2 antigen heavy chain, 4F2HC, CD98 heavy chain, CD98hc, CD98) | Many types of unwanted cells including tumour cells and those involved in inflammatory disease. |
| SOST (sclerostin) | Bone disease including oesteosarcoma and osteoporosis. |
| Staphylococcus epidermidis lipoteichoic acid | Staphylococcus infected tissue. |
| T cell receptor (TR) TR alpha_beta | T-cell lymphoma or autoimmune-causing T-cells. |
| TGFB1 (transforming growth factor beta1, TGF beta) | Many types of unwanted cells including tumour cells and those involved in fibrotic disease. |
| TGFB2 (transforming growth factor beta 2) | Many types of unwanted cells including tumour cells and those involved in fibrotic disease. |
| TNF (tumor necrosis factor (TNF) superfamily member 2, TNFSF2, TNF-alpha, TNFA) | Many types of unwanted cells including tumour cells and those involved in inflammatory disease. |
| TNFRSF10A (tumor necrosis factor receptor (TNFR) superfamily member 10A, death receptor 4, DR4, TNF-related apoptosis-inducing ligand receptor 1, TRAILR1, TRAIL-R1, TR-1, CD261) | Many types of unwanted cells including tumour cells and those involved in inflammatory disease. |
| TNFRSF10B (tumor necrosis factor receptor (TNFR) superfamily member 10B, death receptor 5, DR5, TNF-related apoptosis-inducing ligand receptor 2, TRAILR2, TRAIL-R2, TR-2, CD262) | Many types of unwanted cells including tumour cells and those involved in inflammatory disease. |
| TNFRSF12A (tumor necrosis factor | Many types of unwanted cells including |
| receptor (TNFR) superfamily member 12A, fibroblast growth factor (FGF)-inducible 14 kDa protein, Fn14, TNF-like weak inducer of apoptosis (Tweak) receptor, Tweak receptor, TweakR, CD266 | tumour cells and those involved in inflammatory disease. |
| TNFRSF8 (tumor necrosis factor receptor (TNFR) superfamily member 8, CD30) | Many types of unwanted cells including tumour cells (in particular lymphoma) and those involved in inflammatory disease. |
| TNFRSF9 (tumor necrosis factor receptor (TNFR) superfamily member 9, 4-1 BB, T cell antigen ILA, CD137 | Many types of unwanted cells including tumour cells and those involved in inflammatory and autoimmune disease. |
| TNFSF11 (tumor necrosis factor (TNF) superfamily member 11, osteoclast differentiation factor, ODF, OPGL, RANKL, TRANCE, CD254) | Many types of unwanted cells including tumour cells and those involved in inflammatory and autoimmune disease. |
| TNFSF13 (tumor necrosis factor (TNF) superfamily member 13, a proliferation-including ligand, APRIL, CD256 | Many types of unwanted cells including tumour cells and those involved in inflammatory and autoimmune disease. |
| TNFSF13B (tumor necrosis factor (TNF) superfamily member 13B, B cell activating factor, BAFF, TALL1, BLyS, B lymphocyte activator, CD257) | Many types of unwanted cells including tumour cells and those involved in inflammatory and autoimmune disease. |
| TNFSF14 (tumor necrosis factor (TNF) superfamily member 14, LIGHT, HVEM-L, CD258) | Many types of unwanted cells including tumour cells and those involved in inflammatory and autoimmune disease. |
| TNFSF4 (tumor necrosis factor (TNF) superfamily member 4, OX40 ligand, OX-40L, TAX transcriptionally-activated glycoprotein 1, TXGP1, gp34, CD252) | Many types of unwanted cells including tumour cells and those involved in inflammatory and autoimmune disease. |
| TPBG (trophoblast glycoprotein, 5T4) | Multiple carcinomas. |
| TYRP1 (tyrosinase-related protein 1, 5,6-dihydroxyindole-2-carboxylic acid oxidase, DHICA oxidase, TRP1, melanoma antigen gp75) | Multiple carcinomas. |
| VAP-1 (vascular adhesion protein) | Multiple carcinomas and hepatomas. |
| VEGFA (vascular endothelial growth factor | Multiple carcinomas and hepatomas. |
| A, VEGF-A, VEGF) | |
| VIM (vimentin) | Multiple carcinomas and hepatomas. |

As used herein, the term "antibody" includes but is not limited to polyclonal, monoclonal, chimeric, single chain, Fab fragments, fragments produced by a Fab expression library and bispecific antibodies. Such fragments include fragments of whole antibodies which retain their binding activity for a target substance, Fv, F(ab') and F(ab')2 fragments, as well as single chain antibodies (scFv), fusion proteins and other synthetic proteins which comprise the antigen-binding site of the antibody. A targeting moiety comprising only part of an antibody may be advantageous by virtue of optimising the rate of clearance from the blood and may be less likely to undergo non-specific binding due to the Fc part. Also included are domain antibodies (dAbs), diabodies, camelid antibodies and engineered camelid antibodies. Furthermore, for administration to humans, the antibodies and fragments thereof may be humanised antibodies, which are now well known in the art (Janeway et al (2001) Immunobiology., 5th ed., Garland Publishing); An et al (2009) Therapeutic Monoclonal Antibodies: From Bench to Clinic, ISBN: 978-0-470-11791-0).

Also included are asymmetric IgG-like antibodies (eg triomab/quadroma, Trion Pharma/Fresenius Biotech; knobs-into-holes, Genentech; Cross MAbs, Roche; electrostatically matched antibodies, AMGEN; LUZ-Y, Genentech; strand exchange engineered domain (SEED) body, EMD Serono; biolonic, Merus; and Fab-exchanged antibodies, Genmab), symmetric IgG-like antibodies (eg dual targeting (DT)-Ig, GSK/Domantis; two-in-one antibody, Genentech; crosslinked MAbs, karmanos cancer center; mAb², F-star; and Cov X-body, Cov X/Pfizer), IgG fusions (eg dual variable domain (DVD)-Ig, Abbott; IgG-like bispecific antibodies, Eli Lilly; Ts2Ab, Medimmune/AZ; BsAb, ZymoGenetics; HERCULES, Biogen Idec; TvAb, Roche) Fc fusions (eg ScFv/Fc fusions, Academic Institution; SCORPION, Emergent BioSolutions/Trubion, ZymoGenetics/BMS; dual affinity retargeting technology (Fc-DART), MacroGenics; dual (ScFv)₂-Fab, National Research Center for Antibody Medicine) Fab fusions (eg F(ab)₂, Medarex/AMGEN; dual-action or Bis-Fab, Genentech; Dock-and-Lock (DNL), ImmunoMedics; bivalent bispecific, Biotechnol; and Fab-Fv, UCB-Celltech), ScFv- and diabody-based antibodies (eg bispecific T cell engagers (BiTEs), Micromet; tandem diabodies (Tandab), Affimed; DARTs, MacroGenics; Single-chain diabody, Academic; TCR-like antibodies, AIT, Receptor Logics; human serum albumin ScFv fusion, Merrimack; and COMBODIES, Epigen Biotech), IgG/non-IgG fusions (eg immunocytokins, EMDSerono, Philogen, ImmunGene, ImmunoMedics; superantigen fusion protein, Active Biotech; and immune mobilising mTCR Against Cancer, ImmTAC) and oligoclonal antibodies (eg Symphogen and Merus).

The antibody may possess any of the antibody-like scaffolds described by Carter (2006) "Potent antibody therapeutics by design", Nat Rev Immunol. 6(5): 343-57, and Carter (2011) "Introduction to current and future protein therapeutics: a protein engineering perspective", Exp Cell Res. 317(9): 1261-9. incorporated herein by reference, together with the specificity determining regions described herein. Thus, the term "antibody" also includes affibodies and non-immunoglobulin based frameworks. Examples include adnectins, anticalins, affilins, trans-bodies, darpins, trimerX, microproteins, fynomers, avimers, centgrins and kalbitor (ecallantide).

The advantages of using antibody fragments, rather than whole antibodies, are several-fold. The smaller size of the fragments may lead to improved pharmacological properties, such as better penetration of solid tissue. Moreover, antigen-binding fragments such as Fab, Fv, ScFv and dAb antibody fragments can be expressed in and secreted from *E. coli* or yeast, thus allowing convenient production in the laboratory and economical production on a commercial scale.

The antibody may be of any of the IgG, IgE, IgA, IgM and IgD classes and may be derived from any species. If the antibody is an IgG, it may be any of IgG1, IgG2, IgG3 or IgG4. It is preferred, however, that when the agent is for administration to a particular host, that the antibody, or at least the constant regions thereof, are derived from that host. For example, when the agent is to be administered to a human, the antibody is preferably a human antibody or a humanized antibody, and so on.

Suitable antibodies that bind to particular antigens expressed by unwanted cells can be made by the skilled person using technology long-established in the art. Methods of preparation of monoclonal antibodies and antibody fragments are well known in the art and include hybridoma technology (Kohler & Milstein (1975) "Continuous cultures of fused cells secreting antibody of predefined specificity. Nature 256: 495-497); antibody phage display (Winter et al (1994) "Making antibodies by phage display technology." Annu. Rev. Immunol. 12: 433-455); ribosome display (Schaffitzel et al (1999) "Ribosome display: an in vitro method for selection and evolution of antibodies from libraries." J. Immunol. Methods 231: 119-135); and iterative colony filter screening (Giovannoni et al (2001) "Isolation of anti-angiogenesis antibodies from a large combinatorial repertoire by colony filter screening." Nucleic Acids Res. 29: E27). Further, antibodies and antibody fragments suitable for use in the present invention are described, for example, in the following publications: "Monoclonal Hybridoma Antibodies: Techniques and Application", Hurrell (CRC Press, 1982); "Monoclonal Antibodies: A Manual of Techniques", H. Zola, CRC Press, 1987, ISBN: 0-84936-476-0; "Antibodies: A Laboratory Manual" 1st Edition, Harlow & Lane, Eds, Cold Spring Harbor Laboratory Press, New York, 1988. ISBN 0-87969-314-2; "Using Antibodies: A Laboratory Manual" 2nd Edition, Harlow & Lane, Eds, Cold Spring Harbor Laboratory Press, New York, 1999. ISBN 0-87969-543-9; and "Handbook of Therapeutic Antibodies" Stefan Dübel, Ed., 1st Edition, - Wiley-VCH, Weinheim, 2007. ISBN: 3-527-31453-9.

In another embodiment, the targeting moiety may be a nucleic acid molecule (e.g. DNA or RNA). For example, the targeting moiety may be a nucleic acid aptamer that binds to a specific target molecule.

As an alternative to the targeting moiety being a specific binding partner, the targeting moiety may be a non-specific molecule that is capable, following administration to a subject, of accumulating in the vicinity of the unwanted cells. For example, it is known that macromolecules accumulate non-specifically in tumours. Macromolecules known to accumulate in tumours non-specifically include albumin, immunoglobulins, transferrin, liposomes, nanoparticles (eg colloidal nanoparticles) and biodegradable polymers including dextrans, polyethylene glycol, polylysine and hydroxypropylmethylacrylamide. Macromolecules accumulate in human xenografted tumours in nude mice up to about 2.0% of administered dose per gram of tumour. Macromolecules such as polyethylene glycol and dextrans have been found to modify the clearance rate of substances to which they are attached and modify their concentration in tumours (Melton *et al,* 1987; Eno-Ammoquaye *et al,* 1996). In exceptional tumours, a non-specific macromolecule may accumulate in greater concentration than an antibody directed at the secreted antigen (Searle *et al,* 1981).

The discovery that such macromolecules accumulate in tumours has been called the Enhanced Permeability and Retention (EPR) effect, and has been attributed to the leakiness of tumour capillaries and deficient lymphatic drainage (Matsumura & Macda, 1986).

Thus, when the unwanted cells are tumour cells, the targeting moiety may be any of these macromolecules which accumulate in tumours. Preferably, the macromolecule used in the invention is hydrophilic and is characterised by being soluble in body fluids and in conventional fluids for parenteral administration. Suitably, the macromolecule is biodegradable so that systemic accumulation during repeated administration is avoided. Clearly, however, it must not be degraded so fast as to fail to accumulate at the site of the unwanted cells (e.g. tumour). Preferably, the molecular weight and size of the agent comprising such a macromolecule targeting moiety exceeds that of the renal threshold for urinary excretion (MW 60 000), as this helps the blood concentration to be sufficient to provide an effective blood:tumour concentration gradient. A molecular weight of up to at least 800 000 is generally suitable, for example up to 160 000. The macromolecule is preferably one which is not readily captured by the reticuloendothelial system. The molecular weights given exclude any water of hydration.

Macromolecules that are available as sub-units and are not biodegradable may be linked by biodegradable linking units so that the non-biodegradable components are filtered through the kidneys and excreted in the urine.

Alternatively, it is preferred if the polymer used to make the macromolecule is not biodegradable such that the molecular weight of any non-biodegradable portion of the conjugate should be less than the renal threshold (circa 70000) so that after degradation of the biodegradable portion the residual non-biodegradeable portion is excreted through the kidneys.

Conveniently, the macromolecule may be any of a dextran; a polyamino acid; a nanoparticle (eg colloidal nanoparticle), or a non-tumour-specific protein such as an immunoglobulin, an albumin or a transferrin. Suitably, it may be a copolymer of styrene and maleic anhydride, or may be polyaspartic acid, poly-L-lysine, polyethyleneimine or polyethylene glycol.

It is appreciated that such macromolecules are used in melanocyte-directed enzyme prodrug therapy (MDEPT), as described in WO 1998/024478.

Apart from a targeting moiety, it will be understood that other molecules may be attached to, or incorporated in, the agent of the invention. For example, the agent of the invention may comprise a further therapeutic agent including any of those described further below. Examples of such agents include anti-cancer agents, anti-inflammatory peptides and protease inhibitors. The agent may include a cytokine, a chemokine or an adjuvant. However, it will be appreciated that the larger the size of the additional moiety that is incorporated into the cyclic peptide (eg targeting moiety, further therapeutic agent, cytokine, chemokine or adjuvant), the greater the chance that the cyclic peptide has of itself becoming immunogenic or forming secondary structures that may prevent an enzyme (eg protease) from accessing the one or more cleavage sites. Hence, it is preferred if only the active regions (ie those regions responsible for the desired effect) of those additional moieties are incorporated. For example, it may be advantageous to include the active regions of cytokines such as IL-2 or of chemokines such as CXCL9, 10 or 11 which have been shown to be important in anti-viral memory T cell migration.

In an embodiment where the cyclic peptide comprises a T cell antigen peptide, and optionally, one or more linking moieties that are peptides, and optionally a peptide targeting moiety, it is appreciated that the one or more components may be part of a peptide or polypeptide (eg fusion peptide or polypeptide) that may be encoded by a nucleic acid molecule. The invention includes such a nucleic acid molecule and host cells containing them. For example, using genetic engineering techniques well established in the art a peptide may be produced to contain a T cell antigen peptide, optionally one or more linking peptides (which may contain one or more selectively cleavable cleavage sites) and optionally a peptide targeting moiety. Suitably, the T cell antigen peptide and targeting peptide are joined so that both portions retain their respective activities such that the agent may be targeted to the unwanted cell and the T cell antigen peptide may be presented by the unwanted cell so as to elicit an immune response. The T cell antigen peptide and targeting peptide portions are typically joined by a linker peptide which comprises a cleavage site that is cleavable selectively in the vicinity of the unwanted cells as described above.

Polynucleotides which encode suitable targeting moieties are known in the art or can be readily designed from known sequences such as from sequences of proteins known to interact with surface markers expressed on unwanted cells or contained in nucleotide sequence databases such as the GenBank, EMBL and dbEST databases. Polynucleotides which encode suitable T cell antigen peptides are known in the art or can readily be designed from known sequences and made.

Polynucleotides which encode suitable linker peptides can readily be designed from linker peptide sequences and made.

Thus, polynucleotides which encode the agents used in the invention can readily be constructed using well known genetic engineering techniques.

The nucleic acid is then expressed in a suitable host to produce an agent of the invention. Thus, the nucleic acid encoding the agent of the invention may be used in accordance with known techniques, appropriately modified in view of the teachings contained herein, to construct an expression vector, which is then used to transform an appropriate host cell for the expression and production of the agent of the invention of the invention.

It is appreciated that the nucleic acid encoding the agent of the invention may be joined to a wide variety of other nucleic acid sequences for introduction into an appropriate host. The companion nucleic acid will depend upon the nature of the host, the manner of the introduction of the nucleic acid into the host, and whether episomal maintenance or integration is desired, as is well known in the art.

It will be appreciated that any peptide portions of the agent of the invention can be peptide "mimetics", *ie* peptidomimetics which mimic the structural features of peptides comprising or consisting of the amino acid sequence as described herein. Peptidomimetics can be even more advantageous in therapeutic use, in the resistance to degradation, in permeability or in possible oral administration.

As mentioned above, the inventors have found that by presenting the T cell antigen peptide within a cyclic peptide, it is possible to dispense with a targeting moiety. Thus, in an embodiment of the invention, the agent does not comprise a targeting moiety as described herein.

Preferably, the agent of the invention has a half-life in plasma of at least 2, 3, 4, 5 or 6 hours, and more preferably at least 10, 15, 20 or 24 hours, at 37°C.

It may be desirable to modify the agent of the invention so that it can be more easily detected, for example by biotinylating it or by incorporating any detectable label known in the art such as radiolabels, fluorescent labels or enzymatic labels.

A particular preferred embodiment of the invention provides an agent for preventing or treating cancer, the agent comprising: a cyclic peptide comprising or consisting of a T cell antigen peptide; wherein in cyclised form the T cell antigen peptide is not capable of eliciting a T cell response; and wherein the T cell antigen peptide is rendered capable of eliciting a T cell response by selective cleavage of one or more cleavage sites (eg enzymatic cleavage sites such as protease cleavage sites) in the cyclic peptide in the vicinity of the cancer.

A further preferred embodiment of the invention provides an agent for preventing or treating cancer, the agent comprising: a cyclic peptide comprising or consisting of a peptide T cell antigen peptide; wherein in cyclised form the peptide T cell antigen peptide is not capable of eliciting a T cell response; and wherein the peptide T cell antigen peptide is rendered capable of eliciting a T cell response by selective cleavage of one or more protease cleavage sites in the cyclic peptide in the vicinity of the cancer.

The cancer may be any cancer such as breast cancer, ovarian cancer, endometrial cancer, cervical cancer, bladder cancer, renal cancer, melanoma, lung cancer, prostate cancer, testicular cancer, thyroid cancer, brain cancer, oesophageal cancer, gastric cancer, pancreatic cancer, colorectal cancer, liver cancer, leukaemia, myeloma, non-Hodgkin's lymphoma, Hodgkin's lymphoma, acute myeloid leukaemia, acute lymphoblastic leukaemia, chronic lymphoblastic leukaemia, lymphoproliferative disorder, myelodysplastic disorder, myeloproliferative disease, sarcoma, teratoma, meningioma, glioblastoma, head and neck cancer, skin cancer, adrenal carcinoma, rhabdosarcoma, oesteosarcoma and premalignant disease.

As described above, the inventors have shown that agents of the invention may be used to redirect existing immune responses to kill particular unwanted cells in a specific manner. Since any unwanted cell may be targeted in this way, the agents of the invention offer significant therapeutic potential.

Accordingly, a second aspect of the invention provides a method of preventing or treating a condition characterised by the presence of unwanted cells, the method comprising administering an agent according to the first aspect of the invention to a subject.

Thus, the method may involve identifying a subject who has a condition or who is at risk of developing a condition characterised by unwanted cells (eg cancer), administering the agent according to the first aspect of the invention to the subject, and monitoring the levels of the unwanted cells in the subject either by conducting tests to determine the number of unwanted cells or by monitoring the clinical symptoms of the subject. Depending on the results of the monitoring step, it may be necessary to administer more of the agent.

Similarly, the invention includes an agent according to the first aspect of the invention for use in preventing or treating a condition characterised by the presence of unwanted cells.

The invention also includes the use of an agent according to the first aspect of the invention in the manufacture of a medicament for preventing or treating a condition characterised by the presence of unwanted cells.

Preferences for the condition and unwanted cells are as described above with respect to the first aspect of the invention. Preferably, the unwanted cells are tumour cells and the condition is a tumour.

By preventing or treating a condition we include the meaning of reducing or alleviating symptoms in a patient (i.e. palliative use), preventing symptoms from worsening or progressing, treating the disorder (e.g. by inhibition or elimination of the causative agent), or prevention of the condition or disorder in a subject who is free therefrom.

It will be appreciated that the agents of the invention lend themselves to personalised medicine in the clinic whereby the most appropriate agent to be administered to the patient is determined, and either selected or prepared in the clinic. For example, before the step of administering the agent to the subject, any one of the following may be determined: (i) the MHC alleles of the subject, (ii) the cytotoxic T cell response of the subject to a T cell antigen peptide and (iii) the expression profile of the unwanted cell with regards to a molecule which may be the target of the targeting moiety and/or an enzyme which is able to cleave the cleavage site in the agent of the invention. The MHC alleles of a subject can be assessed by serological assays at the antigen level or by using DNA assays at the genetic level. Determining whether a given antigen stimulates a specific cytotoxic T cell response in a subject can be done by contacting isolated peripheral mononuclear blood cells from the subject with the antigen and using standard assays for cell proliferation. Assessing the expression profile of the unwanted cell may be carried out on a biopsy sample using routine assays for measuring nucleic acid (e.g. DNA or RNA transcripts) or protein levels. For example, transcriptomic or proteomic techniques may be used. In this way, it will be possible to identify tailored targeting moieties that bind specifically to, for example, surface markers expressed by the unwanted cell. It may also be possible to identify appropriate protease cleavage sites that may be selectively cleaved in the vicinity of the unwanted cells.

Thus the method of the second aspect of the invention may include the steps of (i) identifying a subject who has a condition, or who is at risk of developing a condition characterised by the presence of unwanted cells (eg cancer), (ii) taking a sample from the subject, (iii) analysing the sample to identify the T cell antigen peptide and/or cleavage site and/or targeting moiety for preventing or treating the condition in that subject, (iii) preparing the agent of the invention, (iv) administering the agent to the subject, and (v) monitoring the levels of unwanted cells in the subject either by conducting tests to determine the number of unwanted cells or by monitoring the clinical symptoms of the subject.

It is appreciated that an apparatus may be used to select and optionally prepare the most appropriate agent to be used for a particular patient. For example, the apparatus may perform an automated analysis of one or more samples from the subject, and based on this analysis select and optionally prepare a tailor-made agent for that subject. Thus the apparatus may perform serological assays on the sample to determine a subject's MHC alleles and based on this test various peptides for their efficiency in eliciting cytotoxic T cell response, so as to identify the best T cell antigen peptide for use in that patient. Similarly, the apparatus may carry out an expression profile of unwanted cells from the subject (eg from a biopsy sample) so as to determine a suitable cleavage site that will be selectively cleaved in the vicinity of the unwanted cell and/or determine a suitable targeting moiety that will bind to the unwanted cell.

By performing any one or more of these steps in the clinic an agent tailored for a particular subject can be prepared. For example, the agent can contain a T cell antigen peptide that is known to bind to patient's MHC molecules and elicit a strong T cell response, a protease cleavage site that allows the release of the T cell antigen peptide in the vicinity of the unwanted cells, and/or a targeting moiety that is known to bind selectively to surface markers expressed by the unwanted cell.

In one embodiment, the subject is administered a further therapeutic agent in addition to the agent according to the first aspect of the invention. For example, when administering the agent to prevent or treat a particular condition, a further therapeutic agent known to be useful for combating that condition may be administered. As an example, when the agent is for treating cancer, a further anti-cancer agent (eg anti-neoplastic chemotherapy) may be administered to the subject alongside the agent of the invention.. Similarly, the further therapeutic agent may be one that is known to have therapeutic application in allergic disease, inflammatory disease, regenerative medicine and neuroregenerative disease.

It is appreciated that the further therapeutic agent may be administered at the same time as the agent of the invention (i.e. simultaneous administration optionally in a co-formulation) or at a different time to the agent of the invention (i.e. sequential administration).

The further therapeutic agent may be any one or more of a vaccine; an immuno stimulatory drug; an anti-cancer agent; an agent inhibiting an antibody response against the agent of the invention; a protease inhibitor; and/or a targeting moiety capable of targeting to unwanted cells and which is attached to an enzyme capable of cleaving the one or more cleavage sites in the agent of the invention.

For example, in order to boost the effector immune response against the particular T cell antigen peptide used, it may be desirable to vaccinate the subject with the T cell antigen peptide; and/or administer immunostimulating agents such as IL-2, IL-7, IFNα, GM-CSF, metformin, lenalidomide; and/or administer anti-immunoregulatory agents such as Ipilimumab; all of which may be considered as further therapeutic agents.

It is also appreciated that if the subject is one to whom is administered immunosuppressive agents, that these immunosuppressive agents are withdrawn from the subject (e.g. by suspending treatment) when or before being administered the agent of the invention.

Similarly, it may be desirable to employ methods aimed at circumventing any immunogenicity issues relating to the agent of the invention whereby an adverse antibody response is elicited *in vivo.* For example, the subject may also be administered one or more agents that are known to inhibit the activity of B cells, such as any of Rituximab, cyclophosphamide, Syk inhibitors, an anti-BAFF antibody (eg Belimumab), an anti-CD22 antibody, an anti-CD20 antibody and an anti-CD19 antibody, all of which may be considered as further therapeutic agents. In this case, it is particularly preferred if the inhibitor of B cells is administered to the subject prior to the agent of the invention, eg as a pre-treatment to ablate B cells.

In another embodiment, it may be appropriate to administer a particular protease inhibitor so as to improve the target selectivity of the agent of the invention. For example, in the embodiment where the agent comprises a targeting moiety, if that targeting moiety is known to bind cells in both the heart and breast tissue, but only those in the breast are to be targeted, it may be desirable to administer an agent that selectively inhibits the protease responsible for rendering the T cell antigen peptide capable of eliciting a T cell response, in the heart but not the breast. In other words, an agent is administered to inhibit a protease that is capable of selectively cleaving the one or more cleavage sites in the cyclic peptide, but which protease resides in the vicinity (eg at or near the surface of) of wanted cells but not in the vicinity (eg at or near the surface of) of unwanted cells. This is also useful in the event that a protease cleavage site of the agent of the invention is cleavable by multiple proteases, some of which reside in the vicinity of unwanted cells and some of which reside in the vicinity of wanted cells. In this case, targeting specificity may be improved by administering a protease inhibitor that inhibits a protease that resides in the vicinity of wanted cells but nevertheless is capable of cleaving the cleavage site, and therefore rendering the T cell antigen peptide capable of eliciting a T cell response. The effect of administering the inhibitor would be to ensure that the T cell antigen peptide is preferentially rendered capable of eliciting a T cell response, only in the vicinity of the unwanted cells. For instance, if a subject with cancer also has active rheumatoid arthritis where MMP2 and other proteases are active, and the one or more cleavage sites in the agent of the invention are cleavable by multiple proteases including MMP2, it may be beneficial to inhibit MMP2 to prevent cleavage of the agent at the arthritic joint but retain cleavage at the cancer site by another protease.

In another embodiment, it may be desirable to administer a targeting moiety capable of targeting to the unwanted cells, that, in turn, is attached to an enzyme that is capable of cleaving the one or more cleavage sites in the agent of the invention. In this way, the targeting specificity of the agent of the invention would be further improved by ensuring that the one or more cleavage sites in the cyclic peptide can only be cleaved in the desired location. For example, a subject could be first administered a targeting moiety attached to a particular enzyme, so as to ensure that the enzyme is present in significant quantities within the vicinity of the unwanted cells, and second administered the agent of the invention, which would be selectively cleavable by the enzyme attached to the targeting moiety. The targeting moiety and enzyme may be any of those described above in relation to the first aspect of the invention. Preferably, the enzyme is a protease. Ideally, the enzyme (eg protease) is one that acts on a specific cleavage sequence and/or is not inhibited by plasma protease inhibitors such as alpha 2 macroglobulin.

The invention thus includes a composition comprising (i) an agent according to the first aspect of the invention and (ii) a further therapeutic agent, for use in preventing or treating a condition characterised by the presence of unwanted cells. Given that the agent of the invention and the further therapeutic agent may be administered simultaneously or sequentially, it will be appreciated that the invention includes an agent according to the first aspect of the invention for use in preventing or treating a condition characterised by the presence of unwanted cells in a subject who is administered a further therapeutic agent. It also follows that the invention includes a therapeutic agent for use in preventing or treating a condition characterised by the presence of unwanted cells in a subject who is administered an agent according to the first aspect of the invention.

Similarly, the invention includes a use of a composition comprising (i) an agent according to the first aspect of the invention and (ii) a further therapeutic agent, in the manufacture of a medicament for preventing or treating a condition characterised by the presence of unwanted cells. Again, given that the agent of the invention and the further therapeutic agent may be administered simultaneously or sequentially, it will be appreciated that the invention includes the use of a composition comprising an agent according to the first aspect of the invention in the manufacture of a medicament for preventing or treating a condition characterised by the presence of unwanted cells in a subject who is administered a further therapeutic agent. It also follows that the invention includes the use of a therapeutic agent in the manufacture of a medicament for preventing or treating a condition characterised by the presence of unwanted cells in a subject who is administered an agent according to the first aspect of the invention.

The invention also provides a composition comprising (i) an agent according to the first aspect of the invention and (ii) a further therapeutic agent suitable for preventing or treating the same condition characterised by the presence of unwanted cells. It is appreciated that the therapeutic agent mentioned in the immediately preceding two paragraphs may be agents suitable for treating the same condition characterised by the presence of unwanted cells, as treatable by the agents of the invention.

A third aspect of the invention provides an agent according to the first aspect of the invention for use in medicine.

A fourth aspect of the invention a pharmaceutical composition comprising an agent according to the first aspect of the invention, and a pharmaceutically acceptable carrier, diluent or excipient.

Whilst it is possible for the agent of the invention to be administered alone, it is preferable to present it as a pharmaceutical formulation, together with one or more acceptable carriers. The carrier(s) must be "acceptable" in the sense of being compatible with the therapeutic agent and not deleterious to the recipients thereof. Typically, the carriers will be water or saline which will be sterile and pyrogen free.

Where appropriate, the formulations may conveniently be presented in unit dosage form and may be prepared by any of the methods well known in the art of pharmacy. Such methods include the step of bringing into association the active ingredient (agent for treating or preventing a condition characterised by unwanted cells) with the carrier which constitutes one or more accessory ingredients. In general, the formulations are prepared by uniformly and intimately bringing into association the active ingredient with liquid carriers or finely divided solid carriers or both, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

The agent of the invention can be administered in the form of a suppository or pessary, or they may be applied topically in the form of a lotion, solution, cream, ointment or dusting powder. The agent may also be transdermally administered, for example, by the use of a skin patch.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The amount of the agent which is administered to the individual is an amount effective to combat the particular individual's condition. The amount may be determined by the physician.

Preferably, in the context of any aspect of the invention described herein, the subject to be treated is a human. Alternatively, the subject may be an animal, for example a domesticated animal (for example a dog or cat), laboratory animal (for example laboratory rodent, for example mouse, rat or rabbit) or an animal important in agriculture (i.e. livestock), for example horses, cattle, sheep or goats.

The invention provides a kit of parts for preventing of treating a condition characterised by the presence of unwanted cells, the kit comprising: (i) an agent according to the first aspect of the invention, and (ii) a targeting moiety that is capable of targeting to the unwanted cells and which is attached to an enzyme that is capable of cleaving the one or more cleavage sites in the agent according to the first aspect of the invention.

Suitable targeting moieties and enzymes to cleave the one of more cleavage sites are described above in relation to the first aspect of the invention.

For example, the targeting moiety may be a specific binding partner of an entity expressed by the unwanted cells, or a non-specific molecule that is capable, following administration to a subject, of accumulating in the vicinity of the unwanted cells. Examples of specific binding partner include any of an antibody; a hormone; a growth factor; a cytokine; or a receptor ligand, and examples of a non-specific molecule include any of polyethylene glycol; a dextran; a polyamino acid; a non-tumourspecific protein, such as immunoglobulin,; an albumin; or hydroxypropyl methylacrylamide. Specific examples of suitable targeting moieties (eg antibodies) are listed above in relation to the first aspect of the invention.

The targeting moiety and enzyme may be attached by standard techniques known in the art. For example, they may be attached covalently using conventional cross-linking procedures as described herein. Alternatively, they may be attached non-covalently by biotin/avidin/streptavidin or immunological associations. Given that plasma contains free biotin (vitamin H), it will be appreciated that the coupling would need to be performed *ex vivo.*

It will be appreciated that the kit allows one to first administer the targeting moiety attached to the enzyme to the subject, and establish the correct localisation of the targeting moiety in the subject (for example by the targeting moiety being detectably labelled (eg radiolabelled)) before administering the agent of the first aspect of the invention. The T cell antigen peptide is then rendered capable of eliciting a T cell response in the vicinity of the unwanted cells by virtue of the enzyme attached to the targeting moiety cleaving the one or more selectively cleavable cleavage sites in the agent of the invention. This allows the T cell antigen peptide to be presented on the surface of the unwanted cells so as to re-direct an existing immune response to the unwanted cells.

Accordingly, the invention further provides a method of preventing or treating a condition characterised by the presence of unwanted cells, the method comprising administering (i) an agent according to the first aspect of the invention, and (ii) a targeting moiety that is capable of targeting to the unwanted cells which is attached to an enzyme that is capable of cleaving the one or more cleavage sites in the agent of the first aspect of the invention, to a subject. Preferably, the targeting moiety attached to the enzyme is administered before the agent of the first aspect of the invention, for example to allow to correct localisation of the targeting moiety at the unwanted cells to be established. However, the targeting moiety may be administered at the same time as the agent according to the first aspect of the invention.

Similarly, the invention provides an agent according to the first aspect of the invention, and a targeting moiety that is capable of targeting to the unwanted cells which is attached to an enzyme that is capable of cleaving the one or more cleavage sites in the agent according to the first aspect of the invention, for use in preventing or treating a condition characterised by the presence of unwanted cells. Preferably, the targeting moiety attached to the enzyme is administered before the agent of the first aspect of the invention, for example to allow to correct localisation of the targeting moiety at the unwanted cells to be established. However, the targeting moiety may be administered at the same time as the agent according to the first aspect of the invention.

The condition characterised by the presence of unwanted cells may be any of a tumour (benign or malignant), an autoimmune condition, a cardiovascular disease, a degenerative disease, diabetes, an allergic reaction, a neurodegenerative disease such as Alzheimer's, a transplantation patient or an infectious disease.

The invention will now be described in further detail with the aid of the following Figures and Examples.
**Figure 1****:** LCL pulsed with cyclic peptide in the presence or absence of protease inhibitor cocktail.
**Figure 2****:** LCL and healthy B cells pulsed with cyclic peptide in the presence or absence of protease inhibitor cocktail.
**Figure 3****:** Plasma stability of cyclic peptide in LCL model.
**Figure 4****:** SW620 colorectal cell line pulsed with cyclic peptide in the presence or absence of protease inhibitor cocktail.
**Figure 5****:** Plasma stability of cyclic peptide in colorectal cancer model.
**Figure 6****:** Comparison of the plasma stability of cyclic peptide and antibody peptide epitope conjugate (APEC) in LCL model.

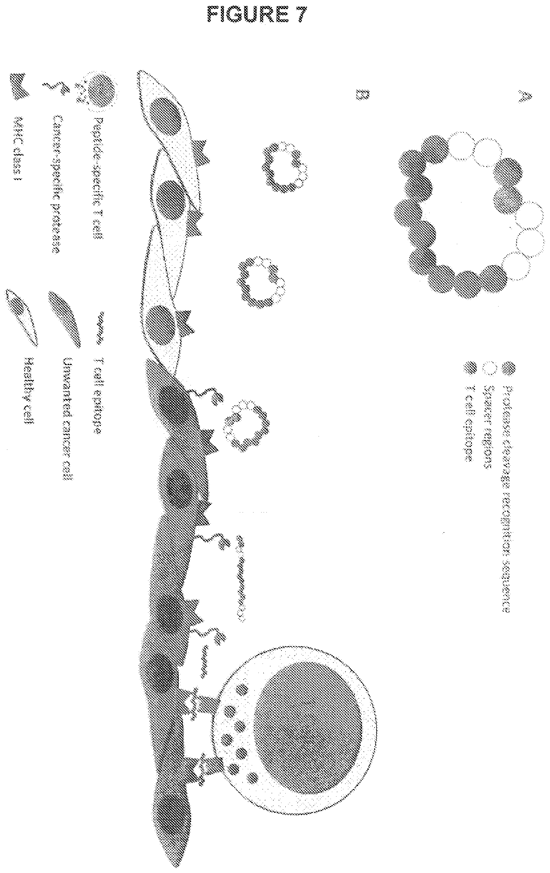

**Figure 7****:** Schematic illustration of example of cyclic peptide of invention.

### Example 1: LCL pulsed with cyclic peptide in the presence or absence of protease inhibitor cocktail

LCLs (tumour B cells) were incubated with or without a protease inhibitor cocktail for 2 hrs at 37°C. 10ug cyclic peptide or 10ug linear peptide was added to treated or untreated cells. The amino acid sequence of the cyclic peptide was FRGGANLVPMVATVAA (SEQ ID No: 278). The protease cleavage sequence "FR" is recognised by Cathepsin B and the T cell epitope NLVPMVATV is restricted to HLA-A*0201. The cyclic peptide was obtained from JPT Peptide Technologies. These cells were incubated with peptide-specific T cells overnight and IFN-y assayed for T cell recognition of the processed peptide.

There is a possibility that the cyclic peptide is not 100% cyclised and so a small amount of linear peptide will remain in the vial. As a control for this, T cells alone were treated with the cyclic peptide. T cells should not process the cyclic form of the peptide but would be able to present the linear form and therefore the level of IFN-y produced in this control would account for the amount of linear peptide present in the vial of cyclic peptide.

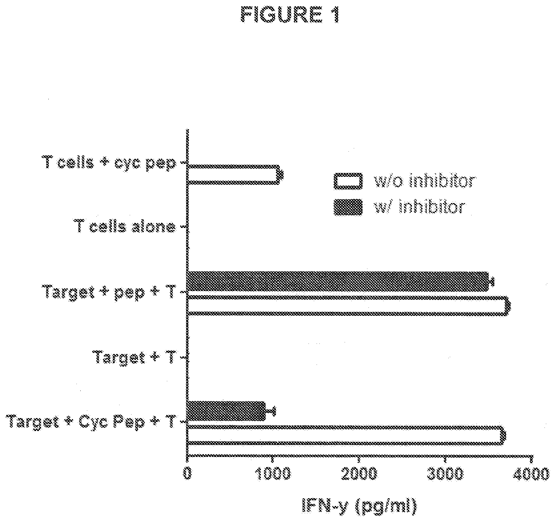

Figure 1 shows the results.

Target cells pulsed with the linear peptide demonstrate similar T cell recognition regardless of whether they were cultured in protease inhibitors or not. This is expected as there is no requirement for a protease to act on the linear peptide to allow it to bind to the target cell MHC.

Target cells treated with the cyclic peptide demonstrate a much larger response to IFN-y when cultured in the absence of the protease inhibitors. This demonstrates the requirement for a protease to act upon the cyclic peptide to liberate the viral epitope before it can bind to the target cell MHC. There is ∼75% decrease in the T cell recognition when the cells were cultured in the presence of the protease inhibitors. Interestingly, the response seen with the protease inhibitors is very similar to the level seen when T cells were cultured with the cyclic peptide alone suggesting that the response seen is also due to linear peptide that would not require any protease activity.

### Example 2: LCL and healthy B cells pulsed with cyclic peptide in the presence or absence of protease inhibitor cocktail

LCLs (tumour B cells) or healthy B cells were incubated with or without a protease inhibitor cocktail for 2 hrs at 37°C. 10ug cyclic peptide or 10ug linear peptide was added to treated or untreated cells. The amino acid sequence of the cyclic peptide was FRGGANLVPMVATVAA (SEQ ID No: 278). The protease cleavage sequence "FR" is recognised by Cathepsin B and the T cell epitope NLVPMVATV is restricted to HLA-A*0201. The cyclic peptide was obtained from JPT Peptide Technologies. These cells were incubated with peptide-specific T cells overnight and IFN-y assayed for T cell recognition of the processed peptide.

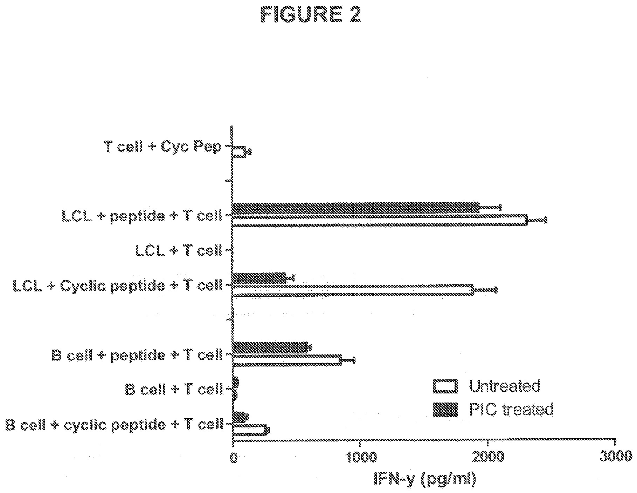

Figure 2 shows the results.

Healthy B cells pulsed with the linear peptide demonstrated similar T cell recognition regardless of whether they were cultured in protease inhibitors or not. This is expected as there is no requirement for a protease to act on the linear peptide to allow it to bind to the target cell MHC. Any small decrease seen when B cells are treated with PIC is likely to be due to a dilution effect of adding the PIC to the target cells, as a similar effect can be seen in the LCL + peptide + T cell when incubated with PIC.

Healthy B cells treated with the cyclic peptide demonstrate a significantly decreased amount of IFN-y compared to the LCL treated with the cyclic peptide and at a level comparable to that seen when T cells were cultured with the cyclic peptide alone. This suggests that this level of IFN-γ production is potentially due to the small amount of linear peptide that is found in the tube along with the cyclised peptide.

There is little difference in the response to healthy B cells treated with cyclic peptide after they had been treated with protease inhibitor cocktail compared with the untreated B cells labelled with cyclic peptide. (The responses seen are at a similar level to the T cells alone + cyclic peptide and are probably due to the small level of linear peptide present in the vial of cyclic peptide.) This demonstrates the inability of healthy B cells to process the cyclic peptide in the same manner that is seen with the labelled LCL. This is believed to be because healthy B cells do not express as much protease as LCLs and are therefore not able to process the peptide to the same level as tumour cells can.

The results for the LCL are similar to that shown in Example 1. These results demonstrate the differential targeting of tumour cell versus healthy cell.

### Example 3: Plasma stability of cyclic peptide in LCL model

LCLs (tumour B cells) were incubated with or without a protease inhibitor cocktail (PIC) for 2 hrs at 37°C. 10ug cyclic peptide or 10ug linear peptide which had been treated for 4 hrs @ 37°C with human plasma, was added to PIC treated or untreated cells. The cyclic peptide was the same as that described in Example 1. These cells were incubated with peptide-specific T cells overnight and IFN-y assayed for T cell recognition of the processed peptide.

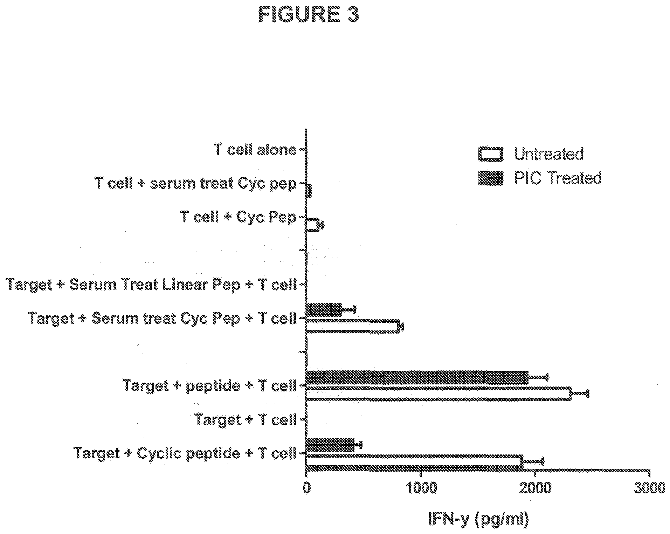

Figure 3 shows the results. The results for the LCL (bottom 3 sets of bars) are similar to that shown in Examples 1 and 2.

After serum treatment of the linear peptide for 4 hrs, there was no T cell response towards the target cells which had been labelled with the peptide regardless of PIC treatment. This suggests the plasma has destroyed the peptide which was expected and previous data has shown linear peptide to have a very short half-life in plasma.

Serum treatment of the cyclic peptide has decreased the responses but there is clearly still a response to the peptide suggesting that the cyclic peptide is stable in plasma for at least 4 hours. The cyclic peptide still needs to be cleaved for it to be "activated" as there is a decrease when target cells were pre-incubated with the protease inhibitor cocktail.

T cells labelled with the cyclic peptide in the absence of serum show a very small response to the peptide which then disappears upon treatment of the peptide with serum. This suggests that the small response seen may have been due to a small amount of linear peptide in the cyclic peptide vial which has been destroyed upon treatment with plasma.

### Example 4: SW620 colorectal cell line pulsed with cyclic peptide in the presence or absence of protease inhibitor cocktail

SW620 cells (colorectal cell line) incubated with or without a protease inhibitor cocktail for 2 hrs at 37°C. 10ug cyclic peptide or 10ug linear peptide was added to treated or untreated cells. These cells were incubated with peptide-specific T cells overnight and IFN-y assayed for T cell recognition of the processed peptide. The cyclic peptide was the same as that described in Example 1.

Similar to the LCL model, there is a possibility that the cyclic peptide is not 100% cyclised and a small amount of linear peptide will remain in the vial. As a control for this, T cells alone were treated with the cyclic peptide. T cells should not process the cyclic form of the peptide but would be able to present the linear form and therefore the level of IFN-y produced in this control would account for the amount of linear peptide present in the vial of cyclic peptide.

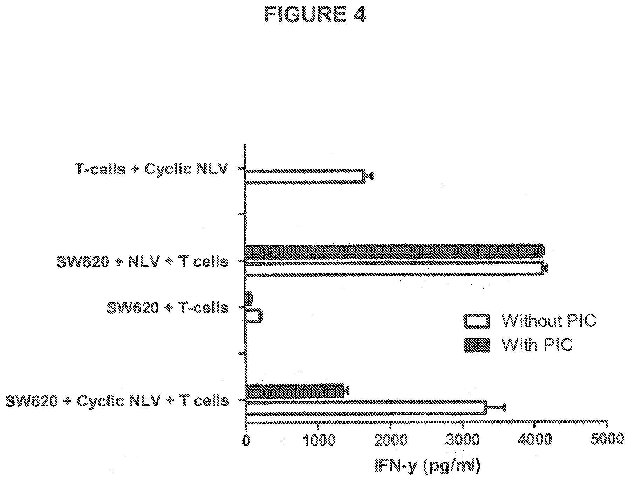

Figure 4 shows the results.

Target cells pulsed with the linear peptide demonstrate similar T cell recognition regardless of whether they were cultured in protease inhibitors or not. This is expected as there is no requirement for a protease to act on the linear peptide to allow it to bind to the target cell MHC.

Target cells treated with the cyclic peptide demonstrate a much larger response to IFN-y when cultured in the absence of the protease inhibitors. This demonstrates the requirement for a protease to act upon the cyclic peptide to liberate the viral epitope before it can bind to the target cell MHC. There is ∼65% decrease in the T cell recognition when the cells were cultured in the presence of the protease inhibitors. Interestingly, the response seen with the protease inhibitors is very similar to the level seen when T cells were cultured with the cyclic peptide alone suggesting that the response seen is also due to linear peptide that would not require any protease activity.

### Example 5: Plasma stability of cyclic peptide in colorectal cancer model

SW620 cells (colorectal cell line) were incubated with 10ug cyclic peptide or 10ug linear peptide which had been treated for 2 hrs @ 37°C with human plasma. These cells were incubated with peptide-specific T cells overnight and IFN-y assayed for T cell recognition of the processed peptide. The cyclic peptide was the same as that described in Example 1.

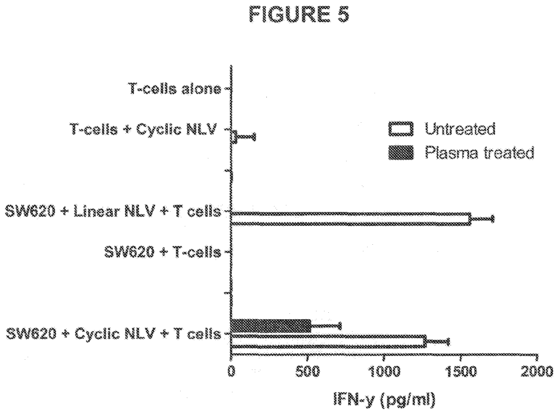

Figure 5 shows the results.

After serum treatment of the linear peptide for 2 hrs, there was no T cell response towards the target cells which had been labelled with the peptide. This suggests the plasma has destroyed the linear peptide which was expected.

Serum treatment of the cyclic peptide has decreased the response by ∼60% but there is clearly still a response to the peptide suggesting that the cyclic peptide is stable in plasma for at least 2 hours.

T cells labelled with the cyclic peptide in the absence of serum show a very small response to the peptide which then disappears upon treatment of the peptide with serum. This suggests that the small response seen may have been due to a small amount of linear peptide in the cyclic peptide vial which has been destroyed upon treatment with plasma.

Alone, the T cells do not spontaneously produce IFN-y and they do not recognise target cells which have previously not been treated with any peptides.

### Example 6: Comparison of the plasma stability of cyclic peptide and antibody-peptide epitope conjugate (APEC) in LCL model

10 ug cyclic peptide or 10 ug APEC, which had been treated for 4 hrs @ 37°C with human plasma, was added to target cells. These cells were incubated with peptide-specific T cells overnight and IFN-γ assayed for T cell recognition of the processed peptide. The cyclic peptide was the same as that described in Example 1.

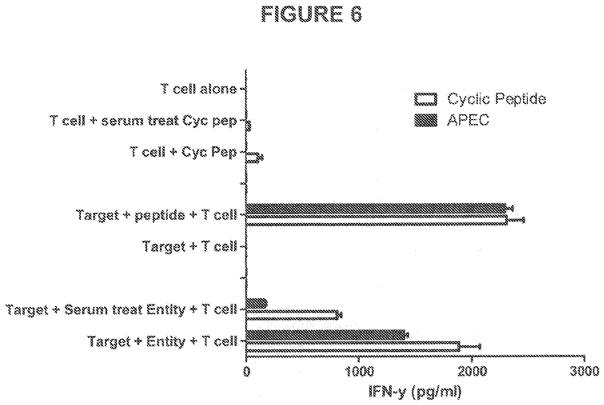

Figure 6 shows the results.

Serum treatment of the cyclic peptide (White bars) has decreased the responses by ∼50% compared with the untreated cyclic peptide but there is clearly still a response to the cyclic peptide suggesting that it is stable in plasma for at least 4 hours. Serum treatment of the APEC (Black bars) has reduced the T cell response to the labelled target cells by ∼90% compared with the cells labelled with untreated APEC. This suggests that the APEC has a decreased stability in plasma compared with the cyclic peptide.

T cells labelled with the cyclic peptide in the absence of serum show a very small response to the peptide which then disappears upon treatment of the peptide with serum. This suggests that the small response seen may have been due to a small amount of linear peptide in the cyclic peptide vial which has been destroyed upon treatment with plasma.

Alone, the T cells do not spontaneously produce IFN-γ and they do not recognise target cells which have previously not been treated with any peptides.

## Claims

1. An agent for use in preventing or treating a condition **characterised by** the presence of unwanted cells, comprising:
a cyclic peptide comprising a T cell antigen peptide;
wherein in cyclised form the T cell antigen peptide is not capable of eliciting a T cell response; and
wherein the T cell antigen peptide is rendered capable of eliciting a T cell response by selective cleavage of one or more cleavage sites in the cyclic peptide in the vicinity of the unwanted cells.

2. An agent for use according to Claim 1, wherein selective cleavage of one or more cleavage sites renders the T cell antigen peptide capable of eliciting a T cell response, in the vicinity of, and outside of the unwanted cells.

3. An agent for use according to Claim 1 or 2, wherein selective cleavage of one or more cleavage sites renders the T cell antigen peptide capable of eliciting a T cell response, at or near to the cell surface of the unwanted cells.

4. An agent for use according to any of Claims 1-3, wherein the one or more cleavage sites are:
(i) cleavable by an enzyme, optionally wherein the one or more cleavage sites are cleavable by any of a protease, a nuclease, a lipase, a lyase, a phosphatase or a carbohydrase; and/or
(ii) a protease cleavage site such as a Cathepsin B protease cleavage site, a cysteine protease cleavage site, an aspartyl protease cleavage site, a serine protease cleavage site, a matrix metalloproteinase cleavage site, a prostate specific antigen cleavage site or a CD10 protease cleavage site.

5. An agent for use according to any of Claims 1-4, wherein the cyclic peptide contains two or more different cleavage sites such as two or more different protease cleavage sites.

6. An agent for use according to any of Claims 1-5, wherein the T cell antigen peptide is any of:
(i) a peptide, optionally a peptide between 9 and 22 amino acids in length, a polypeptide or a phosphopeptide;
(ii) a viral-derived antigen;
(iii) derived from any of Varicella Zoster virus, Herpes simplex virus, cytomegalovirus, Epstein Barr virus, adenovirus, rhinovirus, influenza virus, or derived from a vaccine such as tetanus toxoid;
(iv) an MHC Class I restricted antigen or an MHC Class II restricted antigen; and/or
(v) any of the peptides CRVCCLYVL, NLVPMVATV, RPHERNGFTVL, TPRVTGGGAM, YSEHPTFTSQY, VTEHDTLLY, GLCTLVAML and CLGGLLTMV.

7. An agent for use according to any of Claims 1-6, wherein the cyclic peptide comprises a T cell antigen peptide and a linking moiety connecting the C-terminus of the T cell antigen peptide to the N-terminus of the T cell antigen peptide, optionally wherein:
(i) the linking moiety contains one or more cleavage sites, and/or
(ii) the linking moiety is a peptide, optionally wherein the peptide is 2-8 amino acids in length.

8. An agent for use according to any of Claims 1-7, wherein the condition **characterised by** the presence of unwanted cells is any of a tumour (benign or malignant), an autoimmune condition, a cardiovascular disease, a degenerative disease, an allergic disease, a neurodegenerative disease such as Alzheimer's, a transplantation patient or an infectious disease.

9. An agent for use according to any of Claims 1-8, wherein the condition **characterised by** the presence of unwanted cells is a tumour, the T cell antigen peptide is a peptide and the T cell antigen peptide is rendered capable of eliciting a T cell response by selective cleavage of one or more protease cleavage sites in the cyclic peptide.

10. An agent for use according to any of Claims 1-9, wherein the cyclic peptide comprising the T cell antigen peptide is a peptide between 12 and 30 amino acids in length.

11. An agent for use according to any of Claims 1-10, wherein the agent comprises the peptide sequence FRGGANLVPMVATVAA.

12. A pharmaceutical composition, comprising an agent as defined in any of Claims 1-11, and a pharmaceutically acceptable carrier, diluent or excipient, for use in preventing or treating a condition **characterised by** the presence of unwanted cells.

13. A composition comprising (i) an agent as defined in any of Claims 1-11 and (ii) a further therapeutic agent suitable for preventing or treating a condition **characterised by** the presence of unwanted cells, for use in preventing or treating a condition **characterised by** the presence of unwanted cells.

14. A kit of parts for use in preventing or treating a condition **characterised by** the presence of unwanted cells, the kit comprising: (i) an agent as defined in any of Claims 1-11, and (ii) a targeting moiety that is capable of targeting to the unwanted cells and which is attached to an enzyme that is capable of cleaving the one or more cleavage sites in the agent according to any of Claims 1-11.

15. An agent as defined in any of Claims 1-11, and a targeting moiety that is capable of targeting to the unwanted cells which is attached to an enzyme that is capable of cleaving the one or more cleavage sites in the agent according to any of Claims 1-11, for use in preventing or treating a condition **characterised by** the presence of unwanted cells.

16. The agent for use according to Claim 1, wherein the one or more cleavage sites are cleavable by a tumour associated protease.

17. The agent for use according to Claim 1, wherein the agent does not comprise a targeting moiety.

18. The agent for use according to Claim 1 wherein the unwanted cells are tumour cells.

## Patentansprüche

1. Mittel für den Gebrauch beim Vorbeugen oder Behandeln einer Erkrankung, die durch das Vorliegen von unerwünschten Zellen gekennzeichnet ist, Folgendes umfassend:
ein zyklisches Peptid, umfassend ein T-Zell-Antigenpeptid;
wobei das T-Zell-Antigenpeptid in zyklisierter Form nicht in der Lage ist, eine T-Zell-Antwort hervorzurufen; und
wobei dem T-Zell-Antigenpeptid die Fähigkeit verliehen wird, durch selektives Spalten einer oder mehrerer Spaltstellen im zyklischen Peptid in der Nähe der unerwünschten Zellen eine T-Zell-Antwort hervorzurufen.

2. Mittel für den Gebrauch nach Anspruch 1, wobei das selektive Spalten von einer oder mehreren Spaltstellen dem T-Zell-Antigenpeptid die Fähigkeit verleiht, in der Nähe und außerhalb der unerwünschten Zellen eine T-Zell-Antwort hervorzurufen.

3. Mittel für den Gebrauch nach Anspruch 1 oder 2, wobei das selektive Spalten von einer oder mehreren Spaltstellen dem T-Zell-Antigenpeptid die Fähigkeit verleiht, an oder in der Nähe der Zelloberfläche der unerwünschten Zellen eine T-Zell-Antwort hervorzurufen.

4. Mittel für den Gebrauch nach einem der Ansprüche 1-3, wobei die eine oder die mehreren Spaltstellen Folgendes sind:
(i) spaltbar durch ein Enzym, optional wobei die eine oder die mehreren Spaltstellen spaltbar sind durch eine Protease, eine Nuklease, eine Lipase, eine Lyase, eine Phosphatase oder eine Carbohydrase; und/oder
(ii) eine Protease-Spaltstelle, wie etwa eine Cathepsin-B-Protease-Spaltstelle, eine Cystein-Protease-Spaltstelle, eine Aspartyl-Protease-Spaltstelle, eine Serin-Protease-Spaltstelle, eine Matrix-Metalloprotease-Spaltstelle, eine prostataspezifische Antigen-Spaltstelle oder eine CD10-Protease-Spaltstelle.

5. Mittel für den Gebrauch nach einem der Ansprüche 1-4, wobei das zyklische Peptid zwei oder mehr verschiedene Spaltstellen enthält, wie etwa zwei oder mehr verschiedene Protease-Spaltstellen.

6. Mittel für den Gebrauch nach einem der Ansprüche 1-5, wobei das T-Zell-Antigenpeptid eines der Folgenden ist:
(i) ein Peptid, optional ein zwischen 9 und 22 Aminosäuren langes Peptid, ein Polypeptid oder ein Phosphopeptid;
(ii) ein von Viren abgeleitetes Antigen;
(iii) abgeleitet aus einem der Folgenden: Varicella-Zoster-Virus, Herpes-simplex-Virus, Cytomegalievirus, Epstein-Barr-Virus, Adenovirus, Rhinovirus, Influenza-Virus oder abgeleitet aus einem Impfstoff, wie etwa einem Tetanus-Toxoid;
(iv) ein MHC-Klasse-l-beschränktes Antigen oder ein MHC-Klasse-IIbeschränktes Antigen; und/oder
(v) eines der Peptide CRVCCLYVL, NLVPMVATV, RPHERNGFTVL, TPRVTGGGAM, YSEHPTFTSQY, VTEHDTLLY, GLCTLVAML und CLGGLLTMV.

7. Mittel für den Gebrauch nach einem der Ansprüche 1-6, wobei das zyklische Peptid ein T-Zell-Antigenpeptid und einen verknüpfenden Molekülteil umfasst, der das C-Ende des T-Zell-Antigenpeptids mit dem N-Ende des T-Zell-Antigenpeptids verbindet, optional wobei:
(i) der verknüpfende Molekülteil eine oder mehrere Spaltstellen enthält, und/oder
(ii) der verknüpfende Molekülteil ein Peptid ist, optional wobei das Peptid 2-8 Aminosäuren lang ist.

8. Mittel für den Gebrauch nach einem der Ansprüche 1-7, wobei die Erkrankung, die durch das Vorliegen von unerwünschten Zellen gekennzeichnet ist, eines der Folgenden ist: ein Tumor (gutartig oder bösartig), eine Autoimmunerkrankung, eine Herz-Kreislauf-Erkrankung, eine degenerative Erkrankung, eine allergische Erkrankung, eine neurodegenerative Erkrankung, wie etwa Alzheimer, ein Transplantationspatient oder eine Infektionskrankheit.

9. Mittel für den Gebrauch nach einem der Ansprüche 1-8, wobei die durch das Vorliegen von unerwünschten Zellen gekennzeichnete Erkrankung ein Tumor ist, das T-Zell-Antigenpeptid ein Peptid ist und dem T-Zell-Antigenpeptid die Fähigkeit verliehen wird, durch selektives Spalten einer oder mehrerer Protease-Spaltstellen in dem zyklischen Peptid eine T-Zell-Antwort hervorzurufen.

10. Mittel für den Gebrauch nach einem der Ansprüche 1-9, wobei das zyklische Peptid, welches das T-Zell-Antigenpeptid umfasst, ein zwischen 12 und 30 Aminosäuren langes Peptid ist.

11. Mittel für den Gebrauch nach einem der Ansprüche 1-10, wobei das Mittel die Peptidsequenz FRGGANLVPMVATVAA umfasst.

12. Pharmazeutische Zusammensetzung, umfassend ein Mittel nach einem der Ansprüche 1-11 und einen pharmazeutisch unbedenklichen Träger, Verdünner oder Hilfsstoff für den Gebrauch beim Vorbeugen oder Behandeln einer Erkrankung, **gekennzeichnet durch** das Vorliegen von unerwünschten Zellen.

13. Zusammensetzung, umfassend (i) ein Mittel nach einem der Ansprüche 1-11 und (ii) ein weiteres therapeutisches Mittel, das geeignet ist zum Vorbeugen oder Behandeln einer Erkrankung, die durch das Vorliegen von unerwünschten Zellen gekennzeichnet ist, für den Gebrauch beim Vorbeugen oder Behandeln einer Erkrankung, die durch das Vorliegen von unerwünschten Zellen gekennzeichnet ist.

14. Kit aus Teilen für den Gebrauch beim Vorbeugen oder Behandeln einer Erkrankung, die durch das Vorliegen von unerwünschten Zellen gekennzeichnet ist, wobei das Kit Folgendes umfasst: (i) ein Mittel nach einem der Ansprüche 1-11 und (ii) einen Targeting-Molekülteil, der in der Lage ist, ein Targeting der unerwünschten Zellen zu bewirken, und das an ein Enzym gebunden ist, das in der Lage ist, die eine oder die mehreren Spaltstellen in dem Mittel nach einem der Ansprüche 1-11 zu spalten.

15. Mittel nach einem der Ansprüche 1-11 und ein Targeting-Molekülteil, der in der Lage ist, ein Targeting der unerwünschten Zellen zu bewirken, das an ein Enzym gebunden ist, das in der Lage ist, die eine oder die mehreren Spaltstellen in dem Mittel nach einem der Ansprüche 1-11 zu spalten, für den Gebrauch beim Vorbeugen oder Behandeln einer Erkrankung, die durch das Vorliegen von unerwünschten Zellen gekennzeichnet ist.

16. Mittel für den Gebrauch nach Anspruch 1, wobei die eine oder die mehreren Spaltstellen durch eine tumorassoziierte Protease spaltbar sind.

17. Mittel für den Gebrauch nach Anspruch 1, wobei das Mittel keinen Targeting-Molekülteil umfasst.

18. Mittel für den Gebrauch nach Anspruch 1, wobei die unerwünschten Zellen Tumorzellen sind.

## Revendications

1. Agent pour une utilisation dans la prévention ou le traitement d'un état **caractérisé par** la présence de cellules indésirables, qui comprend :
un peptide cyclique comprenant un peptide antigénique reconnu par les cellules T ;
où, dans la forme cyclisée, le peptide antigénique reconnu par les cellules T n'est pas capable de déclencher une réponse des cellules T ; et
où le peptide antigénique reconnu par les cellules T est rendu capable de déclencher une réponse des cellules T par clivage sélectif de un ou plusieurs sites de clivage du peptide cyclique à proximité des cellules indésirables.

2. Agent pour une utilisation selon la revendication 1, où le clivage sélectif de un ou plusieurs sites de clivage rend le peptide antigénique reconnu par les cellules T capable de déclencher une réponse des cellules T à proximité et à l'extérieur des cellules indésirables.

3. Agent pour une utilisation selon la revendication 1 ou 2, où le clivage sélectif de un ou plusieurs sites de clivage rend le peptide antigénique reconnu par les cellules T capable de déclencher une réponse des cellules T au niveau ou près de la surface des cellules indésirables.

4. Agent pour une utilisation selon l'une quelconque des revendications 1-3, où les un ou plusieurs sites de clivage sont :
(i) clivables par une enzyme, en option où les un ou plusieurs sites de clivage sont clivables par l'une quelconque des enzymes suivantes : protéase, nucléase, lipase, lyase, phosphatase ou carbohydrase ; et/ou
(ii) un site de clivage tel qu'un site de clivage par une protéase de type cathepsine B, un site de clivage par une protéase à cystéine, un site de clivage par une protéase à aspartate, un site de clivage par une protéase à sérine, un site de clivage par une métalloprotéinase matricielle, un site de clivage par l'antigène spécifique de la prostate ou un site de clivage par une protéase de type CD10.

5. Agent pour une utilisation selon l'une quelconque des revendications 1-4, où le peptide cyclique contient deux ou plusieurs sites de clivage différents, comme deux ou plusieurs sites de clivage par une protéase différents.

6. Agent pour une utilisation selon l'une quelconque des revendications 1-5, où le peptide antigénique reconnu par les cellules T est l'un quelconque des suivants :
(i) un peptide, en option un peptide d'entre 9 et 22 acides aminés de longueur, un polypeptide ou un phosphopeptide ;
(ii) un antigène dérivé d'un virus ;
(iii) dérivé de l'un quelconque des virus suivants : virus de la varicelle et du zona, Herpes simplex virus, cytomégalovirus, virus Epstein Barr, adénovirus, rhinovirus, virus de la grippe, ou dérivé d'un vaccin comme la toxine ténanique ;
(iv) un antigène dont la présentation est restreinte au CMH de classe I ou un antigène dont la présentation est restreinte au CMH de classe II ; et/ou
(v) l'un quelconque des peptides suivants : CRVCCLYVL, NLVPMVATV, RPHERNGFTVL, TPRVTGGGAM, YSEHPTFTSQY, VTEHDTLLY, GLCTLVAML et CLGGLLTMV.

7. Agent pour une utilisation selon l'une quelconque des revendications 1-6, où le peptide cyclique comprend un peptide antigénique reconnu par les cellules T et une fraction de liaison raccordant l'extrémité C-terminale du peptide antigénique reconnu par les cellules T à l'extrémité N-terminale du peptide antigénique reconnu par les cellules T, en option où :
(i) la fraction de liaison contient un ou plusieurs sites de clivage et/ou
(ii) la fraction de liaison est un peptide, en option où le peptide est de 2-8 acides aminés de longueur.

8. Agent pour une utilisation selon l'une quelconque des revendications 1-7, où l'état **caractérisé par** la présence de cellules indésirables est l'un quelconque des suivants : tumeur (bénigne ou maligne), affection auto-immune, maladie cardio-vasculaire, maladie dégénérative, maladie allergique, maladie neurodégénérative comme la maladie d'Alzheimer, patient soumis à une transplantation ou maladie infectieuse.

9. Agent pour une utilisation selon l'une quelconque des revendications 1-8, où l'état **caractérisé par** la présence de cellules indésirables est une tumeur, le peptide antigénique reconnu par les cellules T est un peptide et le peptide antigénique reconnu par les cellules T est rendu capable de déclencher une réponse des cellules T par clivage sélectif de un ou plusieurs sites de clivage par une protéase du peptide cyclique.

10. Agent pour une utilisation selon l'une quelconque des revendications 1-9, où le peptide cyclique comprenant le peptide antigénique reconnu par les cellules T est un peptide d'entre 12 et 30 acides aminés de longueur.

11. Agent pour une utilisation selon l'une quelconque des revendications 1-10, où l'agent comprend la séquence peptidique FRGGANLVPMVATVAA.

12. Composition pharmaceutique comprenant un agent tel que défini à l'une quelconque des revendications 1-11 et un véhicule, un diluant ou un excipient pharmaceutiquement acceptable pour une utilisation dans la prévention ou le traitement d'un état **caractérisé par** la présence de cellules indésirables.

13. Composition comprenant (i) un agent tel que défini à l'une quelconque des revendications 1-11 et (ii) un agent thérapeutique supplémentaire qui se prête à la prévention ou au traitement d'un état **caractérisé par** la présence de cellules indésirables, pour une utilisation dans la prévention ou le traitement d'un état **caractérisé par** la présence de cellules indésirables.

14. Kit d'éléments pour une utilisation dans la prévention ou le traitement d'un état **caractérisé par** la présence de cellules indésirables, le kit comprenant : (i) un agent tel que défini à l'une quelconque des revendications 1-11 et (ii) une fraction de ciblage qui est capable de cibler les cellules indésirables et qui est attachée à une enzyme capable de cliver les un ou plusieurs sites de clivage de l'agent selon l'une quelconque des revendications 1-11.

15. Agent tel que défini à l'une quelconque des revendications 1-11, et fraction de ciblage qui est capable de cibler les cellules indésirables et qui est attachée à une enzyme capable de cliver les un ou plusieurs sites de clivage de l'agent selon l'une quelconque des revendications 1-11, pour une utilisation dans la prévention ou le traitement d'un état **caractérisé par** la présence de cellules indésirables.

16. Agent pour une utilisation selon la revendication 1, où les un ou plusieurs sites de clivage sont clivables par une protéase associée à une tumeur.

17. Agent pour une utilisation selon la revendication 1, où l'agent ne comprend pas une fraction de ciblage.

18. Agent pour une utilisation selon la revendication 1, où les cellules indésirables sont des cellules tumorales.
